# EUROPEAN PATENT APPLICATION

(11) **EP 4 442 830 A1**
(43) Date of publication of application: **09.10.2024**
(21) Application number: 22897986.0
(22) Date of filing: 29.11.2022
(51) Int. Cl.: C12N 15/864, A61K 48/00

(54) **AADC/GDNF POLYNUCLEOTIDE, AND USE THEREOF IN TREATING PARKINSON'S DISEASE**

(30) Priority: 29.11.2021 CN 202111434384
(71) Applicant: Shanghai Regenelead Therapies Co., Ltd., Shanghai 201206 (CN)
(72) Inventor: JIANG, Jun, Shanghai 201206 (CN); WU, Mingyue, Shanghai 201206 (CN); NING, Wei, Shanghai 201206 (CN); LIAO, Cheng, Shanghai 201206 (CN)
(74) Representative: Dragotti & Associati S.R.L.
(86) International application number: PCT/CN2022/134993
(87) International publication number: WO 2023/093905

(57) **Abstract**

Provided are an aromatic L-amino acid decarboxylase (AADC)/glial cell line-derived neurotrophic factor (GDNF) polynucleotide, and a use thereof in treating Parkinson's disease. Specifically, provided are a method for and a use in treating neurodegenerative diseases (such as Parkinson's disease) by delivering AADC and a GDNF to specific areas of the brain by a gene delivery system using AAV as a vector.

## Description

The present disclosure claims priority to the Chinese Patent Application (Application No. CN202111434384.4) filed on Nov. 29, 2021.

### TECHNICAL FIELD

The present disclosure relates to the field of gene therapy, and particularly to a gene therapy pharmaceutical composition and a treatment method for treating Parkinson's disease. Specifically, the present disclosure relates to a gene delivery system using AAV as a vector for delivering aromatic L-amino acid decarboxylase (AADC) and glial cell line-derived neurotrophic factor (GDNF) to specific areas of the brain for the treatment of Parkinson's disease.

### BACKGROUND

Parkinson's disease (PD) is a progressive neurodegenerative disease of the central nervous system (CNS), mainly affecting dopaminergic (DA) neurons in the substantia nigra (SN). Clinically, PD is mainly characterized by static tremor, bradykinesia, myotonia, and postural gait disorder, which may cause significant disability of patients within 10 to 15 years of onset and seriously affect the life quality of the patients. The etiology of PD is still unknown. Previous studies have shown that it may be associated with a number of factors, including aging, genetic factors, environmental toxicants, infections, oxidative stress, and formation of radicals. The major motor symptoms of PD result from the death of substantia nigra cells in the midbrain, which results in dopamine insufficiency in the related brain areas of the patients.

Aromatic L-amino acid decarboxylase (AADC) is a homodimeric pyridoxal phosphate-dependent enzyme responsible for the syntheses of dopamine and serotonin, which is capable of catalyzing the decarboxylation of L-3,4-dihydroxyphenylalanine (L-DOPA or levodopa) to dopamine. AADC deficiency may result in serotonin and catecholamine deficiencies and a decrease in the neurotransmitter DOPA, thereby causing severe motor and autonomic dysfunctions (Nutt et al., 2020; Pons et al., 2004). Glial cell line-derived neurotrophic factor (GDNF) is expressed in different brain areas of the central nervous system. The relatively certain cell sources include type I astrocytes and neurons in the substantia nigra-striatum system and basal forebrain. Comparable GDNF mRNA expression is found in DA neuronal projection areas such as basal ganglia and olfactory tubercle, neural structures related to certain movements such as substantia innominata, cerebellar Purkinje cells and trigeminal motor nuclei, and structures related to certain senses such as thalamus, trigeminal sensory nuclei, spinal cord dorsal horn, dorsal root ganglia and locus coeruleus, etc. (Golden et al., 1998) GDNF belongs to neurotrophic factors (NTFs), has a high affinity for DA neurons, and is a highly specific neurotrophic factor for DA neurons. The mode of action of GDNF is mainly target-derived, and there may also be paracrine and autocrine modes of action. GDNF has strong action and a broad-spectrum neurotrophic effect (Lin et al., 1993). Studies show that GDNF has significant nutrition for DAergic neurons in the embryonic midbrain cultured *in vitro* and promotes survival and differentiation, thereby enlarging neuron somata and lengthening axons (Lin et al., 1993); *in vivo,* GDNF also has protection and repair effects on the nigrostriatal DAergic system (Wang et al., 2002). Mice are treated with MPTP or rats are treated with 6-hydroxydopa (6-OHDA). Animals are injected with GDNF to the substantia nigra or striatum before or after the treatment, which can reduce the damage to DAergic neurons caused by MPTP or 6-OHDA, prevent the degeneration of DAergic neurons, induce the growth of new processes in the remaining DAergic neurons, restore DA levels and the density of DAergic nerve fibers, and significantly improve animals' motor behaviors (Kearns et al., 1997; D. Kirik et al., 2001; D. Kirik, Rosenblad, and Bjorklund, 2000; Deniz Kirik, Georgievska, and Björklund, 2004; Wang et al., 2002).

The recombinant adeno-associated virus (rAAV) is derived from a non-pathogenic wild-type adeno-associated virus, has characteristics of good safety, wide host cell range (dividing and non-dividing cells), low immunogenicity, and long duration for expressing exogenous genes *in vivo,* is considered to be one of the most promising gene delivery vectors, and is widely applied to gene therapy and vaccine research worldwide (Snyder, 1999; Xiao, Lentz, and Samulski, 2012).

Currently, the most successful drug on the market for treating PD is the dopamine drug levodopa (L-DOPA). However, with the duration of treatment, the benefits of dopamine therapy are gradually lost due to progressive death of dopaminergic neurons and loss of AADC activity, further causing increased dose-dependence of long-term drug administration. Systemic administration of high doses of dopamine is accompanied with strong side effects, such as recent side effects (gastrointestinal reactions, cardiovascular side effects, sleep disorder, poor mental symptoms, etc.) and long-term side effects (dyskinesia, motor fluctuations, morning stiffness, delayed onset of action, etc.). Therefore, there is an urgent problem to be solved to provide a highly safe and long-term effective therapeutic drug. The present disclosure provides an improved polynucleotide construct, which utilizes AAV and a derived vector thereof to wrap AADC, GDNF, and other genes in the AAV shell and deliver it to a specific area of the brain (such as striatum) for the treatment of PD through gene therapy approaches.

### SUMMARY

The present disclosure provides a polynucleotide expressing AADC protein and/or GDNF protein, a recombinant adeno-associated virus (rAAV) particle, a related expression cassette, a plasmid, a vector, a host cell, a pharmaceutical composition, as well as a method for treating, alleviating, or preventing neurodegenerative diseases (e.g., Parkinson's disease), and a related pharmaceutical use thereof.

### AADC and/or GDNF polynucleotide sequences

The present disclosure provides a nucleic acid molecule comprising a polynucleotide encoding AADC protein (the encoded amino acid sequence of the AADC protein is set forth, for example, in SEQ ID NO: 1), wherein the polynucleotide has at least 60%, at least 70%, at least 75%, at least 80%, at least 90%, or at least 95% identity (e.g., has at least 80.83%, at least 81.21%, at least 79.18%, at least 79.63% or more identity) to SEQ ID NO: 3, or has at least 95% identity to any one of SEQ ID NOs: 4-7. In some embodiments, the polynucleotide encoding the AADC protein comprises the polynucleotide sequence set forth in any one of SEQ ID NOs: 3-7.

The present disclosure provides a nucleic acid molecule comprising a polynucleotide encoding GDNF protein (the encoded amino acid sequence of the AADC protein is set forth, for example, in SEQ ID NO: 2), wherein the polynucleotide has at least 60%, at least 70%, at least 80%, at least 90%, or at least 95% identity (e.g., has at least 75.13%, at least 73.99%, at least 77.52%, at least 77.83% or more identity) to SEQ ID NO: 8, or has at least 95% identity to any one of SEQ ID NOs: 9-12. In some embodiments, the polynucleotide encoding the GDNF protein comprises the polynucleotide sequence set forth in any one of SEQ ID NOs: 8-12.

In some embodiments of the present disclosure, the AADC protein and the GDNF protein encompass analogs thereof.

The present disclosure provides a nucleic acid molecule comprising a first polynucleotide and a second polynucleotide, wherein the first polynucleotide comprises a polynucleotide encoding AADC protein (the encoded amino acid sequence of the AADC protein is set forth, for example, in SEQ ID NO: 1), and the second polynucleotide comprises a polynucleotide encoding GDNF protein (the encoded amino acid sequence of the AADC protein is set forth, for example, in SEQ ID NO: 2). In some embodiments, the first polynucleotide and the second polynucleotide are operably linked, and the first polynucleotide and the second polynucleotide may be located in the same polynucleotide, the same plasmid, or the same expression cassette. Alternatively, in some other embodiments, the first polynucleotide and the second polynucleotide are present independently of each other in two polynucleotides that are present independently of each other, in two different plasmids that are present independently of each other, or in two different expression cassettes.

In some embodiments, the amino acid sequence of the AADC protein comprises or is set forth in SEQ ID NO: 1, and the amino acid sequence of the GDNF protein comprises or is set forth in SEQ ID NO: 2.

In some embodiments, the first polynucleotide sequence encoding the AADC protein and/or the second polynucleotide sequence encoding the GDNF protein are/is codon-optimized. For example, the codon-optimized polynucleotide sequence encoding the AADC protein has at least 60%, at least 70%, at least 75%, at least 80%, at least 90%, or at least 95% identity to SEQ ID NO: 3; the codon-optimized polynucleotide sequence encoding the GDNF protein has at least 60%, at least 70%, at least 80%, at least 90%, or at least 95% identity to SEQ ID NO: 8.

In some embodiments, the polynucleotide sequence encoding the AADC protein comprises a sequence having at least 95% identity to any one of SEQ ID NOs: 4-7, and/or the polynucleotide sequence encoding the GDNF protein comprises a sequence having at least 95% identity to any one of SEQ ID NOs: 9-12. In some specific embodiments, the polynucleotide sequence encoding AADC comprises or is the polynucleotide sequence set forth in any one of SEQ ID NOs: 3-7; the polynucleotide sequence encoding GDNF comprises or is the polynucleotide sequence set forth in any one of SEQ ID NOs: 8-12.

In the present disclosure, "at least 95% identity" is meant to encompass at least 95%, at least 96%, at least 97%, at least 98%, at least 99% or more identity.

The codon-optimized polynucleotide expressing the AADC protein (e.g., any one of SEQ ID NOs: 4-7) results in an increased expression level of the AADC protein as compared to a wild-type or non-codon-optimized polynucleotide (e.g., SEQ ID NO: 3). The codon-optimized polynucleotide expressing the GDNF protein (e.g., any one of SEQ ID NOs: 9-12) results in an increased expression level of the GDNF protein as compared to a wild-type or non-codon-optimized polynucleotide (e.g., SEQ ID NO: 8). The increase is an increase in the protein expression level of at least 10%, at least 20%, at least 30%, at least 50%, at least 75%, at least 100%, or at least several times (including 2, 3, 4, 5, 6, 7, 8, 9, 10 or more) compared to the wild-type or non-codon-optimized polynucleotide.

In some embodiments, the nucleic acid molecule provided by the present disclosure further comprises the same expression control sequence operably linked to the first polynucleotide and the second polynucleotide, or two identical or different expression control sequences operably linked to the first polynucleotide and the second polynucleotide, respectively, wherein the expression control sequence comprises (c) a promoter and/or (d) an enhancer.

In some embodiments, the nucleic acid molecule provided by the present disclosure further comprises any one or any combination of the following:
(a) a 5' inverted terminal repeat (5' ITR);
(b) a 3' inverted terminal repeat (3' ITR);
(e) an intron;
(f) a post-transcriptional regulatory element;
(g) a polyadenylation signal (polyA); and
(h) a multiple cloning site (MCS).

In some specific embodiments, any combination of (a)-(h) is capable of fulfilling the function of allowing expression of a target gene (AADC and/or GDNF), e.g., expression in the brain (such as substantia nigra or striatum) of a subject.

In some specific embodiments, the polynucleotide of any one of (a)-(h), or any combination thereof, is operably linked to the polynucleotide encoding the AADC protein and/or the polynucleotide encoding the GDNF protein.

In some specific embodiments, the 5' ITR and/or 3' ITR are/is derived from AAV1, AAV2, AAV3, AAV4, AAV5, AAV6, AAV7, AAV8, AAV9, AAV9.47, AAV9(hu14), AAV10, AAV11, AAV12, AAVrh8, AAVrh10, AAV-DJ or AAV-DJ8; for example, from AAV2 or AAV9.

In some specific embodiments, the promoter is selected from the group consisting of CMV, CAG, CBh, EFS, EF1 (e.g., EF-1α), PGK, SV40, Ubi, RSV, or any combination thereof.

In some specific embodiments, the enhancer is selected from the group consisting of Ubi, CMV and RSV enhancers, or any combination thereof.

In some specific embodiments, the intron is selected from the group consisting of MVM, SV40, β Globin, EF1 (e.g., EF-1α) and hybrid introns, or any combination thereof.

In some specific embodiments, the polyA is selected from the group consisting of PA75 polyA, SV40 polyA, hGH polyA, BGH polyA, rbGlob polyA, or any combination thereof.

In some specific embodiments, the post-transcriptional regulatory element is selected from the group consisting of WPRE, HPRE, or a combination thereof.

In some embodiments, the nucleic acid molecule comprises a polynucleotide encoding AADC protein, a CMV enhancer (e.g., CMV enhancer 1), and a CBA promoter, and optionally, the first polynucleotide further comprises a hybrid intron and a polyA, the polyA being selected from the group consisting of SV40 polyA and hGH polyA; and/or the nucleic acid molecule comprises a polynucleotide encoding GDNF protein, a CMV enhancer (e.g., CMV enhancer 2), and a CMV promoter, and optionally, the second polynucleotide further comprises a β globin intron and a polyA, the polyA being selected from the group consisting of PA75 polyA and SV40 polyA; wherein the CMV enhancer 1 and the CMV enhancer 2 are both CMV enhancers, and they may be identical or different.

In some embodiments, AADC and GDNF in the polynucleotide may use different expression frameworks independently or share one expression framework by linkage via a linker (e.g., P2A sequence).

The first polynucleotide and the second polynucleotide are operably linked, so that the AADC protein and the GDNF protein are expressed at the same target administration position of a subject, which can prevent the therapeutic effect of the combination of the two proteins from being affected due to inconsistent administration positions of the two proteins, and can reduce side effects caused by leakage of one of the proteins to other positions. Referring to the examples of the present disclosure, the first polynucleotide and the second polynucleotide are operably linked and then administered to a diseased model mouse, and the results show that a significant therapeutic effect can be exerted. In some embodiments, the first polynucleotide and the second polynucleotide are linked by a third polynucleotide; for example, the third polynucleotide encodes an amino acid sequence with linker function. The linking order may be, from the 5' end to the 3' end, first polynucleotide-third polynucleotide-second polynucleotide, or from the 5' end to the 3' end, second polynucleotide-third polynucleotide-first polynucleotide. In some specific embodiments, the amino acid sequence encoded by the third polynucleotide is set forth in any one of SEQ ID NOs: 43-46, for example, the sequence of the third polynucleotide is set forth in any one of SEQ ID NOs: 47-50 or has at least 80%, at least 90%, at least 95%, or at least 98% identity thereto.

In some embodiments, the polynucleotide encoding the AADC protein and the polynucleotide encoding the GDNF protein are regulated and expressed by the same regulatory element, or regulated and expressed by two different regulatory elements, respectively. The regulatory element includes, but is not limited to, 5' ITR, 3' ITR, a promoter, an enhancer, an intron, a polyA, a post-transcriptional regulatory element, and the like.

In some embodiments, the nucleic acid molecule comprises, from the 5' end to the 3' end:
a) a CMV enhancer, a CBA promoter, a first polynucleotide encoding AADC protein, a second polynucleotide encoding GDNF protein, and a polyA;
   in some optional embodiments, a hybrid intron is further comprised between the CBA promoter and the first polynucleotide encoding the AADC protein;
   in some optional embodiments, a WPRE sequence is further comprised between the second polynucleotide encoding the GDNF protein and the polyA;
   in some specific embodiments, the nucleic acid molecule comprises: a CMV enhancer, a CBA promoter, a hybrid intron, a first polynucleotide encoding AADC protein, a second polynucleotide encoding GDNF protein, a WPRE sequence, and a polyA;
b) a CMV enhancer, a CBA promoter, a second polynucleotide encoding GDNF protein, a first polynucleotide encoding AADC protein, and a polyA;
   in some optional embodiments, a hybrid intron is further comprised between the CBA promoter and the second polynucleotide encoding the GDNF protein;
   in some optional embodiments, a WPRE sequence is further comprised between the first polynucleotide encoding the AADC protein and the polyA;
   in some specific embodiments, the nucleic acid molecule comprises: a CMV enhancer, a CBA promoter, a hybrid intron, a second polynucleotide encoding GDNF protein, a first polynucleotide encoding AADC protein, a WPRE sequence, and a polyA;
c) a CMV enhancer, a CBA promoter, a first polynucleotide encoding AADC protein, a polyA, a CMV enhancer, a CMV promoter, a second polynucleotide encoding GDNF protein, and a polyA;
   in some optional embodiments, a hybrid intron is further comprised between the CBA promoter and the first polynucleotide encoding the AADC protein;
   in some optional embodiments, a β globin intron is further comprised between the CMV promoter and the second polynucleotide encoding the GDNF protein;
   in some optional embodiments, a WPRE sequence is further comprised between the second polynucleotide encoding the GDNF protein and the polyA;
   in some specific embodiments, the nucleic acid molecule comprises:
      CMV enhancer 1, a CBA promoter, a hybrid intron, a first polynucleotide encoding AADC protein, a polyA, CMV enhancer 2, a CMV promoter, a second polynucleotide encoding GDNF protein, and a polyA; or
      CMV enhancer 1, a CBA promoter, a hybrid intron, a first polynucleotide encoding AADC protein, a polyA, CMV enhancer 2, a CMV promoter, a second polynucleotide encoding GDNF protein, a WPRE sequence, and a polyA; or
      CMV enhancer 1, a CBA promoter, a hybrid intron, a first polynucleotide encoding AADC protein, a polyA, CMV enhancer 2, a CMV promoter, a β globin intron, a second polynucleotide encoding GDNF protein, and a polyA;
   d) a CMV enhancer, a CMV promoter, a second polynucleotide encoding GDNF protein, a polyA, a CMV enhancer, a CBA promoter, a first polynucleotide encoding AADC protein, and a polyA;
      in some optional embodiments, a β globin intron or an MVM intron is further comprised between the CMV promoter and the second polynucleotide encoding the GDNF protein; in some optional embodiments, a hybrid intron is further comprised between the CBA promoter and the first polynucleotide encoding the AADC protein;
      in some optional embodiments, a WPRE sequence is further comprised between the first polynucleotide encoding the AADC protein and the polyA;
      in some specific embodiments, the nucleic acid molecule comprises:
         CMV enhancer 2, a CMV promoter, a β globin intron, a second polynucleotide encoding GDNF protein, a polyA, CMV enhancer 1, a CBA promoter, a hybrid intron, a first polynucleotide encoding AADC protein, and a polyA;
         CMV enhancer 2, a CMV promoter, a second polynucleotide encoding GDNF protein, a polyA, CMV enhancer 1, a CBA promoter, a hybrid intron, a first polynucleotide encoding AADC protein, a WPRE sequence, and a polyA; or
         CMV enhancer 2, a CMV promoter, an MVM intron, a second polynucleotide encoding GDNF protein, a polyA, CMV enhancer 1, a CBA promoter, a hybrid intron, a first polynucleotide encoding AADC protein, a WPRE sequence, and a polyA;
   e) a CMV enhancer, a CBA promoter, a first polynucleotide encoding AADC protein, a third polynucleotide, a second polynucleotide encoding GDNF protein, and a polyA;
      in some optional embodiments, a hybrid intron is further comprised between the CBA promoter and the first polynucleotide encoding the AADC protein;
      in some optional embodiments, a WPRE sequence is further comprised between the second polynucleotide encoding the GDNF protein and the polyA;
      in some specific embodiments, the nucleic acid molecule comprises: a CMV enhancer, a CBA promoter, a hybrid intron, a first polynucleotide encoding AADC protein, a third polynucleotide, a second polynucleotide encoding GDNF protein, a WPRE sequence, and a polyA;
   f) a CMV enhancer, a CBA promoter, a second polynucleotide encoding GDNF protein, a third polynucleotide, a first polynucleotide encoding AADC protein, and a polyA;
      in some optional embodiments, a hybrid intron is further comprised between the CBA promoter and the second polynucleotide encoding the GDNF protein;
      in some optional embodiments, a WPRE sequence is further comprised between the first polynucleotide encoding the AADC protein and the polyA;
      in some specific embodiments, the nucleic acid molecule comprises: a CMV enhancer, a CBA promoter, a hybrid intron, a second polynucleotide encoding GDNF protein, a third polynucleotide, a first polynucleotide encoding AADC protein, a WPRE sequence, and a polyA;
   g) a CMV enhancer, a CBA promoter, a first polynucleotide or a second polynucleotide, and a polyA;
      in some optional embodiments, a hybrid intron is further comprised between the CBA promoter and the first polynucleotide, or a hybrid intron is further comprised between the CBA and the second polynucleotide;
      in some optional embodiments, a WPRE sequence is further comprised between the first polynucleotide and the polyA, or a WPRE sequence is further comprised between the second polynucleotide and the polyA;
      in some specific embodiments, the nucleic acid molecule comprises: a CMV enhancer, a CBA promoter, a hybrid intron, a first polynucleotide, and a polyA;
      a CMV enhancer, a CBA promoter, a hybrid intron, a second polynucleotide, and a polyA;
      a CMV enhancer, a CBA promoter, a hybrid intron, a first polynucleotide, a WPRE sequence, and a polyA; or
      a CMV enhancer, a CBA promoter, a hybrid intron, a second polynucleotide, a WPRE sequence, and a polyA.

In some embodiments, the polyA is selected from the group consisting of hGH polyA, PA75 polyA, or SV40 polyA.

In some embodiments, provided is a polynucleotide, and the nucleic acid molecule comprises, from the 5' end to the 3' end:
(1) CMV enhancer-CBA promoter-hybrid intron-first polynucleotide encoding AADC-third polynucleotide-second polynucleotide encoding GDNF-WPRE-SV40 polyA;
(2) CMV enhancer-CBA promoter-hybrid intron-second polynucleotide encoding GDNF-third polynucleotide-first polynucleotide encoding AADC-WPRE-SV40 polyA;
(3) CMV enhancer-CBA promoter-hybrid intron-first polynucleotide encoding AADC-SV40 polyA-CMV enhancer-CMV promoter-second polynucleotide encoding GDNF-PA75 polyA;
(4) CMV enhancer-CBA promoter-hybrid intron-first polynucleotide encoding AADC-SV40 polyA-CMV enhancer-CMV promoter-second polynucleotide encoding GDNF-WPRE-PA75 polyA;
(5) CMV enhancer-CBA promoter-hybrid intron-first polynucleotide encoding AADC-SV40 polyA-CMV enhancer-CMV promoter-β globin intron-second polynucleotide encoding GDNF-PA75 polyA;
(6) CMV enhancer-CMV promoter-β globin intron-second polynucleotide encoding GDNF-PA75 poly A-CMV enhancer-CBA promoter-hybrid intron-first polynucleotide encoding AADC-SV40 polyA;
(7) CMV enhancer-CMV promoter-second polynucleotide encoding GDNF-PA75 poly A-CMV enhancer-CBA promoter-hybrid intron-first polynucleotide encoding AADC-WPRE-SV40 polyA; or
(8) CMV enhancer-CMV promoter-MVM intron-second polynucleotide encoding GDNF-PA75 poly A-CMV enhancer-CBA promoter-hybrid intron-first polynucleotide encoding AADC-WPRE-SV40 polyA.

In some embodiments, provided is a polynucleotide comprising, in the direction from 5' to 3', 1)-13) as follows or any combination thereof
1) 5' ITR, CMV enhancer 1, a CBA promoter, a hybrid intron, a polynucleotide encoding AADC or a polynucleotide encoding GDNF, hGH polyA, and 3' ITR;
2) 5' ITR, CMV enhancer 1, a CBA promoter, a hybrid intron, a polynucleotide encoding AADC or a polynucleotide encoding GDNF, SV40 polyA, and 3' ITR;
3) 5' ITR, CMV enhancer 1, a CBA promoter, a hybrid intron, a polynucleotide encoding AADC or a polynucleotide encoding GDNF, WPRE, SV40 polyA, and 3' ITR;
4) 5' ITR, CMV enhancer 1, a CBA promoter, a hybrid intron, a polynucleotide encoding AADC, P2A, a polynucleotide encoding GDNF, WPRE, SV40 polyA, and 3' ITR;
5) 5' ITR, CMV enhancer 1, a CBA promoter, a hybrid intron, a polynucleotide encoding GDNF, P2A, a polynucleotide encoding AADC, WPRE, SV40 polyA, and 3' ITR;
6) 5' ITR, MCS, CMV enhancer 1, a CBA promoter, a hybrid intron, a polynucleotide encoding AADC, SV40 polyA, CMV enhancer 2, a CMV promoter, a polynucleotide encoding GDNF, PA75 polyA, and 3' ITR;
7) 5' ITR, MCS, CMV enhancer 1, a CBA promoter, a hybrid intron, a polynucleotide encoding AADC, SV40 polyA, CMV enhancer 2, a CMV promoter, a polynucleotide encoding GDNF, WPRE, PA75 polyA, and 3' ITR;
8) 5' ITR, MCS, CMV enhancer 1, a CBA promoter, a hybrid intron, a polynucleotide encoding AADC, SV40 polyA, CMV enhancer 2, a CMV promoter, a β globin intron, a polynucleotide encoding GDNF, PA75 polyA, and 3' ITR;
9) 5' ITR, CMV enhancer 2, a CMV promoter, a β globin intron, a polynucleotide encoding GDNF, PA75 polyA, CMV enhancer 1, a CBA promoter, a hybrid intron, a polynucleotide encoding AADC, SV40 polyA, and 3' ITR;
10) 5' ITR, CMV enhancer 2, a CMV promoter, a polynucleotide encoding GDNF, PA75 polyA, CMV enhancer 1, a CBA promoter, a hybrid intron, a polynucleotide encoding AADC, WPRE, SV40 polyA, and 3' ITR;
11) 5' ITR, CMV enhancer 2, a CMV promoter, an MVM intron, a polynucleotide encoding GDNF, PA75 polyA, CMV enhancer 1, a CBA promoter, a hybrid intron, a polynucleotide encoding AADC, WPRE, SV40 polyA, and 3' ITR;
12) 5' ITR, CMV enhancer 1, a CBA promoter, a hybrid intron, a polynucleotide encoding AADC, P2A, a polynucleotide encoding GDNF, WPRE, SV40 polyA, and 3' ITR; and
13) 5' ITR, CMV enhancer 2, a CMV promoter, a β globin intron, a polynucleotide encoding GDNF, PA75 polyA, CMV enhancer 1, a CBA promoter, a hybrid intron, a polynucleotide encoding AADC, SV40 polyA, and 3' ITR.

In some specific embodiments,
the CMV enhancer 1 comprises or is the sequence set forth in SEQ ID NO: 27;
the CMV enhancer 2 comprises or is the sequence set forth in SEQ ID NO: 37;
the CBA promoter comprises or is the sequence set forth in SEQ ID NO: 28;
the CMV promoter comprises or is the sequence set forth in SEQ ID NO: 35;
the β globin intron comprises or is the sequence set forth in SEQ ID NO: 38;
the hybrid intron comprises or is the sequence set forth in SEQ ID NO: 29;
the MVM intron comprises or is the sequence set forth in SEQ ID NO: 39;
the PA75 polyA comprises or is the sequence set forth in SEQ ID NO: 36;
the SV40 polyA comprises or is the sequence set forth in SEQ ID NO: 32;
the hGH polyA comprises or is the sequence set forth in SEQ ID NO: 30;
the HPRE comprises or is the sequence set forth in SEQ ID NO: 33;
the WPRE comprises or is the sequence set forth in SEQ ID NO: 34;
the 5' ITR comprises or is the sequence set forth in SEQ ID NO: 26;
the 3' ITR comprises or is the sequence set forth in SEQ ID NO: 31;
the MCS comprises or is the sequence set forth in SEQ ID NO: 40; and/or
the P2A comprises or is the sequence set forth in SEQ ID NO: 33.

In some embodiments, provided is a nucleic acid molecule comprising a polynucleotide, wherein the polynucleotide has at least 95% identity to the sequence set forth in any one of SEQ ID NOs: 13-25. In some embodiments, the polynucleotide comprises the sequence set forth in any one of SEQ ID NOs: 13-25.

In some embodiments, the AADC protein (e.g., AADC protein set forth in SEQ ID NO: 1) comprises a variant (e.g., the variant has at least 60%, at least 70%, at least 80%, at least 90%, at least 95% or more identity to SEQ ID NO: 1, NP_000781.2, NP_001076440.2, NP_001229815.2, NP_001229816.2, NP_001229817.2, NP_001229818.2, or NP_001229819.2) or a fragment thereof, the variant or the fragment having the same or similar biological activity or functions as the AADC protein; the GDNF protein (e.g., GDNF protein set forth in SEQ ID NO: 2) comprises a variant (e.g., the variant has at least 60%, at least 70%, at least 80%, at least 90%, at least 95% or more identity to SEQ ID NO: 2, NP_001177397.1, NP_001177398.1, NP_001265027.1, or NP_954701.1) or a fragment thereof, the variant or the fragment having the same or similar biological activity or functions as the GDNF protein.

In some embodiments, the polynucleotide comprises a precursor molecule that is processed inside a cell. The AADC and/or GDNF polynucleotides or processed forms thereof may be encoded in a plasmid, a vector, a genome, or other polynucleotide expression vectors for delivery to a cell.

In some embodiments, the vector comprises an intact replicon, such that, for example, upon transfection, infection, or transformation of a cell, the vector can be replicated in the cell. In some embodiments, the vector is or is derived from a retrovirus, adenovirus, herpesvirus, baculovirus, papillomavirus, or modified or engineered viruses thereof. In some embodiments, methods for delivering the vector include, but are not limited to, direct delivery of naked DNA or delivery via cationic or anionic liposomes, via complexation with cationic polymers, via forming complexes with proteins or polypeptides.

In some embodiments, the polynucleotide is designed as a component of an AAV vector genome (or viral genome) and packaged in rAAV particles that are processed in a cell to produce AADC and/or GDNF proteins, and the AADC and/or GDNF proteins may be wild-type proteins or variants thereof.

In some embodiments, the polynucleotide may be a payload of the rAAV particles.

### AAV capsid and AAV particle

The polynucleotide according to any one of the foregoing of the present disclosure may be wrapped using an AAV capsid to form an AAV particle.

The AAV capsid comprises or is derived from any natural or recombinant AAV serotype, including but not limited to: PHP.B, PHP.A, AAV1, AAV2, AAV2G9, AAV3, AAV3a, AAV3b, AAV3-3, AAV4, AAV4-4, AAV5, AAV6, AAV6.1, AAV6.2, AAV6.1.2, AAV7, AAV7.2, AAV8, AAV9, AAV9.11, AAV9.13, AAV9.16, AAV9.24, AAV9.45, AAV9.47, AAV9.61, AAV9.68, AAV9.84, AAV9.9, AAV10, AAV11, AAV12, AAV16.3, AAV24.1, AAV27.3, AAV42.12, AAV42-1b, AAV42-2, AAV42-3a, AAV42-3b, AAV42-4, AAV42-5a, AAV42-5b, AAV42-6b, AAV42-8, AAV42-10, AAV42-11, AAV42-12, AAV42-13, AAV42-15, AAV42-aa, AAV43-1, AAV43-12, AAV43-20, AAV43-21, AAV43-23, AAV43-25, AAV43-5, AAV44.1, AAV44.2, AAV44.5, AAV223.1, AAV223.2, AAV223.4, AAV223.5, AAV223.6, AAV223.7, AAV1-7/rh.48, AAV1-8/rh.49, AAV2-15/rh.62, AAV2-3/rh.61, AAV2-4/rh.50, AAV2-5/rh.51, AAV3.1/hu.6, AAV3.1/hu.9, AAV3-9/rh.52, AAV3-11/rh.53, AAV4-8/r11.64, AAV4-9/rh.54, AAV4-19/rh.55, AAV5-3/rh.57, AAV5-22/rh.58, AAV7.3/hu.7, AAV16.8/hu.10, AAV16.12/hu.11, AAV29.3/bb.1, AAV29.5/bb.2, AAV106.1/hu.37, AAV114.3/hu.40, AAV127.2/hu.41, AAV127.5/hu.42, AAV128.3/hu.44, AAV130.4/hu.48, AAV145.1/hu.53, AAV145.5/hu.54, AAV145.6/hu.55, AAV161.10/hu.60, AAV161.6/hu.61, AAV33.12/hu.17, AAV33.4/hu.15, AAV33.8/hu.16, AAV52/hu.19, AAV52.1/hu.20, AAV58.2/hu.25, AAVA3.3, AAVA3.4, AAVA3.5, AAVA3.7, AAVC1, AAVC2, AAVC5, AAV-DJ, AAV-DJ8, AAVF3, AAVF5, AAVH2, AAVrh.72, AAVhu.8, AAVrh.68, AAVrh.70, AAVpi.1, AAVpi.3, AAVpi.2, AAVrh.60, AAVrh.44, AAVrh.65, AAVrh.55, AAVrh.47, AAVrh.69, AAVrh.45, AAVrh.59, AAVhu.12, AAVH6, AAVLK03, AAVH-1/hu.1, AAVH-5/hu.3, AAVLG-10/rh.40, AAVLG-4/rh.38, AAVLG-9/hu.39, AAVN721-8/rh.43, AAVCh.5, AAVCh.5R1, AAVcy.2, AAVcy.3, AAVcy.4, AAVcy.5, AAVCy.5R1, AAVCy.5R2, AAVCy.5R3, AAVCy.5R4, AAVcy.6, AAVhu.1, AAVhu.2, AAVhu.3, AAVhu.4, AAVhu.5, AAVhu.6, AAVhu.7, AAVhu.9, AAVhu.10, AAVhu.11, AAVhu.13, AAVhu.15, AAVhu.16, AAVhu.17, AAVhu.18, AAVhu.20, AAVhu.21, AAVhu.22, AAVhu.23.2, AAVhu.24, AAVhu.25, AAVhu.27, AAVhu.28, AAVhu.29, AAVhu.29R, AAVhu.31, AAVhu.32, AAVhu.34, AAVhu.35, AAVhu.37, AAVhu.39, AAVhu.40, AAVhu.41, AAVhu.42, AAVhu.43, AAVhu.44, AAVhu.44R1, AAVhu.44R2, AAVhu.44R3, AAVhu.45, AAVhu.46, AAVhu.47, AAVhu.48, AAVhu.48R1, AAVhu.48R2, AAVhu.48R3, AAVhu.49, AAVhu.51, AAVhu.52, AAVhu.54, AAVhu.55, AAVhu.56, AAVhu.57, AAVhu.58, AAVhu.60, AAVhu.61, AAVhu.63, AAVhu.64, AAVhu.66, AAVhu.67, AAVhu.68, AAVhu.14/9, AAVhu.t19, AAVrh.2, AAVrh.2R, AAVrh.8, AAVrh.8R, AAVrh.10, AAVrh.12, AAVrh.13, AAVrh.13R, AAVrh.14, AAVrh.17, AAVrh.18, AAVrh.19, AAVrh.20, AAVrh.21, AAVrh.22, AAVrh.23, AAVrh.24, AAVrh.25, AAVrh.31, AAVrh.32, AAVrh.33, AAVrh.34, AAVrh.35, AAVrh.36, AAVrh.37, AAVrh.37R2, AAVrh.38, AAVrh.39, AAVrh.40, AAVrh.46, AAVrh.48, AAVrh.48.1, AAVrh.48.1.2, AAVrh.48.2, AAVrh.49, AAVrh.51, AAVrh.52, AAVrh.53, AAVrh.54, AAVrh.56, AAVrh.57, AAVrh.58, AAVrh.61, AAVrh.64, AAVrh.64R1, AAVrh.64R2, AAVrh.67, AAVrh.73, AAVrh.74, AAVrh8R, AAVrh8R A586R mutant, AAVrh8R R533A mutant, AAAV, BAAV, goat AAV, bovine AAV, ovine AAV, AAVhE1.1, AAVhEr1.5, AAVhER1.14, AAVhEr1.8, AAVhEr1.16, AAVhEr1.18, AAVhEr1.35, AAVhEr1.7, AAVhEr1.36, AAVhEr2.29, AAVhEr2.4, AAVhEr2.16, AAVhEr2.30, AAVhEr2.31, AAVhEr2.36, AAVhER1.23, AAVhEr3.1, AAV2.5T, AAV-PAEC, AAV-LK01, AAV-LK02, AAV-LK03, AAV-LK04, AAV-LK05, AAV-LK06, AAV-LK07, AAV-LK08, AAV-LK09, AAV-LK10, AAV-LK11, AAV-LK12, AAV-LK13, AAV-LK14, AAV-LK15, AAV-LK16, AAV-LK17, AAV-LK18, AAV-LK19, AAV-PAEC2, AAV-PAEC4, AAV-PAEC6, AAV-PAEC7, AAV-PAEC8, AAV-PAEC11, AAV-PAEC12, AAV-2-pre-miRNA-101, AAV-8h, AAV-8b, AAV-h, AAV-b, AAV SM 10-2, AAVShuffle 100-1, AAV Shuffle 100-3, AAV Shuffle 100-7, AAV Shuffle 10-2, AAVShuffle 10-6, AAV Shuffle 10-8, AAV Shuffle 100-2, AAV SM 10-1, AAV SM 10-8, AAVSM 100-3, AAV SM 100-10, BNP61 AAV, BNP62 AAV, BNP63 AAV, AAVrh.50, AAVrh.43, AAVrh.62, AAVrh.48, AAVhu.19, AAVhu.11, AAVhu.53, AAV4-8/rh.64, AAVLG-9/hu.39, AAV54.5/hu.23, AAV54.2/hu.22, AAV54.7/hu.24, AAV54.1/hu.21, AAV54.4R/hu.27, AAV46.2/hu.28, AAV46.6/hu.29, AAV128.1/hu.43, true type AAV (ttAAV), UPENNAAV 10, Japanese AAV 10 serotype, AAV CBr-7.1, AAV CBr-7.10, AAV CBr-7.2, AAV CBr-7.3, AAVCBr-7.4, AAV CBr-7.5, AAV CBr-7.7, AAV CBr-7.8, AAV CBr-B7.3, AAV CBr-B7.4, AAVCBr-E1, AAV CBr-E2, AAV CBr-E3, AAV CBr-E4, AAV CBr-E5, AAV CBr-e5, AAV CBr-E6, AAVCBr-E7, AAV CBr-E8, AAV CHt-1, AAV CHt-2, AAV CHt-3, AAV CHt-6.1, AAV CHt-6.10, AAVCHt-6.5, AAV CHt-6.6, AAV CHt-6.7, AAV CHt-6.8, AAV CHt-P 1, AAV CHt-P2, AAV CHt-P5, AAV CHt-P6, AAV CHt-P8, AAV CHt-P9, AAV CKd-1, AAV CKd-10, AAV CKd-2, AAV CKd-3, AAVCKd-4, AAV CKd-6, AAV CKd-7, AAV CKd-8, AAV CKd-B1, AAV CKd-B2, AAV CKd-B3, AAV CKd-B4, AAV CKd-B5, AAV CKd-B6, AAV CKd-B7, AAV CKd-B8, AAV CKd-H1, AAV CKd-H2, AAV CKd-H3, AAV CKd-H4, AAV CKd-H5, AAV CKd-H6, AAV CKd-N3, AAV CKd-N4, AAV CKd-N9, AAV CLg-F1, AAV CLg-F2, AAV CLg-F3, AAV CLg-F4, AAV CLg-F5, AAV CLg-F6, AAV CLg-F7, AAV CLg-F8, AAV CLv-1, AAV CLv1-1, AAV Clv1-10, AAV CLv1-2, AAV CLv-12, AAV CLv1-3, AAV CLv-13, AAV CLv1-4, AAV Clv1-7, AAV Clv1-8, AAV Clv1-9, AAV CLv-2, AAV CLv-3, AAV CLv-4, AAV CLv-6, AAV CLv-8, AAV CLv-D1, AAV CLv-D2, AAV CLv-D3, AAV CLv-D4, AAV CLv-D5, AAV CLv-D6, AAV CLv-D7, AAV CLv-D8, AAV CLv-E1, AAV CLv-K1, AAV CLv-K3, AAV CLv-K6, AAV CLv-L4, AAV CLv-L5, AAV CLv-L6, AAV CLv-M1, AAV CLv-M11, AAV CLv-M2, AAV CLv-M5, AAV CLv-M6, AAV CLv-M7, AAV CLv-M8, AAV CLv-M9, AAV CLv-R1, AAV CLv-R2, AAV CLv-R3, AAV CLv-R4, AAV CLv-R5, AAV CLv-R6, AAV CLv-R7, AAV CLv-R8, AAV CLv-R9, AAV CSp-1, AAV CSp-10, AAV CSp-11, AAV CSp-2, AAV CSp-3, AAV CSp-4, AAV CSp-6, AAV CSp-7, AAVCSp-8, AAV CSp-8.10, AAV CSp-8.2, AAV CSp-8.4, AAV CSp-8.5, AAV CSp-8.6, AAV CSp-8.7, AAV CSp-8.8, AAV CSp-8.9, AAV CSp-9, AAVhu.48R3, AAVVR-355, AAV3B, AAV4, AAV5, AAVF1/HSC1, AAVF11/HSC11, AAVF12/HSC12, AAVF13/HSC13, AAVF14/HSC14, AAVF15/HSC15, AAVF16/HSC16, AAVF17/HSC17, AAVF2/HSC2, AAVF3/HSC3, AAVF4/HSC4, AAVF5/HSC5, AAVF6/HSC6, AAVF7/HSC7, AAVF8/HSC8, AAVF9/HSC9, PHP.B(AAV-PHP.B), PHP. A(AAVPHP. A), G2B-26, G2B-13, TH1.1-32, TH1.1-35, AAVPHPB2, AAVPHPB3, AAVPHP.N/PHP.B-DGT, AAVPHP.B-EST, AAVPHPB-GGT, AAVPHPB-ATP, AAVPHP.B-ATT-T, AAVPHP.B-DGT-T, AAVPHPB-GGT-T, AAVPHP.B-SGS, AAVPHPB-AQP, AAVPHPB-QQP, AAVPHPB-SNP(3), AAVPHPB-SNP, AAVPHP.B-QGT, AAVPHP.B-NQT, AAVPHPB-EGS, AAVPHP.B-SGN, AAVPHP.B-EGT, AAVPHPB-DST, AAVPHPB-DST, AAVPHPB-STP, AAVPHPB-PQP, AAVPHP.B-SQP, AAVPHPB-QLP, AAVPHP.B-TMP, AAVPHPB-TTP, AAVPHP.S/G2A12, AAVG2A15/G2A3, AAVG2B4, AAVG2B5, AAVDJ8, and variants thereof.

In some embodiments, the AAV capsid may be modified or mutated, for example, containing one or more mutations of Y252F, Y272F, Y444F, Y500F, Y700F, Y704F, Y730F, Y275F, Y281F, Y508F, Y576F, Y612G, Y673F, and Y720F; for example, containing one or more mutations of F129L, D418E, K531E, L584F, V598A, and H642N; for example, containing substitution of at least one of Tyr residues at positions 252, 272, 444, 500, 700, 704, and 730 of AAV2 with, for example, a Phe residue; for example, containing an N587A, E548A, or N708A mutation in the AAV2 capsid; for example, containing a T446F mutation in the AAV9 capsid; for example, containing a V708K mutation in the AAV capsid; for example, the AAV capsid is generated via an AAV9 capsid library having mutations in amino acids at positions 390-627 (VP1 numbering).

The AAV capsids in WO2018232055 (e.g. Table 1 thereof), WO2005033321, WO2015168666, WO2015121501, WO2015038958, WO2016065001, WO2016130589, WO2016049230, WO2016134375, WO2017100671, WO2017083722, WO2017015102, WO2017058892, WO2017066764, US9546112, US7198951, US9233131, US6156303, US9624274, US9475845, US8734809, US20130224836, US20140359799, US20150315612, US20150376240, US20150159173, US20150376607, US20150238550, US20160369298, US20160361439, US20170145405, and NPulicherla et al., (MolecularTherapy19(6):1070-1078(2011)) are incorporated in the present disclosure by reference in their entirety. In some specific embodiments, the AAV capsids used in the present disclosure are the AAV2 and AAV9 capsids in the prior art described above. In some embodiments, the AAV capsid is engineered, e.g., is a hybrid AAV capsid from two or more parental serotypes. For example, the AAV capsid may be AAV2G9, which comprises sequences from AAV2 and AAV9. The sequence of AAV2G9 in US20160017005 is incorporated herein by reference in its entirety.

In some embodiments, the AAV capsid is an AAV2 or AAV9 capsid, e.g., comprising the amino acid sequence set forth in SEQ ID NO: 41 or 42.

In some embodiments, the AAV particle may comprise all or part of the vector genome of a nucleotide sequence or a variant of any naturally occurring and/or recombinant AAV capsid, in addition to the encoded heterologous payload.

In some embodiments, the AAV particle may be replication-deficient (e.g., it lacks sequences encoding functional Rep and Cap proteins in the vector genome). In some specific embodiments, the replication-deficient AAV particle may lack most or all of the parental coding sequences, and carry essentially only one or two AAV ITR sequences and a polynucleotide of interest for delivery to a cell, tissue, organ, or organism.

In some embodiments, the AAV particle may be a recombinant AAV (rAAV) particle.

In some embodiments, the AAV particle may be selected from the group consisting of a single-stranded AAV particle (e.g., ssAAV) and a self-complementary AAV particle (e.g., scAAV). By skipping the synthesis of a second strand, scAAV achieves rapid expression in cells.

In some embodiments, the AAV particle wraps a polynucleotide having at least 95% identity to the sequence set forth in any one of SEQ ID NOs: 13-25.

In some embodiments, the AAV particle wraps a polynucleotide comprising the sequence set forth in any one of SEQ ID NOs: 13-25.

### Regulatory element

The vector genome (or viral genome) of the polynucleotide or AAV particle according to any one of the foregoing of the present disclosure comprises at least one regulatory element, such that a target gene (e.g., AADC and GDNF) in a payload can be replicated, transcribed, and translated.

In some embodiments, the regulatory element includes, but is not limited to, sequences for transcription initiation and/or termination, promoter and/or enhancer sequences, efficient RNA processing signals (e.g., splicing and polyadenylation signals), sequences that stabilize cytoplasmic mRNA, sequences that enhance translation efficiency (e.g., Kozak consensus sequences), sequences that enhance protein stability, and/or sequences that enhance protein processing and/or secretion. Exemplary regulatory elements include, but are not limited to, promoters, enhancers, introns, endogenous miRNAs, post-transcriptional regulatory elements (PREs), polyadenylation (PolyA) signal sequences, and upstream enhancers (USEs).

In some embodiments, the regulatory elements described below (e.g., promoters) drive expression of the payload in a target tissue, e.g., the brain (e.g., striatum or substantia nigra) of a subject, for a period of time, for example, for 1 day, 2 days, 3 days, 4 days, 5 days, 6 days, 7 days, 1 week, 2 weeks, 3 weeks, 4 weeks, 1 month, 2 months, 3 months, 4 months, 5 months, 6 months, 7 months, 8 months, 9 months, 10 months, 11 months, 12 months, 1 year, 2 years, 3 years, 4 years, 5 years, 6 years, 7 years, 8 years, 9 years, 10 years, 20 years, 30 years, 40 years, 50 years, 60 years or more, for another example, for 1-2 weeks, 1-3 weeks, 1-4 weeks, 1-2 months, 1-4 months, 1-6 months, 2-6 months, 3-6 months, 3-9 months, 4-8 months, 6-12 months, 1-2 years, 1-5 years, 2-5 years, 3-6 years, 3-8 years, 4-8 years, 5-10 years, 10-20 years, 10-30 years, 20-40 years, 20-50 years, or the lifetime of the subject.

### Inverted terminal repeat (ITR)

The vector genome (or viral genome) of the polynucleotide or AAV particle according to any one of the foregoing of the present disclosure comprises at least one ITR, e.g., has two ITRs flanking a payload at the 5' end and the 3' end, respectively.

In some embodiments, the ITRs have origin of replication function.

In some embodiments, the ITRs comprise sequence regions that may be complementary and symmetrically arranged.

In some embodiments, the ITRs may consist of naturally occurring polynucleotide sequences or recombinantly derived polynucleotide sequences.

In some embodiments, the ITRs may be derived from the same or different serotype as the capsid. In some embodiments, the 5' ITR and the 3' ITR may be derived from the same serotype or may be derived from different serotypes. For example, both 5' ITR and 3' ITR are from AAV2, or both are from AAV9. For another example, the 5' ITR comprises the sequence set forth in SEQ ID NO: 26, and/or the 3' ITR comprises the nucleotide sequence set forth in SEQ ID NO: 31.

In some embodiments, the length of each ITR may be about 100 to about 150 nucleotides, such as 100-105 nucleotides, 106-110 nucleotides, 111-115 nucleotides, 116-120 nucleotides, 121-125 nucleotides, 126-130 nucleotides, 131-135 nucleotides, 136-140 nucleotides, 141-145 nucleotides, or 146-150 nucleotides. In one embodiment, the length of the ITR is 140-142 nucleotides, such as 141 nucleotides. Non-limiting examples of the length of the ITR are 102 nucleotides, 140 nucleotides, 141 nucleotides, 142 nucleotides, 145 nucleotides, and nucleotides having at least 95% identity thereto.

### Promoter

The vector genome (or viral genome) of the polynucleotide or AAV particle according to any one of the foregoing of the present disclosure comprises at least one promoter, including but not limited to, species-specific promoters, inducible promoters, tissue-specific promoters, or cell cycle-specific promoters.

In some embodiments, the promoter drives expression of a protein or polypeptide (e.g., AADC and/or GDNF) encoded in a payload of the vector genome of the AAV particle. In some embodiments, the promoter is specific to or has tropism for a target tissue, such as a promoter capable of expressing a payload in a neural tissue.

In some embodiments, the promoter may be a viral promoter, a plant promoter, a mammalian promoter, or a human promoter.

In some embodiments, the promoter includes, but is not limited to, CMV, CBA (including derivatives CAG, CBh, etc.), EF-1α, PGK, UBC, RSV, EFS, EF1, GUSB (hGBp), UCOE (the promoter of HNRPA2B1-CBX3), NSE, Synapsin, MeCP2, MeP418, MeP426, VMD2, MRHO, TRE, Ac5, Polyhedrin, CaMKIIa, Gall, TEF1, GDS, ADHl, Ubi, GFAP, or PKG promoters, and may be selected from the group consisting of neurofilament light (NFL) promoters, neurofilament heavy (NFH) promoters, SCN8A promoters, frataxin (FXN) promoters (or known as FRDA promoters), H1 promoters, RNA pol III promoters (e.g., U6 or H1), and small nuclear RNA (ULB or ULA) promoters. In some embodiments, the promoter is a liver or skeletal muscle promoter; the liver promoter is, for example, human α-1-antitrypsin (hAAT) and thyroxine-binding globulin (TBG), and the skeletal muscle promoter is, for example, desmin, MCK, or synthetic C5-12.

In some embodiments, the AAV vector genome of the present disclosure comprises two promoters, for example, CMV and CBA promoters, the sequences of which are set forth in SEQ ID NOs: 35 and 28, respectively; for another example, an EF1α promoter and a CMV promoter, or a combination of any two promoters of the foregoing promoters.

In some embodiments, the promoter is a tissue-specific expression element that can limit expression to certain cell types, including but not limited to, a muscle-specific promoter, a B cell promoter, a monocyte promoter, a leukocyte promoter, a macrophage promoter, a pancreatic acinar cell promoter, an endothelial cell promoter, a lung tissue promoter, an astrocyte promoter, or a nervous system promoter, which can be used to limit expression to neurons, astrocytes, or oligodendrocytes.

In some embodiments, the promoter is a neuronal tissue-specific expression element, including but not limited to, neuron-specific enolase (NSE), platelet-derived growth factor (PDGF), platelet-derived growth factor B chain (PDGF-β), synapsin (Syn), methyl CpG binding protein 2 (MeCP2), Ca²⁺/calmodulin-dependent protein kinase II (CaMKII), metabotropic glutamate receptor 2 (mGluR2), neurofilament light (NFL) or heavy (NFH), β-globin minigene nβ2, preproenkephalin (PPE), enkephalin (Enk), and excitatory amino acid transporter 2 (EAAT2) promoters. Non-limiting examples of tissue-specific expression elements for astrocytes include glial fibrillary acidic protein (GFAP) and EAAT2 promoters. A non-limiting example of tissue-specific expression elements for oligodendrocytes includes a myelin basic protein (MBP) promoter.

In some embodiments, the promoter is a truncation or a variant of the foregoing promoters, having a length of less than 1 kb, such as 200-300, 200-400, 300-400, 200-500, 200-600 or more.

In some embodiments, the promoter may be a combination of two or more components of the same or different initial or parental promoters, such as CMV and CBA.

### Enhancer

The vector genome (or viral genome) of the polynucleotide or AAV particle according to any one of the foregoing of the present disclosure comprises at least one enhancer, including but not limited to, species-specific enhancers, inducible enhancers, tissue-specific enhancers, or cell cycle-specific enhancers.

In some embodiments, the enhancer is specific to or has tropism for a target tissue, such as an enhancer capable of regulating expression of a payload in a neural tissue.

In some embodiments, the enhancer may be or be derived from a viral enhancer, a plant enhancer, a mammalian enhancer, or a human enhancer.

In some embodiments, the enhancer may be located upstream or downstream of a promoter and operably linked to the promoter. Expression of a target gene (e.g., AADC and/or GDNF) is enhanced by at least 20%, at least 50%, at least 80%, at least 100%, or at least several times (including 1, 2, 3, 4, 5, 6, 7, 8, 9, 10 times or more) when the enhancer is present as compared to when the enhancer is not present.

In some embodiments, the enhancer includes, but is not limited to, EF-1α, Ubc, humanb-actin, CAG, TRE, Ac5, Polyhedrin, CaMKIIa, Gall, TEF1, GDS, ADH1, Ubi, and α-1-antitrypsin (hAAT), or variants or fragments of the above enhancers. In some embodiments, the enhancer is selected from the group consisting of IRBP, RSV, or CMV enhancers, or variants or fragments thereof.

In some embodiments, the enhancer comprises or is an enhancer set forth in SEQ ID NO: 27 or 37 or a variant or a fragment thereof.

### Intron

An intron or a portion thereof may be comprised in the vector genome (or viral genome) of the polynucleotide or AAV particle according to any one of the foregoing of the present disclosure.

In some embodiments, the intron includes, but is not limited to, MVM (67-97 bp), F.IX truncated intron 1 (about 300 bp), β-globin SD/immunoglobulin heavy chain splice acceptor (about 250 bp), adenovirus splice donor/immunoglobulin splice acceptor (about 500 bp), SV40 late splice donor/splice acceptor (19S/16S) (180 bp) and hybrid adenovirus splice donor/IgG splice acceptor (about 230 bp), hemoglobin introns, hybrid introns, β-globin introns, or variants or fragments of the above introns.

In some embodiments, the length of the intron is 100-800 nucleotides, such as about 100 nucleotides, about 200 nucleotides, about 300 nucleotides, about 400 nucleotides, about 500 nucleotides, and about 600 nucleotides, and further such as 200-250 nucleotides, 200-300 nucleotides, 100-200 nucleotides, 300-500 nucleotides, 500-600 nucleotides, 400-600 nucleotides, and 100-200 nucleotides.

In some embodiments, the intron in the technical solutions of the present disclosure is selected from the group consisting of a hybrid intron and a β-globin intron, the sequences of which are set forth in SEQ ID NO: 29 and 38, respectively.

### Untranslated region (UTR)

An untranslated region (UTR) may be comprised in the vector genome (or viral genome) of the polynucleotide or AAV particle according to any one of the foregoing of the present disclosure, wherein the UTR is selected from the group consisting of a 5' UTR and/or a 3' UTR, to regulate (e.g., increase or decrease) polynucleotide stability and protein production. Typically, the 5' UTR starts at a transcription start site and terminates at a start codon, and the 3' UTR starts immediately after a stop codon and terminates at a transcription termination signal. The UTR may be a wild type, a variant thereof, or an artificial UTR.

In some embodiments, the 5' UTR comprises a Kozak sequence. In other embodiments, the 5' UTR does not comprise a Kozak sequence.

In some embodiments, the 3' UTR is rich in AU. In some embodiments, the 3' UTR is selected from the group consisting of: class I AREs, for example, but not limited to, c-Myc and MyoD, containing several dispersed copies of AUUUA motifs within the U-rich region; class II AREs, for example, but not limited to, GM-CSF and TNF-a, possessing two or more overlapping UUAUUUA(U/A)(U/A) nonamers; and class III ARES, for example, but not limited to, c-Jun and myogenin.

In some embodiments, the 3' UTR may comprise an oligomeric (dT) sequence for templated addition of a polyadenylation sequence (PolyA).

### Polyadenylation sequence (PolyA)

A sequence encoding a PolyA may be comprised in the vector genome (or viral genome) of the polynucleotide or AAV particle according to any one of the foregoing of the present disclosure, wherein the PolyA may be a wild type, a variant thereof, or a modified PolyA, for protein translation.

In some embodiments, the sequence encoding the PolyA is located between the 3' end of a coding sequence of a payload and the 5' end of a 3' ITR.

In some embodiments, the length of the sequence encoding the PolyA is 0-500 nucleotides, such as about 75 nucleotides, about 100 nucleotides, about 110 nucleotides, about 120 nucleotides, about 150 nucleotides, about 160 nucleotides, about 200 nucleotides, or about 300 nucleotides, further such as 50-100, 50-150, 50-160, 50-200, 60-100, 60-150, 60-160, 60-200, 70-100, 70-150, 70-160, 70-200, 80-100, 80-150, 80-160, 80-200, 90-100, 90-150, 90-160, or 90-200.

In some embodiments, the encoding PolyA may encode continuous PolyA or discontinuous PolyA. When the discontinuous PolyA is encoded, the coding sequence may be interrupted or separated by other nucleotides. For example, the polyA has at least two 60-adenylate fragments that are separated by a sequence comprising 10-90 nucleotides.

In some embodiments, the sequence encoding the PolyA or polyA in the technical solutions of the present disclosure is selected from the group consisting of β-globin polyA, SV40 polyA, bGH polyA, PA75 polyA, MeCP2 polyA, RDH1 polyA, BGH polyA, SPA49 polyA, sNRP-TK65 polyA, sNRP polyA, TK65 polyA, or variants or fragments of the above PolyA.

In some embodiments, the polyA set forth in SEQ ID NOs: 32 and 36, or variants or fragments thereof are in the technical solutions of the present disclosure. polyA or coding sequences thereof in WO2016005324, WO2016005004, WO2016091391, WO2019036513, and WO2020074642 are incorporated herein by reference in their entirety, all of which may be used in the technical solutions of the present disclosure.

### Stuffer sequence

A stuffer sequence may be comprised in the vector genome (or viral genome) of the polynucleotide or AAV particle according to any one of the foregoing of the present disclosure, such that the length of the vector genome is the optimal size for packaging, for example, such that the length of the vector genome is about 2.3 kb, about 4.6 kb, about 4.7 kb, or about 5.1 kb.

In some embodiments, the vector genome is a single-stranded or double-stranded genome, and the packaged AAV particle is ssAAV or scAAV.

In some embodiments, one vector genome may comprise one or more (e.g., 1, 2, 3, 4, 5, 6, 7, 8, 9, or 10) stuffer sequences.

In some embodiments, the stuffer sequence may be located between or within a plurality of regulatory elements, e.g., between two introns, at 3' of a 5' ITR sequence, at 5' of a 5' ITR sequence, at 5' of a 3' ITR sequence, at 3' of a 3' ITR sequence, and before or after a promoter, an intron, an enhancer, a polyA, a multiple cloning site (MCS) region, an exon, or other regions.

### Production or preparation method for rAAV particles

The present disclosure provides methods for producing, preparing, and/or modifying rAAV particles. The production, preparation, and/or modification methods for rAAV particles in WO200028004, WO200123001, WO2004112727, WO 2005005610, WO2005072364, WO2013123503, WO2015191508, and US20130195801 are incorporated herein by reference in their entirety. The rAAV particles may have enhanced delivery efficiency, may be efficiently packaged, and may successfully infect a target cell (e.g., a mammalian or human cell) with high frequency and minimum toxicity.

In some embodiments, provided is a production method for rAAV particles, comprising packaging any of the polynucleotides or vector genomes (or viral genomes) or vectors of the present disclosure into an AAV capsid. In some specific embodiments, the method comprises the following steps: 1) co-transfecting competent bacterial cells with a baculovirus vector and a viral construct vector and/or an AAV payload construct vector, 2) isolating the resulting viral construct expression vector and AAV payload construct expression vector, and separately transfecting viral replication cells, 3) isolating and purifying the resulting payload and viral construct particles comprising the viral construct expression vector or AAV payload construct expression vector, 4) co-infecting the viral replication cells with both the AAV payload and the viral construct particles comprising the viral construct expression vector or AAV payload construct expression vector, and 5) harvesting and purifying AAV particles comprising a viral genome.

In some embodiments, provided is a production method for rAAV particles, comprising the following steps:
1) co-transfecting a mammalian cell (e.g., an HEK293 cell) with any of the polynucleotides or vector genomes (or viral genomes) or vectors of the present disclosure, and a construct expressing Rep and Cap genes and a helper construct (to achieve helper functions) simultaneously; and
2) harvesting and purifying rAAV particles comprising a viral genome.

In some embodiments, the viral genome of the foregoing rAAV particles optionally encodes a selection marker. The selection marker may include a cell surface marker, such as any protein expressed on the cell surface, including but not limited to, a receptor CD marker, a lectin, an integrin, or truncated forms thereof.

In some embodiments, provided is an AAV production system for producing the rAAV particles of the present disclosure, wherein the production system comprises:
1) a polynucleotide sequence encoding an AAV capsid;
2) any polynucleotide or vector genome (or viral genome) or vector of the present disclosure; and
3) sufficient AAV rep function and helper function to allow packaging of the polynucleotide or vector genome (or viral genome) or vector in (b) into the AAV capsid. In the foregoing embodiments, the AAV capsid is selected from the group consisting of any of the AAV capsids of the present disclosure described above, such as AAV1, AAV2, AAV3, AAV4, AAV5, AAV6, AAV7, AAV8, AAV9, AAV9.47, AAV9(hu14), AAV10, AAV11, AAV12, AAVrh8, AAVrh10, AAV-DJ, or AAV-DJ8.

In some specific embodiments, the sufficient AAV rep function and helper function are provided by a packaging cell, wherein the packaging cell may comprise three plasmids pHelper, pRC9, and pGOI.

In some embodiments, the Rep genes encode the non-structural proteins that regulate functions such as the replication of the AAV genome, and may be selected from the group consisting of Rep78, Rep68, Rep52 and Rep40. Rep78 and Rep68 are generally transcribed from the p5 promoter, while Rep52 and Rep40 are generally transcribed from the p19 promoter. The Cap genes encode the structural proteins VP1, VP2 and/or VP3 that assemble to form the viral capsid. The Cap genes are generally transcribed from the p40 promoter.

In some embodiments, provided are rAAV particles produced by the AAV production system described above.

### Cell

The present disclosure provides a cell capable of packaging AAV, which is any cell suitable for the production of a heterologous protein.

In some embodiments, the cell is a mammalian cell, including but not limited to, HEK293, HELA, CHO, NSO, SP2/0, PER.C6, VERO, RD, BHK, HT 1080, A549, COS-7, ARPE-19, and MRC-5.

In some embodiments, the cell is an insect cell and may be from Spodoptera frugiperda, a Drosophila cell line, or mosquito cell lines, such as Aedesalbopictus derived cell lines, including but not limited to, Se301, SeIZD2109, SeUCR1, Sf9, Sf900+, Sf21, BTI-TN-5B1-4, MG-1, Tn368, HzAm1, Ha2302, Hz2E5, HighFive (Invitrogen, CA, USA), AO38, and BM-N.

### Pharmaceutical Composition

The present disclosure provides a pharmaceutical composition containing a prophylactically or therapeutically effective amount of an active ingredient, such as any of the polynucleotides (including the AADC polynucleotide and/or GDNF polynucleotide) or rAAV particles of the present disclosure described above, and one or more pharmaceutically acceptable excipients.

In some embodiments, the pharmaceutical composition may comprise 0.01 to 99 weight% of a polynucleotide (including AADC polynucleotide and/or GDNF polynucleotide) or rAAV particles in a unit dose. In some other specific embodiments, the pharmaceutical composition comprises 0.1 to 10 × 10¹³ gene copies of AADC and/or 0.1 to 10 × 10¹³ gene copies of GDNF in a unit dose.

In some embodiments, the pharmaceutical composition comprises a formulation including, but not limited to, normal saline, liposomes, lipid nanoparticles, polymers, peptides, proteins, and combinations thereof. The formulation may be prepared by any method known in the field of pharmacology or later developed.

In some embodiments, cells may be transfected with rAAV particles for transfer or transplantation into a subject.

In some embodiments, the pharmaceutical composition comprises a rAAV particle that wraps a polynucleotide, wherein the polynucleotide has at least 95% identity to the sequence set forth in any one of SEQ ID NOs: 13-25, for example, the polynucleotide comprises the sequence set forth in any one of SEQ ID NOs: 13-25.

### Method for treating disease and pharmaceutical use

The present disclosure provides a method for using, or use of, any of the foregoing polynucleotides (including the AADC polynucleotide and/or GDNF polynucleotide), rAAV particles, and pharmaceutical compositions of the present disclosure in treating, alleviating, or ameliorating a disease or symptom.

The present disclosure provides a method for using, or use of, any of the foregoing polynucleotides, rAAV particles, and pharmaceutical compositions in combination with levodopa in treating, alleviating, or ameliorating the foregoing neurological disease or symptom.

In some embodiments, provided is any of the foregoing polynucleotides, rAAV particles, and pharmaceutical compositions of the present disclosure for administration in combination with levodopa.

In some embodiments, provided is levodopa, wherein the levodopa is administered in combination with any of the foregoing polynucleotides, rAAV particles, and pharmaceutical compositions of the present disclosure.

In some embodiments, the disease or symptom is caused by or associated with AADC protein deficiency, and/or the disease or symptom is caused by or associated with GDNF protein deficiency.

In some embodiments, the disease or symptom is a neurological disease or symptom, such as a central nervous system disease or symptom. The central nervous system disease or symptom may be a disease or symptom affecting any part of the brain (including hemispheres, diencephalon, brainstem, and cerebellum) or the spinal cord. The neurological disease or symptom includes, but is not limited to, diseases or symptoms characterized by neuromuscular diseases, lysosomal diseases, traumas, bone marrow injuries, pains (including neuropathic pain), cancers of the nervous system, demyelinating diseases, autoimmune disorders of the nervous system, neurotoxic syndromes, sleep disorder, hereditary brain diseases, and developmental CNS disorders. The polynucleotides, rAAV particles, and pharmaceutical compositions of the present disclosure may alleviate or relieve symptoms caused by the level and/or function abnormity (e.g., a deficiency or defect in a protein) of a gene product in a subject in need thereof, or otherwise confer a benefit to a CNS disorder in a subject in need thereof.

In some embodiments, the disease or symptom is a neurological disease or symptom, including but not limited to Alzheimer's disease (AD), amyotrophic lateral sclerosis (ALS), creutzfeldt-jakob disease (CJD), Huntington's disease (HD), Friedreich's ataxia (FA), Parkinson's disease (PD), multiple system atrophy (MSA), spinal muscular atrophy (SMA), multiple sclerosis (MS), primary progressive aphasia, progressive supranuclear palsy (PSP), dementia, brain cancer, degenerative neurological disorders, encephalitis, epilepsy, hereditary brain disorders causing neurodegeneration, retinitis pigmentosa (RP), head and brain malformations, hydrocephalus, stroke, prion diseases, and infantile neuronal ceroid lipofuscinosis (INCL) (neurodegenerative diseases in children caused by a deficiency in a lysosomal enzyme palmitoyl-protein thioesterase 1 (PPT1), etc.).

In some embodiments, the disease or symptom is a disease associated with impaired growth and development of the central nervous system, i.e., a neurodevelopmental disorder. In certain aspects, such neurodevelopmental disorders may be caused by genetic mutations, including but not limited to, fragile X syndrome (caused by a mutation of the FMR1 gene), Down's syndrome (caused by trisomy 21), Rett syndrome, Williams syndrome, Angelman syndrome, Smith-Magenis syndrome, ATR-X syndrome, Barth syndrome, and immune dysfunction and/or infectious diseases in infancy, such as Sydenham chorea, schizophrenia, congenital toxoplasmosis, congenital rubella syndrome, metabolic disorders (such as diabetes and phenylketonuria), nutritional deficiencies and/or brain traumas, autism, and autism spectrum.

In some embodiments, the disease or symptom is a tumor in the central nervous system, including but not limited to, acoustic neuroma, astrocytoma (grades I, II, III, and IV), chordoma, CNS lymphoma, craniopharyngioma, neuroglioma (e.g., brain stem neuroglioma, ependymoma, optic nerve neuroglioma, subependymoma), medulloblastoma, meningioma, metastatic encephaloma, oligodendroglioma, pituitary tumor, primitive neuroectodermal (PNET) tumor, and schwannoma.

In some embodiments, the disease or symptom is a functional neurological disorder with motor and/or sensory symptoms, which has a nervous system origin in the central nervous system, including but not limited to, chronic pain, epileptic seizure, language problems, involuntary movement, and sleep disorder.

In some embodiments, the disease or symptom is a white matter disorder (a group of diseases that affect nerve fibers in the central nervous system), including but not limited to, Pelizaeus-Merzbacher disease, hypomyelination with basal ganglion and cerebellar atrophy, Aicardi-Goutières syndrome, megalencephalic leukoencephalopathy with subcortical cysts, congenital muscular dystrophy, myotonic dystrophy, Wilson's disease, Lowe syndrome, syndrome, PIBD or Tay syndrome, Cockayne disease, cerebrotendinous xanthomatosis, Zellweger syndrome, neonatal adrenoleukodystrophy, infantile refsum disease, Zellweger-like syndrome, pseudo-Zellweger-like syndrome, pseudo-neonatal adrenoleukodystrophy, bifunctional protein deficiency, X-linked adrenoleukodystrophy and adrenomyeloneuropathy, and Refsum disease.

In some embodiments, the disease or symptom is lysosomal storage disorders (LSDs), Gaucher's disease (caused by mutations in the β-glucocerebrosidase (GBA) gene), GM1/GM2 gangliosidosis, mucopolysaccharidosis disorders, Pompe disease, or neuronal ceroid lipofuscinosis that may be caused by the inability of cells in the central nervous system to decompose metabolic end products, wherein the LSD includes, but is not limited to, Niemann-Pick disease, metachromatic leukodystrophy (MLD), globoid-cellleukodystrophy (GLD), and fabry disease.

In some embodiments, the disease or symptom is Friedreich's ataxia, amyotrophic lateral sclerosis (ALS), Huntington's disease, or spinal muscular atrophy (SMA).

In some embodiments, provided is a method for using, or use of, any of the foregoing polynucleotides, rAAV particles, and pharmaceutical compositions of the present disclosure in treating, alleviating, or preventing Parkinson's disease or symptom.

In some embodiments, a subject previously suffering from Parkinson's disease has observed motor symptoms, such as tremors and changes in motion, prior to administration of the polynucleotides, rAAV particles, and pharmaceutical compositions described herein. Non-limiting examples of tremors include unilateral or bilateral mild tremors, bilateral or midline moderate tremors, or refractory tremors. Non-limiting examples of changes in motion include mild bradykinesia, moderate bradykinesia, severe bradykinesia, and morning bradykinesia.

In some embodiments, the subject with Parkinson's disease may have a change in balance, for example, but not limited to, impaired balance, impaired righting reflex, a significant balance disorder, or a fall.

In some embodiments, the subject with Parkinson's disease may have a change in non-motor symptoms; as a non-limiting example, the subject may have a mild to moderate cognitive disorder prior to administration of the composition described herein; as another non-limiting example, the subject with Parkinson's disease may have a significant cognitive disorder, such as dementia, which may also include behavior disorders, such as hallucination.

In some embodiments, the subject with Parkinson's disease may have motor fluctuations that cause mild to moderate disability to one or more dopaminergic drugs. In some embodiments, prior to administration of the composition described herein, the subject with Parkinson's disease may have medically refractory motor fluctuations consisting of "wearing off' and/or levodopa-induced dyskinesia, which cause significant disability.

In some embodiments, the subject may have mild symptoms associated with Parkinson's disease, for example, but not limited to, no cognitive impairment (diagnosed within the last 5 years), a satisfactory response with limited fluctuations to one or more dopaminergic drugs, unilateral or bilateral mild tremors, little or no impact on quality of life, and/or no balance impairment.

In some embodiments, the subject may have moderate symptoms associated with Parkinson's disease, for example, but not limited to, a mild to moderate cognitive disorder, preliminary signs of impaired balance and righting reflex, motor fluctuations causing mild to moderate disability to one or more dopaminergic drugs (diagnosed within the last 5 to 10 years), bilateral or moderate tremors, moderate bradykinesia, and/or subjects experiencing certain restrictions in activities of daily living.

In some embodiments, the subject may have advanced symptoms associated with Parkinson's disease, for example, but not limited to, diagnosis of Parkinson's disease for more than 10 years, moderate refractory motor fluctuations wearing off and/or levodopa-induced dyskinesia, causing major disability, refractory tremors, a significant balance disorder and/or a fall, a significant cognitive disorder (e.g., dementia with or without behavior disorders), severe bradykinesia, and a significant reduction in quality of life due to diseases and/or morning dyskinesia.

In some embodiments, the subject with Parkinson's disease uses levodopa (e.g., DUOPA^{™}) in combination with the polynucleotides, rAAV particles, and pharmaceutical compositions of the present disclosure. As a non-limiting example, the subject may achieve success by using levodopa (e.g., DUOPA^{™}) alone. As a non-limiting example, using DUOPA^{™} alone, the subject may have not achieved any success or achieved limited success.

In some embodiments, a UPDRS-3 (or UPDRS-III) drug score of the subject is assessed prior to administration of the polynucleotides, rAAV particles, and pharmaceutical compositions of the present disclosure. In some embodiments, the UPDRS-3 (or UPDRS-III) drug score of the subject is reduced after administration of the polynucleotides, rAAV particles, and pharmaceutical compositions of the present disclosure.

In some embodiments, after administration of the polynucleotides, rAAV particles, and pharmaceutical compositions of the present disclosure, the subject has improved motor functions, and/or a reduction in the amount of the levodopa drug required by the subject to manage symptoms.

In some embodiments, provided is a method for using, or use of, any of the foregoing polynucleotides, rAAV particles, and pharmaceutical compositions of the present disclosure in treating a disease or symptom associated with circadian rhythms and wake cycle, including but not limited to, sleep disorder (e.g., insomnia), depression, bipolar disorder, seasonal affective disorder, obesity, and diabetes.

In some embodiments, the polynucleotides, rAAV particles, and pharmaceutical compositions of the present disclosure may be administered by any route of delivery that results in a therapeutically effective result, including but not limited to, enteral, gastrointestinal, epidural, oral, transdermal, intracerebral, intracerebroventricular (including intralateral ventricular), epidermal, intradermal, subcutaneous, nasal, intravenous, intravenous, intra-arterial, intramuscular, intraosseous infusion (including into the bone marrow), intrathecal (including into the spinal canal), intraparenchymal (into the brain tissue), intraperitoneal (infusion or injection into the peritoneum), percutaneous (diffusion through the intact skin, for systemic distribution), transmucosal, transtracheal (through the tracheal wall), and the like, for example, intraoral, intranasal, subcutaneous, intramuscular, transvascular (e.g., intravenous), intrathecal, intracerebral, and/or intracerebroventricular.

In some embodiments, the polynucleotides, rAAV particles, and pharmaceutical compositions of the present disclosure are delivered or administered by the route of injection into the CSF, for example, delivered or administered to cells of the central nervous system (e.g., parenchymal cells); for example, delivered or administered intrathecally and intracerebroventricularly; for example, delivered or administered to the substantia nigra or striatum, including the substantia nigra pars compacta (SNpc) and ventral tegmental area (VTA), or optionally, to the striatum (into the caudate nucleus and putamen) or the subthalamic nucleus (STN); for example, delivered or administered to DRG nociceptive neurons, cerebellomerlullary cistern, and transduced spinal motor neurons, and/or astrocytes. The delivery or administration may be injection or direct infusion.

In some embodiments, the polynucleotides, rAAV particles, and pharmaceutical compositions are allowed to be administered in a manner that crosses the blood-brain barrier, vascular barrier, or other epithelial barriers.

In some embodiments, the polynucleotides, rAAV particles, and pharmaceutical compositions of the present disclosure may be administered through a single site or multiple sites (e.g., 1, 2, 3, 4, 5, 6, 7, 8, 9, or 10 sites).

In some embodiments, the polynucleotides, rAAV particles, and pharmaceutical compositions of the present disclosure may be administered in a single dose. A single dose of intravenous delivery may provide sustained alleviation to a subject suffering from a central nervous system disease (e.g., Parkinson's disease) and/or associated symptoms. The alleviation may last several minutes, days, weeks, months (e.g., 1 month, 2 months, 3 months, 4 months, 5 months, 6 months, 7 months, 8 months, 9 months, 10 months, 11 months, or 12 months), or years (e.g., 1 year, 2 years, 3 years, 4 years, 5 years, 6 years, 7 years, 8 years, 9 years, 10 years, 11 years, 12 years, 13 years, 14 years, 15 years, 16 years, 17 years, 18 years, 19 years, 20 years or more).

The foregoing polynucleotides include any AADC polynucleotide and/or GDNF polynucleotide of the present disclosure.

### Kit and device

The present disclosure provides a kit and a device comprising any of the foregoing polynucleotides, rAAV particles, and pharmaceutical compositions of the present disclosure.

In some embodiments, the kit comprises a container device generally including at least one vial, test tube, flask, bottle, syringe, or other container devices, into which the polynucleotides, rAAV particles, and pharmaceutical compositions may be placed. Generally, the kit may further comprise a second, third, or other additional containers in which additional components may be separately placed, for example, for containing sterile pharmaceutically acceptable buffers and/or other diluents. In some embodiments, the kit comprises a package insert.

In some embodiments, the polynucleotides, rAAV particles, and pharmaceutical compositions of the present disclosure may be combined with, applied to, or embedded in the device. The device may include, but is not limited to, a stent, a pump, and/or other implantable therapeutic devices. In addition, the polynucleotides, rAAV particles, and pharmaceutical compositions may be delivered to a subject when the subject uses a compression device, for example, but not limited to, a compression device to reduce the chance of deep venous thrombosis (DVT) in the subject.

In some embodiments, the polynucleotides, rAAV particles, and pharmaceutical compositions of the present disclosure may be delivered using devices, including but not limited to, stents, tubes (including catheters, pipelines, and straws), and gene guns. The polynucleotides, rAAV particles, and pharmaceutical compositions of the present disclosure may be administered to a subject using a delivery system that integrates image-guided therapy and integrates imaging, including but not limited to, a laser, MRgFUS, an endoscope, and a robotic surgical device. The polynucleotides, rAAV particles, and pharmaceutical compositions of the present disclosure may be administered to a subject using an automated delivery system. As a non-limiting example, the device described in US5865744 is incorporated herein by reference in its entirety. A computer adjusts needle administration to an appropriate depth for a particular subject according to images acquired by the delivery system. The polynucleotides, rAAV particles, and pharmaceutical compositions of the present disclosure may be delivered using device localization to a target site of a subject, an enhanced delivery device, or a MIR guide device. As non-limiting examples, the methods, systems, and/or computer programs described in US8340743 are incorporated herein by reference in their entirety.

### Definitions

In order to facilitate the understanding of the present disclosure, some technical and scientific terms are specifically defined below. Unless otherwise specifically defined in the present disclosure, all other technical and scientific terms used in the present disclosure have the meanings generally understood by those of ordinary skill in the art to which the present disclosure pertains.

Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to".

The three-letter and single-letter codes for amino acids used herein are as described in J. biol. chem, 243, p3558 (1968).

"Aromatic L-amino acid decarboxylase", "aromatic amino acid dopa decarboxylase", or "AADC (amino acid decorboxylase)" is a homodimeric pyridoxal phosphate-dependent enzyme responsible for the syntheses of dopamine and serotonin. AADC catalyzes the decarboxylation of L-3,4-dihydroxyphenylalanine (L-DOPA or levodopa) to dopamine, L-5-hydroxytryptophan to serotonin, and L-tryptophan to tryptamine. The AADC proteins and polynucleotides encoding same in the present disclosure encompass wild-type AADC proteins, variants thereof, and related encoding polynucleotides. Exemplary AADC protein sequences and polynucleotide sequences encoding same are set forth in SEQ ID NOs: 1 and 3 of the present disclosure. The wild-type AADC proteins may be any naturally occurring isoform or variants from the DOPA decarboxylase (DDC) gene, and multiple alternatively spliced transcript variants encoding different isoforms of AADC have been identified. Specifically, the DDC gene produces seven transcript variants that encode six different isoforms. DDC transcript variants 1 and 2 both encode AADC isoform 1. In some embodiments, the AADC polynucleotide encodes DDC transcript variant 2, thereby encoding natural AADC isoform 1(NCBI database: NP_000781.1). AADC in WO2018232055A is also incorporated in the present disclosure by reference in its entirety as an exemplary AADC. "AADC polynucleotide" is any polynucleotide polymer that encodes AADC protein and expresses such AADC protein in a cell, a tissue, an organ, or an organism when present in a vector, a plasmid, or a translatable construct.

"Glial cell line-derived neurotrophic factor" or "GDNF" is a neurotrophic factor that may support the development and survival of neurons from peripheral sympathetic nerves, parasympathetic nerves, enteric nerves, and sensory nerves, as well as dopamine neurons and motor neurons in the midbrain, and the GDNF proteins and polynucleotides encoding same in the present disclosure encompass wild-type GDNF proteins, variants thereof, and related encoding polynucleotides. Two GDNF splice variants, pre-(α)pro-GDNF (previously known as GDNFα) and pre-(β)pro-GDNF (previously known as GDNFβ), are known to exist (Suter-Crazzolara and Unsicker; 1994), which are produced by differential splicing of GDNF mRNA. The mature GDNF proteins produced by these two splice variants are very likely identical, and the mature GDNF consists of 134 amino acids and contains two putative N-glycosylation sites and 7 conserved cysteines. Such cysteines are at the same relative spacing to the other members of the TGF-β protein family. The biologically active mature GDNF dimers are formed by covalent disulfide bonds between unpaired cysteines in the monomers (Lin et al., 1993; Eigenbrot and Gerber, 1997; Chang et al., 2002). Exemplary GDNF protein sequences and polynucleotide sequences encoding same are set forth in SEQ ID NOs: 2 and 8 of the present disclosure. The (α)pro-GDNF protein in US6362319, the truncated form of GDNF in US6184200, and GDNF in WO2009053536A are also incorporated in the present disclosure by reference in their entirety as exemplary GDNF. "GDNF polynucleotide" is any polynucleotide polymer that encodes GDNF protein and expresses such GDNF protein in a cell, a tissue, an organ, or an organism when present in a vector, a plasmid, or a translatable construct.

"Adeno-associated virus (AAV)" encompasses "recombinant adeno-associated virus (rAAV)", which is a replication-deficient and non-enveloped virus and is a member of the family Parvoviridae of the genus Dependovirus. "AAV particle" or "rAAV particle" consists of at least two components, including a capsid and a polynucleotide wrapped therein. AAV particles may be derived from any serotype described in the present disclosure or known in the art, including combinations of serotypes (i.e., "pseudotyped" AAV) or various genomes (e.g., single-stranded or self-complementary). Additionally, the AAV particles may be replication-deficient and/or targeted. The AAV of the present disclosure encompasses variants that may have sequences with significant homology at the polynucleotide (genome or capsid) and amino acid levels (capsid) to produce constructs, and the constructs are generally physical and functional equivalents, replicate by similar mechanisms, and assemble by similar mechanisms.

"Polypeptide" and "protein" are used interchangeably, refer to a polymer of amino acid residues, and are suitable for naturally occurring amino acid polymers and non-naturally occurring amino acid polymers. Unless otherwise stated, a particular polypeptide sequence also implicitly encompasses conservatively modified variants thereof.

"Polypeptide variant" or "protein variant" refers to a molecule that has some difference in its amino acid sequence as compared to a natural or starting sequence. The variant may have substitutions, deletions, and/or insertions at certain positions within the amino acid sequence. The "native" or "starting" sequence should not be confused with the wild-type sequence. As used in the present disclosure, the natural or starting sequence is a relative term that refers to the original molecule with which the comparison may be made. The "native" or "starting" sequence or molecule may represent a wild-type sequence (a sequence found in nature), but need not be a wild-type sequence. Generally, the variant has at least about 70% identity, preferably at least about 80%, and more preferably at least about 90% identity to the natural sequence.

"Analog" refers to a polypeptide variant that includes a difference of one or more amino acid changes (for example, substitutions, additions, or deletions of amino acid residues) and still retains the properties of a parent polypeptide. The analog may be obtained by a conservative substitution of the parent polypeptide, and the method for such conservative substitution is commonly used in the art.

"Conservative substitution", when in a protein or polypeptide, refers to a substitution to another amino acid residue having similar properties to the original amino acid residue. For example, lysine, arginine, and histidine have similar properties in that they have basic side chains, and aspartic acid and glutamic acid have similar properties in that they have acidic side chains. In addition, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is substituted, it will not exhibit a particular change in properties.

"Linker" refers to a linker unit that links two polypeptide fragments or polynucleotides encoding same and generally has some flexibility, such that the use of the linker does not result in the loss of the original function of the protein domain. The linker may be a peptide linker or a polynucleotide encoding same comprising one or more amino acids (typically about 1-30, 2-24, or 3-15 amino acids) or polynucleotides encoding same. The linkers used in the present disclosure may be identical or different.

"Fusion" or "linkage" means that components (e.g., polynucleotides encoding two target genes or encoded proteins or polypeptides thereof) are linked directly or by a covalent bond via one or more linkers. When the linker is a peptide linker, the covalent bond is a peptide bond.

"Polynucleotide" and "polynucleotide" are used interchangeably and refer to a deoxyribonucleotide or ribonucleotide polymer in either linear or circular conformation and in either single-stranded or double-stranded form. In the present disclosure, the term is not to be construed as limiting in length. The term may encompass known analogs of natural nucleotides, as well as nucleotides that are modified in the base, sugar, and/or phosphate moieties (e.g., phosphorothioate backbones). Generally, an analog of a particular nucleotide has the same base pairing specificity, e.g., an analog of A is subjected to base pairing with T. A polynucleotide is "operably linked" when it is placed into a functional relationship with another polynucleotide sequence. For example, if an expression control sequence (e.g., a promoter or an enhancer) affects the transcription of a target gene, the expression control sequence is operably linked to the target gene.

"RNA" and "ribopolynucleotide" refer to a polymer of ribonucleotides. "DNA" and "deoxyribopolynucleotide" refer to a polymer of deoxyribonucleotides. DNA and RNA may be naturally synthesized (e.g., by DNA replication and DNA transcription, respectively) or by chemical synthesis. DNA and RNA may be single-stranded (i.e., ssRNA or ssDNA, respectively) or multi-stranded (e.g., double-stranded, i.e., dsRNA and dsDNA, respectively). "mRNA" or "messenger RNA" refers to a single-stranded RNA encoding an amino acid sequence of one or more polypeptide chains.

"Operably linked" refers to a functional linkage between two or more molecules, constructs, transcripts, entities, moieties, and the like, encompassing both situations where an expression control sequence is adjacent to a target gene and where an expression control sequence acts in trans or remotely to control the expression of a target gene.

"A polynucleotide is operably linked to an expression control sequence" means that the polynucleotide is expressed under the regulation and control of the expression control sequence. Two or more polynucleotides may be expressed under the regulation and control of the same expression control sequence, and may also be expressed under the regulation and control of two identical or different expression control sequences.

As used herein, the "expression control sequence" encompasses any polynucleotide that controls the expression of a coding sequence, including but not limited to, promoters, enhancers, and the like.

"Sequence" refers to an amino acid sequence or polynucleotide sequence in any length; when it is a polynucleotide sequence, it may be DNA or RNA, may be linear, circular or branched, and may be single-stranded or double-stranded.

"Homology", "identity", or "sequence identity" refers to the overall correlation between polymer molecules (e.g., oligonucleotides, polynucleotides, or polypeptides). For example, the percent identity of two polynucleotide sequences may be calculated by aligning the two. The numerical value of identity may differ due to gaps and penalties introduced in the calculation, but the best way may be selected (e.g., a gap may be introduced in one or both of two sequences to be aligned to achieve the best alignment, and the different sequences may be ignored to achieve the comparison, one of the sequences being a target sequence to be aligned and the other being a reference sequence). In some embodiments, the length of the target sequence to be aligned may be at least 30%, at least 40%, at least 50%, at least 60%, at least 70%, at least 80%, at least 90%, at least 95%, or 100% of the length of the reference sequence, and then the nucleotides at the corresponding nucleotide positions are compared. When a position in the target sequence to be aligned is occupied by the identical nucleotide at the corresponding position in the reference sequence, the two are identical at this position. The percent identity between two sequences is a function of the number of identical positions shared by the sequences, taking into account the number of gaps and the length of each gap, wherein the gaps need to be introduced to achieve the optimal alignment of the two sequences. The comparison of sequences and determination of percent identity between two sequences may be accomplished using a mathematical algorithm. For example, the percent identity between two polynucleotide sequences may be determined using methods such as those described in the following: Computational Molecular Biology, Lesk, A.M., ed., Oxford University Press, New York, 1988; Biocomputing: Informatics and Genome Projects, Smith, D.W., ed., Academic Press, New York, 1993; Sequence Analysis in Molecular Biology, von Heinje, G., Academic Press, 1987; Computer Analysis of Sequence Data, Part I, Griffin, A.M., and Griffin, H.G., eds., Humana Press, New Jersey, 1994; and Sequence Analysis Primer, Gribskov, M. and Devereux, J., eds., M Stockton Press, New York, 1991 (all incorporated in the present disclosure in their entirety). For example, the percent identity between two nucleotide sequences may be determined using the Meyers and Miller (CABIOS, 1989, 4:11-17) algorithm, which has been incorporated into the ALIGN program (version 2.0), and using a PAM120 weight residue table with a gap length penalty of 12 and a gap penalty of 4. Alternatively, the percent identity between two nucleotide sequences may be determined using NWSgapdna.CMP matrix and using the GAP program in the GCG software package. Methods commonly used to determine the percent identity between sequences include, but are not limited to, Carillo, H., and Lipman, D., SIAM J Applied Math., 48:1073.

"Conserved" refers to nucleotides or amino acid residues of polynucleotide sequences or polypeptide sequences that are invariable at identical positions of two or more sequences to be compared. A relatively conserved nucleotide or amino acid is a conserved nucleotide or amino acid in a sequence that is more relevant than nucleotides or amino acids appearing elsewhere in the sequence. In some embodiments, two or more sequences are "fully conserved" if they are 100% identical to each other; two or more sequences are "highly conserved" if they are at least 70%, 75%, 80%, 90%, 95% or more identical to each other; two or more sequences are "conserved" if they are at least 30%, 40%, 50%, 60% or more identical to each other. Sequence conservation may apply to the full length of an oligonucleotide, polynucleotide, or polypeptide, or may apply to a portion or a region thereof. A conserved sequence may be discontinuous, and one skilled in the art will understand how to achieve alignment when gaps in the continuous alignment exist between sequences, and how to align corresponding residues without tolerating the presence of insertions or deletions.

"Gene expression" refers to the process of converting information contained in a gene into a gene product, e.g., a polynucleotide undergoing successful transcription and, in most cases, translation to produce a protein or peptide. The gene product may be a direct transcription product of the gene (e.g., mRNA, tRNA, rRNA, antisense RNA, ribozyme, structural RNA, or any other type of RNA) or a protein produced by translation of mRNA. The gene product also includes RNA modified by processes such as capping, polyadenylation, methylation, and editing, as well as proteins modified by, for example, methylation, acetylation, phosphorylation, ubiquitination, ADP-ribosylation, myristoylation, and glycosylation. "Regulation" of gene expression refers to a change in gene activity, and the regulation of expression may include, but is not limited to, gene activation, gene optimization, and gene repression. Genome editing (e.g., cleavage, alteration, inactivation, or random mutation) may be used to regulate the expression. When referring to measurement of "gene expression", it should be understood to mean that the measurement may be of a transcribed polynucleotide product, such as RNA or mRNA, or of a translated amino acid product, such as a polypeptide or peptide. Methods for measuring the amount or level of RNA, mRNA, polypeptides, and peptides are well known in the art.

"Transfection" refers to a method for introducing an exogenous polynucleotide into a cell. Transfection methods include, but are not limited to, chemical methods, physical treatments, and cationic lipids or mixtures.

"Payload" refers to one or more polynucleotides or polynucleotide regions encoded by or encoded within a viral genome, or expression products of such polynucleotides or polynucleotide regions, e.g., a transgene, a polynucleotide encoding a polypeptide, or a regulatory polynucleotide. In some embodiments of the present disclosure, the payload encodes or expresses a target gene, and the target gene is, for example, AADC and/or GDNF. "Payload construct" is one or more polynucleotide regions encoding or comprising a payload flanked on one or both sides by inverted terminal repeat (ITR) sequences, and the payload construct is a template that is replicated in a viral production cell to produce a viral genome. "Payload construct vector" is a vector that encodes or comprises a payload construct and regulatory regions for replication and expression in bacteria and cells. "Payload construct expression vector" is a vector that encodes or comprises a payload construct, and further comprises one or more polynucleotide regions encoding or comprising components for viral expression in a viral replication cell.

"Vector" is a construct that transports, transduces, or otherwise serves as a heterologous molecule, e.g., a construct capable of delivering and expressing one or more target genes in a host cell. Examples of vectors include, but are not limited to, viral vectors, naked DNA or RNA expression vectors, plasmid, cosmid or phage vectors, DNA or RNA expression vectors bound to cationic polymers, and DNA or RNA expression vectors encapsulated in liposomes. In some embodiments, the vector may be produced by recombination, may be produced based on an AAV parental or reference sequence, or may comprise an AAV parental or reference sequence. The parental or reference AAV sequence may serve as the original or subsequent sequence for the engineered vector. The parental or reference AAV sequence may comprise a polynucleotide sequence expressing a target gene (encoding a target protein, polypeptide, or a portion thereof) and a regulatory element (e.g., a promoter or an enhancer), which may be wild-type or modified or mutated.

"Vector genome" is a polynucleotide comprising and encoding at least one inverted terminal repeat (ITR), at least one regulatory sequence, and at least one payload. The vector genome is derived by replicating a payload construct from a payload construct expression vector. The vector genome encodes at least one copy of the payload construct.

"Viral construct vector" is a vector comprising one or more polynucleotide regions encoding or comprising Rep and/or Cap proteins. "Viral construct expression vector" is a vector comprising one or more polynucleotide regions encoding or comprising Rep and/or Cap, which further comprises one or more polynucleotide regions encoding or comprising components for viral expression in a viral replication cell.

"Modified" means that the changed state or structure of a molecule or entity as compared to a parent or reference molecule or entity may be modified in a variety of ways, including chemically, structurally, and functionally. In some embodiments, the polynucleotides, polypeptides, vectors, constructs, capsids, etc. of the present disclosure are modified by the introduction of non-natural amino acids or non-natural nucleotides.

"Host cell" includes individual cells or cell cultures which may be or have been the recipient of a vector for incorporation of a polynucleotide insert. The host cell includes progeny of a single host cell, and the progeny may not necessarily be completely identical (in morphology or genomic DNA complement) to the original parent cell due to natural, accidental or deliberate mutations. The host cell includes cells transfected and/or transformed *in vivo* with polynucleotides of the present disclosure. "Cell", "cell line", and "cell culture" are used interchangeably, and all such designations include their progeny. It should also be understood that all progenies may not be precisely identical in DNA content due to intentional or unintentional mutations. Mutant progeny with identical function or biological activity as screened in the original transformed cells is included. Host cells may include microbial (e.g., bacterial), plant, or animal cells. Bacteria susceptible to transformation include members of the *Enterobacteriaceae* family, such as strains of *Escherichia coli* or *Salmonella;* members of the *Bacillaceae* family, such as *Bacillus subtilis; Pneumococcus; Streptococcus* and *Haemophilus influenzae.* Suitable microorganisms include *Saccharomyces cerevisiae* and *Pichia pastoris.* Suitable animal host cell lines include CHO (Chinese hamster ovary cell lines), NS0 cells, and 293 cells.

"Pharmaceutical composition" refers to a mixture comprising one or more of the rAAV described herein and other chemical components, as well as other components such as physiological/pharmaceutically acceptable carriers and excipients. The pharmaceutical composition is intended to promote administration to an organism and facilitate the absorption of the active ingredient so that it can exert its biological activity.

"Pharmaceutically acceptable carrier" or "pharmaceutically acceptable excipient" includes any material that, when combined with an active ingredient, allows the ingredient to retain biological activity and is non-reactive with the immune system of a subject. Examples include, but are not limited to, any standard pharmaceutical carrier, such as a phosphate buffered saline solution, water, an emulsion such as an oil/water emulsion, and various types of wetting agents.

"Giving", "administering" and "treating", when applied to animals, humans, experimental subjects, cells, tissues, organs, or biological fluid, refer to contact of an exogenous drug, a therapeutic agent, a diagnostic agent, or a composition with the animals, humans, subjects, cells, tissues, organs or biological fluid, e.g., therapeutic, pharmacokinetic, diagnostic, research, and experimental methods. The treatment of cells comprises contacting the reagent with the cells and contacting the reagent with fluid, where the fluid is in contact with the cells. "Giving", "administering", and "treating" also refer to treating, e.g., a cell, by a reagent, diagnosis, a binding composition, or by another cell *in vitro* and *ex vivo.* When applied to humans, veterinary or research subjects, they refer to therapeutic treatment, preventive or prophylactic measures, and research and diagnostic applications.

"Treating" or "treatment" refers to administering a therapeutic agent, such as a therapeutic agent comprising any one of the polynucleotides or encoded polypeptides thereof of the present disclosure, rAAV comprising the polynucleotide, or a related pharmaceutical composition, either internally or externally, to a subject who has had, is suspected of having, or is predisposed to having one or more neurodegenerative diseases or symptoms thereof on which the therapeutic agent is known to have a therapeutic effect. Typically, the therapeutic agent is administered in an amount effective to alleviate one or more symptoms of the disease in the subject or population being treated, whether by inducing regression of such symptoms or inhibiting the development of such symptoms into any clinically measurable degree. The amount of therapeutic agent effective to alleviate any particular symptom of the disease (also referred to as the "therapeutically effective amount") may vary depending on factors such as the disease state, age and weight of the subject, and the ability of the drug to produce a desired therapeutic effect in the subject. Whether a symptom of a disease has been alleviated can be evaluated by any clinical testing methods commonly used by doctors or other health care professionals to evaluate the severity or progression of the symptom. Although embodiments of the present disclosure (e.g., treatment methods or articles of manufacture) may be ineffective in alleviating symptoms of a disease of interest in a certain subject, they shall alleviate the symptoms of the disease of interest in a statistically significant number of subjects as determined by any statistical test method known in the art, such as the Student's t-test, Chi-square test, U-test by Mann and Whitney, Kruskal-Wallis test (H-test), Jonckheere-Terpstra test and Wilcoxon test.

"Combined administration" means that two or more agents (e.g., rAAV particles) are administered to a subject simultaneously or at certain intervals, such that there may be an overlap in the effects of each drug on the patient and/or the subject being exposed to both simultaneously at a time point. In some embodiments, they are administered within about 60 min, 30 min, 15 min, 10 min, 5 min, or 1 min of each other or within about 24 h, 12 h, 6 h, or 3 h of at least one dose of one or more other agents.

"Delivery" refers to the act or manner of delivering a compound, such as a parvovirus, e.g., an AAV and/or an AAV compound, a substance, an entity, a portion, a cargo, or a payload, to a target. Such targets may be cells, tissues, organs, organisms, or systems.

"Ameliorating" refers to reducing the severity of at least one indicator of a condition or disease. For example, in the context of neurodegenerative diseases, ameliorating includes reducing neuron loss.

"Effective amount" includes an amount that cures, alleviates, or ameliorates one or more symptoms of a medical disorder or a disorder itself, to achieve a beneficial or desired result in a clinical result. "Effective amount" depends on the environment in which it is applied, and the effective amount for a subject may vary depending on factors such as the disorder to be treated, the general health of the subject, the method and dose of administration, and the severity of side effects. For example, in the case of administration of a pharmaceutical composition for treating Parkinson's disease, the effective amount is, for example, an amount sufficient to achieve treatment of Parkinson's disease as defined in the present disclosure as compared to the response obtained without administration of the pharmaceutical composition.

"Wrapping" refers to encapsulating, surrounding, or enclosing, e.g., using an AAV capsid to encapsulate, surround, or enclose a vector.

"Delivery" refers to the act or manner of delivering a compound, such as a parvovirus, e.g., an AAV and/or an AAV compound, a substance, an entity, a portion, a cargo, or a payload, to a target. Such targets may be cells, tissues, organs, organisms, or systems (whether biological or produced). "Delivery agent" refers to any agent or substance that at least partially facilitates *in vivo* and/or *in vitro* delivery of a polynucleotide and/or one or more substances (including but not limited to, the compounds and/or compositions of the present invention, such as viral particles or expression vectors) to a targeted cell.

"Optional" or "optionally" means that the event or circumstance subsequently described may, but does not necessarily, occur, and that the description includes instances where the event or circumstance occurs or does not occur.

"Subject" or "patient" means a mammal, particularly a primate, and particularly a human.

"About" or "approximately" means that a numerical value is within an acceptable error range for the specific value determined by those of ordinary skill in the art, and the numerical value depends in part on how the value is measured or determined (i.e., the limitations of the measurement system). For example, "about" may mean a standard deviation within 1 or more than 1. Alternatively, "about" or "comprising essentially" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes an embodiment of the given number. Unless otherwise specified, when a specific value is provided in the present application and claims, the meaning of "about" or "substantially comprising" should be assumed to be within an acceptable error range for that specific value.

### BRIEF DESCRIPTION OF THE DRAWINGS

FIG. 1 is a structural schematic diagram of a polynucleotide expressing AADC and/or GDNF of the present disclosure.
FIG. 2A shows the concentration of DOPA detected by HPLC after SHSY5Y cells were transfected with plasmids and treated with L-DOPA; FIG. 2B shows the concentration of GDNF detected by ELISA after SHSY5Y cells were transfected with plasmids.
FIG. 3A shows the amount of AADC protein expressed by each AAV plasmid after AADC codon optimization; FIG. 3B shows the amount of GDNF protein expressed by each AAV plasmid after GDNF codon optimization.
FIG. 4A shows immunofluorescence signals of AADC in the mouse striatum after transfection with AAV2 and AAV9 serotypes; FIG. 4B shows immunofluorescence coverage areas of AADC in the mouse striatum after transfection with AAV2 and AAV9 serotypes.
FIG. 5A shows the expression level of AADC in the striatum after injection of AAV2-02A and AAV9-02A; FIG. 5B shows the expression level of GDNF in the striatum after injection of AAV2-02A and AAV9-02A.
FIG. 6A shows the response of mice to L-DOPA (counterclockwise rotation) after injection of HRPDAAV02-AI (i.e., 02AI, the same applies hereinafter) and HRPDAAV03-DI (i.e., 03DI, the same applies hereinafter) drugs; FIG. 6B shows the response of mice to L-DOPA (contralateral forelimb utilization rate) after the injection of HRPDAAV02-AI and HRPDAAV03-DI drugs.

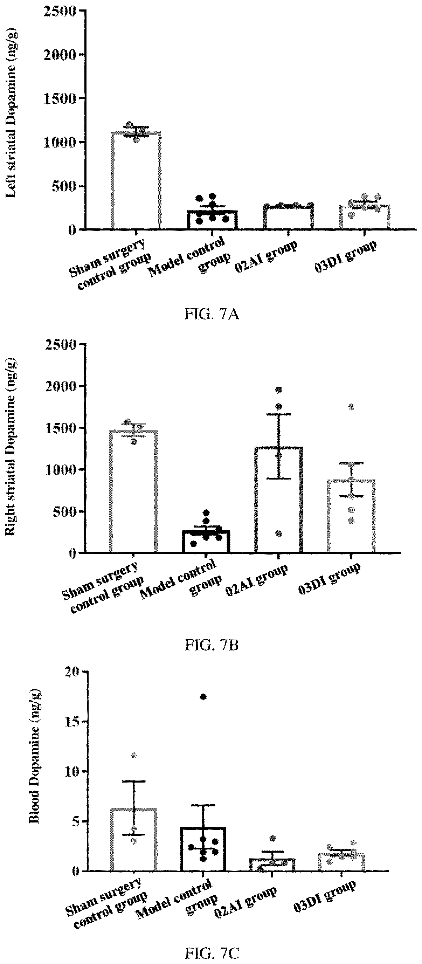

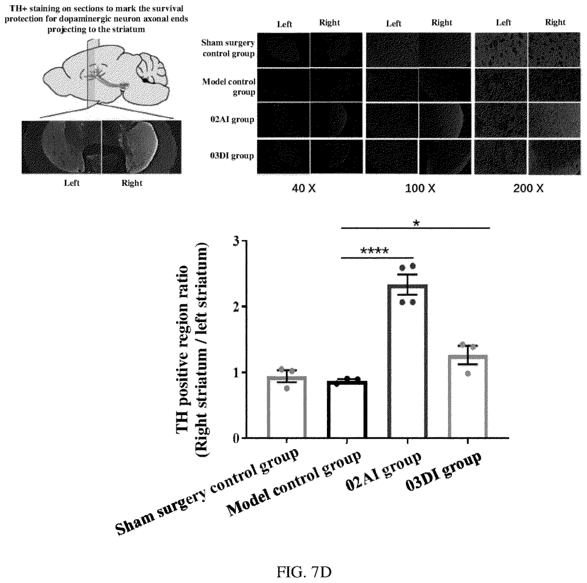

FIG. 7A shows the response of mice to L-DOPA (DOPA concentration at the non-administration side) after the injection of HRPDAAV02-AI and HRPDAAV03-DI drugs; FIG. 7B shows the response of mice to L-DOPA (DOPA concentration at the administration side) after the injection of HRPDAAV02-AI and HRPDAAV03-DI drugs; FIG. 7C shows the response of mice to L-DOPA (DOPA concentration in blood) after the injection of HRPDAAV02-AI and HRPDAAV03-DI drugs; FIG. 7D shows the protection of dopamine neurons in mice after the injection of HRPDAAV02-AI and HRPDAAV03-DI drugs, in which the immunofluorescence signal density of dopamine nerve terminals is statistically analyzed.

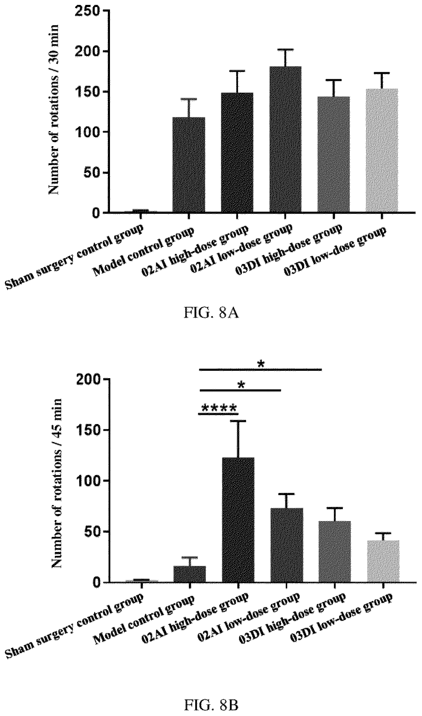

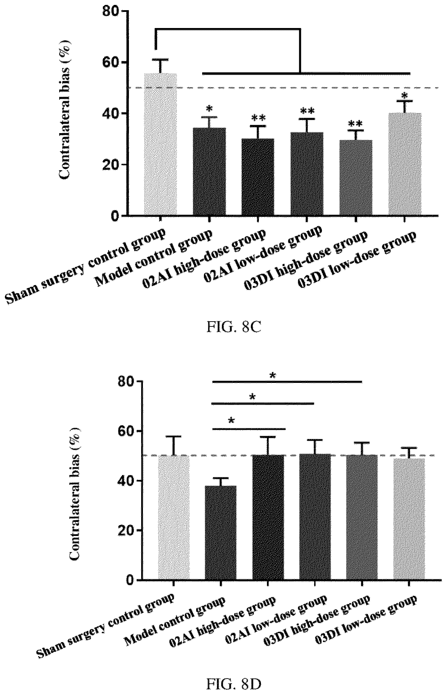

FIG. 8A shows the response of animals in each group to apomorphine after 6-OHDA modeling; FIG. 8B shows the response of rats to L-DOPA after the injection of HRPDAAV02-AI and HRPDAAV03-DI drugs (rotation test); FIG. 8C shows the detection results of a rat model in which a cylinder test was established; FIG. 8D shows the response of rats to L-DOPA (contralateral forelimb utilization rate) after the injection of HRPDAAV02-IA and HRPDAAV03-DI drugs; 8E shows the detection result of the metabolic capacity of striatum to L-DOPA (DOPA ratio of affected side/healthy side) after the administration of HRPDAAV02-IA and HRPDAAV03-DI; 8F shows the protection effect of HRPDAAV-03DI on DA neurons after administration. In the present disclosure, **** represents *p <* 0.0001, *** represents *p* between 0.0001 and 0.001, both representing extremely significant statistical differences; ** representsp between 0.001 and 0.01, indicating a very significant statistical difference; * represents *p* between 0.01 and 0.05, indicating a significant statistical difference.

### DETAILED DESCRIPTION

The present disclosure is further described below with reference to examples, but these examples are not intended to limit the scope of the present disclosure.

Experimental procedures without specific conditions indicated in the examples or test examples are generally conducted according to conventional conditions, or according to conditions recommended by the manufacturer of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY Reagents without specific origins indicated are commercially available conventional reagents.

### Example 1. Design of AADC and GDNF Polynucleotide Structures

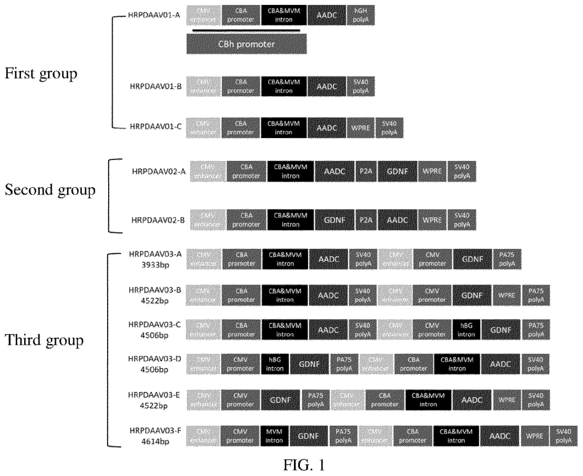

In this example, sequence design and engineering of polynucleotides encoding AADC and GDNF were performed for transduction of cells or for *in vivo* transduction of humans and animals. The sequences could be inserted into plasmid, lentivirus, adenovirus, mRNA-LNP, and adeno-associated virus (AAV) vectors, for example, to achieve *in vivo* and *in vitro* transduction by a recombinant adeno-associated virus (rAAV). The specific sequences between the ITRs of the rAAV plasmid structure are shown in Table 2, the enhancer used may include, for example, a CMV enhancer, and the promoter may include, for example, a CMV promoter and a CBA promoter. The sequences in Table 2 were all non-codon-optimized. The structural schematic diagram is shown in FIG. 1.

**Table 2. Engineered AADC/GDNF polynucleotide sequences**

| rAAV plasmid name | Name in attached drawings | Insertion of target gene | Sequence No. | Structural characteristic |
|---|---|---|---|---|
| HRPDAAV01-A | 01A | AADC | SEQ ID NO:13 | Single-gene expression framework |
| HRPDAAV01-B | 01B | AADC | SEQ ID NO:14 | Single-gene expression framework |
| HRPDAAV01-C | 01C | AADC | SEQ ID NO:15 | Single-gene expression framework |
| HRPDAAV02-A | 02A | AADC/GDNF | SEQ ID NO:16 | Double-gene single-promoter framework |
| HRPDAAV02-B | 02B | AADC/GDNF | SEQ ID NO:17 | Double-gene single-promoter framework |
| HRPDAAV03-A | 03A | AADC/GDNF | SEQ ID NO:18 | Double-gene double-promoter framework |
| HRPDAAV03-B | 03B | AADC/GDNF | SEQ ID NO:19 | Double-gene double-promoter framework |
| HRPDAAV03-C | 03C | AADC/GDNF | SEQ ID NO:20 | Double-gene double-promoter framework |
| HRPDAAV03-D | 03D | AADC/GDNF | SEQ ID NO:21 | Double-gene double-promoter framework |
| HRPDAAV03-E | 03E | AADC/GDNF | SEQ ID NO:22 | Double-gene double-promoter framework |
| HRPDAAV03-F | 03F | AADC/GDNF | SEQ ID NO:23 | Double-gene double-promoter framework |

| | | | | |
|---|---|---|---|---|
| (Note: AADC/GDNF indicates both AADC and GDNF are comprised, the same applies hereinafter) | | | | |

### Example 2. Validation of Transformation Efficiency of AADC for L-DOPA and GDNF Expression by Expression of AADC/GDNF Polynucleotides In Vitro

DOPA is mainly produced in the substantia nigra region of the midbrain, with the following signaling pathway:

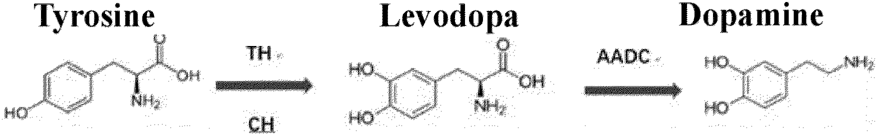

SHSY5Y is a human neuroblastoma cell line. In this example, a rAAV vector plasmid containing AADC/GDNF was transduced into SHSY5Y cells (ATCC), levodopa (L-DOPA) was added to the medium, and the supernatant was collected. 1. The concentration of dopamine (DOPA) was detected by HPLC to observe the transformation efficiency of L-DOPA to DOPA by the rAAV vector.

The method was as follows: on day 0, SHSY5Y cells were plated in a 6-well plate at 2E+5 to 6E+5 cells/well and incubated for 24 h to achieve a cell density of 60%-80% for transfection. On day 1, HRPDAAV01-A to HRPDAAV03-F transfection systems were separately prepared, including a tube A (2.5 µg of a rAAV plasmid, 250 µL of Opti-MEM (GIBCO, 31985088), and 5 µL of Lipofectamine 3000 (Thermo Fisher Scientific, L3000001)) and a tube B (250 µL of Opti-MEM and 7.5 µL of Lipofectamine 3000). After the tube A and the tube B were separately prepared, the resulting solutions were mixed well and left to stand for 2-3 min, and then the solution in the tube A was slowly added to the tube B. The resulting solution was gently pipetted and mixed well, and then left to stand for 10-15 min. 1.5 mL of a medium (DMEM/F12 (GIBCO, 31331093) + 15% FBS (GIBCO, 26140079)) was used for medium exchange before transfection, and then the prepared transfection system was added dropwise; 6 h after transfection, the medium was exchanged for 3 mL of a medium (DMEM/F12 + 15% FBS); 24 h after transfection, the medium was exchanged for 3 mL of a medium (DMEM/F12 + 15% FBS) containing L-DOPA (Sigma-Aldrich, D9628) (2 mMol); 48 h after transfection, the supernatant was collected and detected for DOPA concentration by HPLC to detect the expression and function of AADC.

HPLC steps were as follows: pre-cooled PBS (1 mg tissue: 10 µL PBS) was added during tissue lysis, and after a sample was thoroughly homogenized using a pre-cooled tissue homogenizer, 100 µL of homogenate was collected and used for HPLC experiments. An equal volume of a pre-cooled sample treatment solution (0.2 M perchloric acid, 0.2 mM sodium metabisulfite, and 0.01% EDTA 2Na) was added, and 0.6 µM DHBA was also contained as an internal standard. The mixture was centrifuged in a centrifuge pre-cooled at 4 °C at 10000 g for 20 min, and the supernatant was collected and subjected to pressure filtration with a 0.22 µm aqueous-phase microporous filter membrane. The product was stored at 20 °C. The sample was detected using an HPLC EDC system. The mobile phase was 16% aqueous methanol solution (containing 40 mM sodium acetate, 15 mM citric acid, 0.25 mM sodium octane sulfonate, and 0.2 mM EDTA 2Na, with a pH of 4.3). Chromatographic peaks were analyzed using the Yingpu chromatographic data workstation software V5.0 and peak areas were corrected using DHBA as the internal standard. A standard curve was fitted through the standard sample injection results, the content of dopamine in the sample was calculated, and the result was expressed by "ng dopamine/mg tissue". 2. The concentration of GDNF was detected by ELISA to observe the expression efficiency of GDNF by the rAAV vector.

ELISA experiments were performed strictly according to the kit (RayBiotech, ELH-DDC) instructions, and the specific experimental steps were as follows: pre-cooled PBS (1 mg tissue: 10 µL PBS) was added during tissue lysis, and after a sample was thoroughly homogenized using the pre-cooled tissue homogenizer, the supernatant was collected by centrifugation and used for the ELISA experiments. 100 µL of a standard substance or sample was added to each well of the kit (RayBiotech, ELH-DDC), and the mixture was incubated at room temperature for 2.5 h; 100 µL of biotin antibody was added to each well, and the mixture was incubated at room temperature for 1 h; 100 µL of Streptavidin solution was added to each well, and the mixture was incubated at room temperature for 45 min; and 100 µL of TMB was added to each well, and the mixture was incubated at room temperature for 30 min. 50 µL of stop solution was added to each well. Spectral reading was immediately performed at 450 nm.

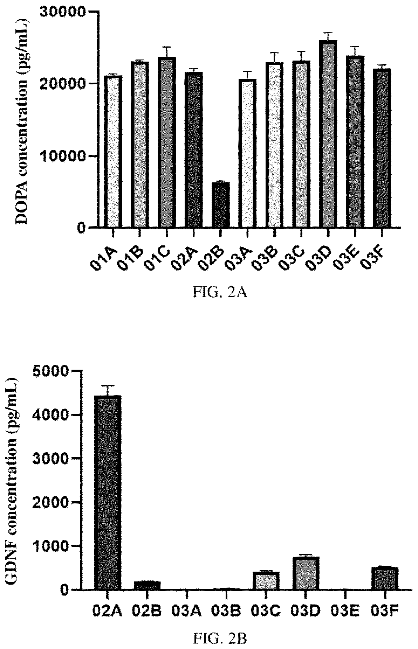

The detection results are shown in FIGs. 2A and 2B and show that HRPDAAV02-A and HRPDAAV03-D were relatively strong in the expression and function of AADC and GDNF.

### Example 3. Codon Optimization of AADC and GDNF

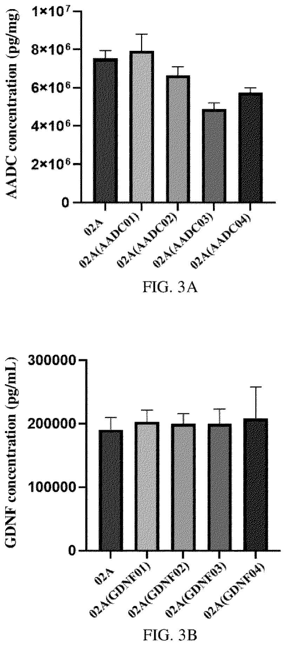

In this example, 4 codon-optimized polynucleotide sequences were separately designed for AADC and GDNF. The wild-type polynucleotide sequence of AADC is set forth in SEQ ID NO: 3, the codon-optimized sequences AADC01, AADC02, AADC03, and AADC04 are set forth in SEQ ID NOs: 4-7, respectively, and the sequence identities of SEQ ID NOs: 4-7 to SEQ ID NO: 3 are 80.83%, 81.21%, 79.18%, and 79.63%, respectively. The wild-type polynucleotide sequence of GDNF is set forth in SEQ ID NO: 8, the codon-optimized sequences GDNF01, GDNF02, GDNF03, and GDNF04 are set forth in SEQ ID NOs: 9-12, respectively, and the sequence identities of SEQ ID NOs: 9-12 to SEQ ID NO: 8 are 75.13%, 73.99%, 77.52%, and 77.83%, respectively. The AADC sequence in HRPDAAV02-A was replaced by AADC01, AADC02, AADC03, or AADC04 to obtain HRPDAAV02-A(AADC01), HRPDAAV02-A(AADC02), HRPDAAV02-A(AADC03), or HRPDAAV02-A(AADC04), which corresponded to 02A(AADC01), 02A(AADC02), 02A(AADC03), and 02A(AADC04) in the attached drawings, respectively. The GDNF sequence in HRPDAAV02-A was replaced by GDNF 01, GDNF 02, GDNF 03, or GDNF 04 to obtain HRPDAAV02-A(GDNF01), HRPDAAV02-A(GDNF02), HRPDAAV02-A(GDNF03), or HRPDAAV02-A(GDNF04), which corresponded to 02A(GDNF01), 02A(GDNF02), 02A(GDNF03), and 02A(GDNF04) in the attached drawings, respectively. The screening in Example 2 was then repeated to obtain AADC and GDNF codon-optimized sequences with the most strong expression and function, AADC01 and GDNF04. The results are shown in FIG. 3A and FIG. 3B.

AADC and GDNF in HRPDAAV02-A and HRPDAAV03-D were replaced by AADC01 (SEQ ID NO: 3) and GDNF04 (SEQ ID NO: 12) to obtain HRPDAAV02-AI (SEQ ID NO: 24) and HRPDAAV03-DI (SEQ ID NO: 25). HRPDAAV02-AI and HRPDAAV03-DI corresponded to 02AI and 03DI, respectively, in the attached drawings. The structures of both are shown in Table 3.

**Table 3. HRPDAAV02-AI and HRPDAAV03-DI sequence structures**

| HRPDAAV02-AI (i.e., 02AI) | | | | |
|---|---|---|---|---|
| Region | Starting position | Terminating position | Length of region | Sequence No. |
| 5'ITR | 1 | 141 | 141 | SEQ ID NO:26 |
| CMV enhancer | 157 | 442 | 286 | SEQ ID NO:27 |
| CBA promoter | 444 | 721 | 278 | SEQ ID NO:28 |
| Hybrid intron | 722 | 949 | 228 | SEQ ID NO:29 |
| AADC01 | 979 | 2418 | 1440 | SEQ ID NO:4 |
| P2A | 2419 | 2475 | 57 | SEQ ID NO:48 |
| GDNF04 | 2476 | 3111 | 636 | SEQ ID NO:12 |
| WPRE | 3118 | 3706 | 589 | SEQ ID NO:34 |
| SV40 poly(A) | 3707 | 3828 | 122 | SEQ ID NO:32 |
| 3'ITR | 3848 | 3988 | 141 | SEQ ID NO:31 |

| HRPDAAV03-DI (i.e., 03DI) | | | | |
|---|---|---|---|---|
| Region | Starting position | Terminating position | Length of region | Sequence No. |
| 5'ITR | 1 | 141 | 141 | SEQ ID NO:26 |
| CMV enhancer 2 | 151 | 454 | 286 | SEQ ID NO:37 |
| CMV promoter | 455 | 657 | 203 | SEQ ID NO:35 |
| β-globin intron | 658 | 1230 | 573 | SEQ ID NO:38 |
| GDNF04 | 1237 | 1872 | 636 | SEQ ID NO:12 |
| PA75 polyA | 1873 | 1947 | 75 | SEQ ID NO:36 |
| CMV enhancer | 1594 | 2239 | 304 | SEQ ID NO:27 |
| CBA promoter | 2241 | 2518 | 278 | SEQ ID NO:28 |
| Hybrid intron | 2519 | 2746 | 228 | SEQ ID NO:29 |
| AADC01 | 2746 | 4218 | 1443 | SEQ ID NO:4 |
| SV40 PolyA | 4225 | 4346 | 122 | SEQ ID NO:32 |
| 3'ITR | 4366 | 4506 | 141 | SEQ ID NO:31 |

### Example 4. Screening of AAV Serotypes

HRPDAAV-02A was packaged and purified by the AAV2 serotype (with the amino acid sequence set forth in SEQ ID NO: 41) or AAV9 serotype (with the amino acid sequence set forth in SEQ ID NO: 42) to obtain AAV2-02A and AAV9-02A.

The virus packaging process was as follows:
Packaging cells (viral production cells, Thermo A3152801) were seeded into a 3-L shake flask containing 1 L of a medium (Irvine 91165) at a density of 0.5 × 10⁶ vc/mL; after 3 days of culture on a CO₂ shaker, the cells were diluted to 3.0 × 10⁶ vc/mL using a fresh medium;
a total of 4 µg of three packaging plasmids (pHelper, pRC9, and pGOI) were added to 25 mL of fresh medium and mixed well; PEIpro (Polyplus 115-100) was added to another 25 mL fresh medium and mixed well; then the PEIpro dilution was poured into the plasmid dilution, and the resulting solution was quickly mixed well; after the solution was left to stand at room temperature for 15 min, the transfection complex was added to the packaging cells;
after 3 days of culture on the CO₂ shaker, 50 mL of cell lysis buffer (0.5 M hepes, 40 mM MgCl₂, and 10% tween-20) and 200 µL of Benzonase (Merck 1.01697.0010) were added, and the mixture was placed back into the CO₂ shaker and lysed for 4 h; the resulting solution was a harvest liquid.

The virus purification process was as follows:
The harvest liquid was filtered through a depth filter (Cobetter) to remove cell debris, then concentrated to 10 fold using a 100 kDa hollow fiber column (Repligen, D06-E100-05-N) and replaced into PBS (5 volumes);
the feed liquid after buffer exchange was loaded onto a PBS-balanced affinity chromatography column (Thermo A36652), rinsed with 5 column volumes of PBS, and
then eluted with 100 mM glycine with a pH of 3.0, the eluate was collected, an appropriate volume of 1 M BTP was added as soon as possible, and the pH was adjusted from 10.0 to about 7.0;
30 volumes of IEX equilibration buffer (25 mM BTP, pH 9.5) was added to dilute the eluate neutralization solution, the resulting solution was loaded onto a buffer-equilibrated anion exchange column (BIA 311.5113-2), rinsed with 10 column volumes of IEX equilibration buffer, and eluted with a gradient of NaCl salt concentrations, and the solid peaks were collected according to UV 260/280;
the eluate was concentrated to 1 mL through an ultrafiltration centrifuge tube (Merck, UFC810008), replaced into PBS + 0.01% Pluronic F-68, and filtered using a 0.22 µm needle filter to obtain a purified virus. AAV2-02A and AAV9-02A were injected into the striatum of the brain by stereotactic injection in rats (SD strain, male, purchased from Shanghai JieSiJie Laboratory Animal Co., Ltd.), the striata of some rats were collected 4 weeks and 8 weeks after injection, the expression of AADC and GDNF was detected by EILSA, and AADC immunofluorescence sectioning was performed 8 weeks after injection.

The method was as follows:
SD male rats were first anesthetized with 50 mg/kg Zoletil (i.p.), and the animals were bound to a cerebral stereotaxic apparatus; the skin was incised along the median sagittal direction of the cranium to expose the bregma; position 1: AP-0.0 mm, ML-2.6 mm and DV-4.7 mm and position 2: AP0.0 mm, ML-2.6 mm and DV-4.7 mm in the right striatal region were positioned. After the skull was opened by a dental drill, a micro-syringe was inserted into each site, and 2 µL of a test drug was automatically injected using a micro-peristaltic pump (0.2 µL/min). After the injection was completed, the needle was retained for 10 min and then slowly withdrawn, and the wound was sutured. Each rat was injected subcutaneously with 0.3 mL of levofloxacin injection to prevent infections.

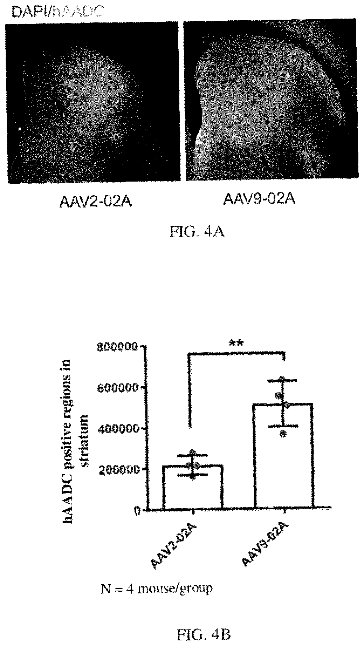

The rats were fed for another 8 weeks after the operation.
1) Cardiac perfusion was performed on the rats with 4% paraformaldehyde, the brain was collected and fixed for more than 24 h, and the sample was transferred into a 30% sucrose solution for full sediment and dehydration. The striatum coronal was continuously sectioned with a section thickness of 30 µm using a freezing microtome, and the sections were placed into a cryopreservation solution and stored in a refrigerator at 4 °C. The brain sections were washed three times in PBS, treated with 0.3% TritonX100 for 10 min, and blocked in PBS containing 10% goat serum for 1 h, the primary antibody was a rabbit anti-AADC antibody (Absin, abs110350) (1:1000), and the mixture was incubated overnight at 4 °C. The next day, the sections were washed 3 times with PBS, 5 min each time; a donkey anti-rabbit IgGAlexaFluor488 antibody (1:1500) and a nuclear dye DAPI (1:15000) were incubated at room temperature for 1 h, and the mixture was washed 3 times with PBS; the sections were mounted with a Dako antifade mountant. The sections were observed under an Olympus fluorescent microscope and photographed, as shown in FIG. 4A and FIG. 4B. The staining results show that the fluorescence area of AADC was significantly larger after the injection of AAV9-02A compared to AAV2-02A (P < 0.01, AAV2-02A vs AAV9-02A), demonstrating that AAV9 has higher expression efficiency in the striatal region than that of AAV2.
2) The rats were fed for 4 weeks or 8 weeks after the brain positioning operation, and then the striatum tissue at the injection side was directly extracted by decapitation. The tissue was weighed and cryopreserved in a refrigerator at -80 °C. Pre-cooled PBS (1 mg tissue:10 µL PBS) was added during tissue lysis, and after a sample was thoroughly homogenized using a pre-cooled tissue homogenizer, 100 µL of homogenate was collected and used for HPLC experiments, 40 µL of homogenate was collected and used for tissue DNA extraction and qPCR experiments, and the remaining volume of homogenate was used for ELISA experiments.

The concentrations of AADC and GDNF were detected by ELISA. ELISA experiments were performed strictly according to the kit (RayBiotech, ELH-DDC) instructions, and the specific experimental steps were as follows:
The method for detecting AADC was as follows: 100 µL of dissolved standard substance or sample was added to each well of the kit (RayBiotech, ELH-DDC), and the mixture was incubated at room temperature for 2.5 h, spun to dryness, and washed; 100 µL of biotin antibody was added to each well, and the mixture was incubated at room temperature for 1 h, spun to dryness, and washed; 100 µL of Streptavidin solution was added to each well, and the mixture was incubated at room temperature for 45 min, spun to dryness, and washed; and 100 µL of TMB was added to each well, and the mixture was incubated at room temperature for 30 min, spun to dryness, and washed. 50 µL of stop solution was added to each well. Spectral reading was immediately performed at 450 nm.

The method for detecting GDNF was as follows: 100 µL of dissolved standard substance or sample was added to each well of the kit (R&D, DY212), and the mixture was incubated at room temperature for 2 h, spun to dryness, and washed; 100 µL of test antibody was added to each well, and the mixture was incubated at room temperature for 2 h, spun to dryness, and washed; 100 µL of Streptavidin-HRP solution was added to each well, and the mixture was incubated at room temperature for 20 min, spun to dryness, and washed; and 100 µL of substrate solution was added to each well, and the mixture was incubated at room temperature for 20 min. 50 µL of stop solution was added to each well. Spectral reading was immediately performed at 450 nm.

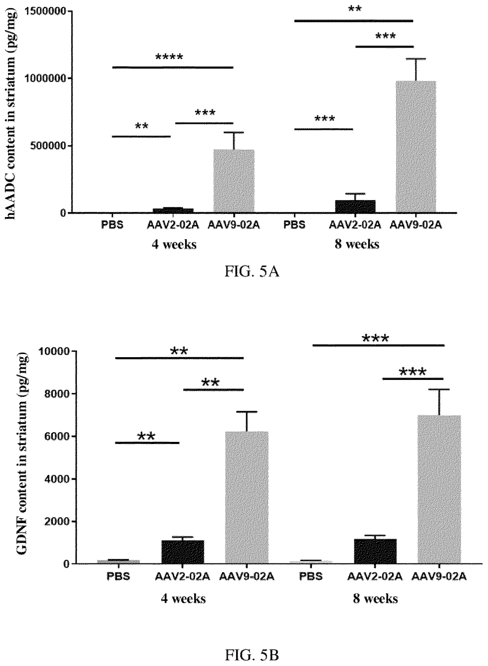

As shown in FIG. 5A and FIG. 5B, the detection results of the expression level of the target gene show that the expression levels of AADC and GDNF in the striatum of the AAV9-02A group were significantly higher than those in the AAV2-02A group, demonstrating that AAV9 has higher transduction efficiency in the striatum region than that of AAV2.

### Example 5. Efficacy Verification in MPTP-Induced Mouse PD Model

In this example, 1-methyl-4-phenyl-1,2,3,6-tetrahydropyridine (MPTP) was selected to build a mouse Parkinson's disease model. MPTP has high lipid solubility, is easy to permeate the blood-cerebrospinal fluid barrier, and can be converted into methyl-phenylpyridine ions (MPP+) under the action of glial cell monoamine oxidase B after entering the brain, and MPP+ is actively taken into the mitochondria of dopaminergic (DA) neurons by DA transporters, causing the degeneration and death of DAergic neurons. This process well simulates the symptoms and pathogenesis of PD. Adult male mice are intraperitoneally injected with MPTP for 5 consecutive days, which can induce the apoptosis of DA neurons and result in Parkinson's disease-like symptoms in the mice.

The method was as follows: mice were divided into four groups, i.e., a sham surgery control group (i.e., normal control), a model control group (i.e., MPTP model control, the same applied hereinafter), a 02AI group (i.e., HRPDAAV-02AI), and a 03DI group (i.e., HRPDAAV-03DI). HRPDAAV-02AI and HRPDAAV-03DI were injected into the unilateral striatum of 8-week male mice, both using AAV9 capsids. Mice in the sham surgery control group and the model control group were subjected to sham surgery. C57BL/6N male mice were taken and anesthetized by intraperitoneal injection of 1% pentobarbital sodium (at a dose of 35-50 mg/kg); after the mice were confirmed to be in an anesthetic state, the mice were fixed to a stereotaxic apparatus (RWD, model: 68018) in the prone position; the scalp was incised after the instruments were sterilized conventionally, and soft tissues and periosteum were stripped bluntly to expose the right parietal bone; and a bone window was drilled at the position of 0.8 mm (AP = +0.8 mm) in the front direction of the bregma and 1.8 mm (ML = 1.8 mm) beside the midline. AAV drugs were injected using a micro-syringe (specification: 1 µL; outer diameter: 0.55 mm) at a needle insertion speed of 1 mm/min; the needle was inserted to subdural 3 mm (DV = -3 mm), and withdrawn to 2.8 mm (DV = -2.8 mm); the liquid was injected at a constant speed of 0.2 µL/min; after the injection was completed, the needle was retained for 3 min, and the needle withdrawing speed was 1 mm/min.

28 days after the striatum injection, the mice in the model control group and the administration groups were intraperitoneally injected with 30 mg/kg of MPTP with an injection volume of 10 mL/kg, and the mice in the sham surgery group were intraperitoneally injected with 10 mL/kg of normal saline. 5 days after molding, the mice were allowed to rest for 3 days and subjected to the behavioral detection.

### 1) Rotation test

To verify the response of AADC delivered by the HRPDAAV-02AI and HRPDAAV-03DI drugs to L-DOPA after unilateral striatum expression, a rotation test was performed. Levodopa (L-DOPA) (20 mg/kg) + benserazide (6 mg/kg) were intragastrically administered to the experimental mice in each group. After levodopa was administered to the mice, the mice were placed in a test environment for adaptation; 45 min after administration, the number of rotations of the mice began to be recorded; and the rotation at the healthy side (rotation at the left side) of the mice within 30 min was detected.

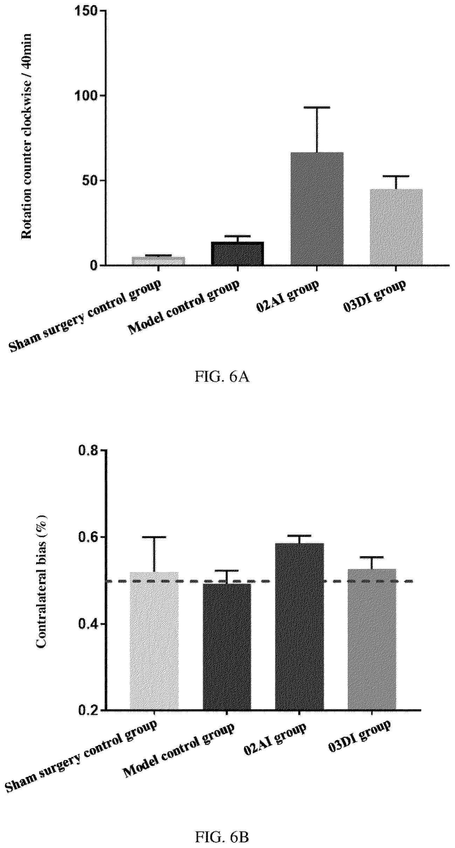

The results are shown in FIG. 6A and Table 4. Compared to the sham surgery control group and the model control group, the HRPDAAV-02AI and HRPDAAV-03DI administration groups showed significantly increased numbers of rotations after L-DOPA was administered ("02AI group vs. model control group", *p* = 0.07; "03DI group vs. model control group", *p* = 0.01), demonstrating that AADC delivered and expressed by AAV has a very good response to L-DOPA.

**Table 4. Rotation test results in mice**

| Group | Number of animals | Mean ± SEM |
|---|---|---|
| Sham surgery control group | 6 | 5.00±0.86 |
| Model control group | 13 | 14.08±3.19 |
| 02AI group | 11 | 66.45±26.59 |
| 03DI group | 12 | 44.92±7.72 |

### 2) Cylinder test

To detect the forelimb utilization preference of mice in the background state, a cylinder test was performed. Four days after the last injection of MPTP in the mice, L-DOPA (10 mg/kg) + benserazide (3 mg/kg) were intragastrically administered; the mouse was placed in a transparent cylinder (with a diameter of 10 cm and a height of 45 cm), and the number of times the mouse touched the wall with the left forelimb, the right forelimb, and the bilateral forelimbs within 5 min was observed and recorded; and the mouse touched the wall with one or both of forelimbs using the hind limbs as fulcrums, and then returned to the bottom of the cylinder, which was recorded as one time (one limb of the mouse touched the wall first, and the other limb touched the wall later, which was recorded as bilateral touching). The cylinder was wiped with alcohol after each mouse was tested. Left forelimb utilization of the mice was analyzed: left forelimb usage rate (%) = (left + 0.5 bilateral)/(right + left + bilateral) × 100.

The results are shown in FIG. 6B and Table 5 and show that after administration of L-DOPA, the contralateral forelimb utilization rates of the HRPDAAV-02AI and HRPDAAV-03DI administration groups were increased compared to the model control group, with 20.4% increase in the 02AI group and 8.2% increase in the 03DI group compared to the model control group, further demonstrating the response effect of AADC delivered and expressed by AAV on L-DOPA.

**Table 5. Cylinder test results for mice**

| Group | Number of animals | Mean ± SEM | Change value compared to model control group (%) |
|---|---|---|---|
| Sham surgery control group | 6 | 0.52±0.08 | 6.1% |
| Model control group | 13 | 0.49±0.03 | 0 |
| 02AI group | 11 | 0.59±0.02 | 20.4% |
| 03DI group | 12 | 0.53±0.03 | 8.2% |

### 3) Dopamine detection in striatum and blood

After the behavioral tests, the mice in each group were taken, and L-DOPA (20 mg/kg, 5 mL/kg) and benserazide (5 mg/kg, 5 mL/kg) were intragastrically administered. The mice were sacrificed 1 h after administration; peripheral blood was collected before sacrifice, and dopamine in the blood was detected. The striata at the healthy side and the affected side were separately collected after sacrifice. DOPA was detected by using a UPLC-MS/MS method.

Referring to FIG. 7A and Table 6-1, the DOPA detection in the left striatum (non-administration side) showed a significant decrease in DOPA for both the model control group and the administration groups compared to the sham surgery control group, demonstrating the success of MPTP molding.

**Table 6-1. DOPA detection results in mouse left striatum (non-administration side)**

| Group | Number of animals | Mean ± SEM |
|---|---|---|
| Sham surgery control group | 3 | 1122.0±49.7 |
| Model control group | 7 | 224.9±44.6 |
| 02AI group | 4 | 274.7±4.2 |
| 03DI group | 6 | 287.9±34.2 |

Referring to FIG. 7B and Table 6-2, the DOPA detection in the right striatum (administration side) showed a significant increase in DOPA for both HRPDAAV-02AI and HRPDAAV-03DI administration groups compared to the model control group. The DOPA levels returned to the level of the sham surgery control group.

**Table 6-2. DOPA detection results in mouse right striatum (administration side)**

| Group | Number of animals | Mean ± SEM | Fold change compared to model control group |
|---|---|---|---|
| Sham surgery control group | 3 | 1473.6±72.6 | 4.43 |
| Model control | 7 | 271.3±48.1 | 0 |
| group | | | |
| 02AI group | 4 | 1277.1±385.1 | 3.71 |
| 03DI group | 6 | 880.8±200.2 | 2.25 |

Referring to FIG. 7C and Table 6-3, the plasma DOPA detection found that the groups differed little, demonstrating that the effects of HRPDAAV-02AI and HRPDAAV-03DI are primarily localized to the striatum of the brain, with no significant leakage.

**Table 6-3. DOPA detection results in mouse plasma**

| Group | Number of animals | Mean ± SEM |
|---|---|---|
| Sham surgery control group | 3 | 6.33±2.67 |
| Model control group | 7 | 4.44±2.19 |
| 02AI group | 4 | 1.29±0.68 |
| 03DI group | 6 | 1.85±0.29 |

### 4) Immunohistochemical detection of brain tissue

Cardiac perfusion was performed on mice in each group, and the brain tissue was collected for fluorescence immunohistochemical detection. Specifically, after general observation was performed on the experimental mice, the heart tissue in the thoracic cavity was dissociated, a venous transfusion needle was inserted from the left ventricle and fixed, and the right auricle was cut off; perfusion was performed on the right auricle by using pre-cooled PBS until a transparent liquid flowed out, and then perfusion was performed on the heart by using 4% paraformaldehyde; the brain tissue was cut off, collected, and placed into a fixing agent (4% paraformaldehyde solution); and the fixed tissue was stained with tyrosine dehydrogenase TH by paraffin sectioning.

Referring to FIG. 7D and Table 7, the fluorescence immunohistochemical analysis found that TH fluorescence intensities were comparable on both sides in the sham surgery control group and the model control group, while TH fluorescence intensities were significantly increased in the right striatum (administration side) compared to the left striatum (non-administration side) in the HRPDAAV-02AI and HRPDAAV-03DI administration groups, demonstrating that the HRPDAAV-02AI and HRPDAAV-03DI drugs have good protection effects on mouse dopamine neurons.

**Table 7. Immunohistochemical detection results of mouse brain tissue**

| Group | Number of animals | Right side average/left side average |
|---|---|---|
| Sham surgery control group | 3 | 0.94 |
| Model control group | 3 | 0.88 |
| 02AI group | 4 | 2.34 |
| 03DI group | 3 | 1.26 |

### Example 6. Efficacy Verification in 6-OHDA-Induced Rat PD Model

In this example, 6-OHDA was used to establish a rat PD model. 6-OHDA is a neurotoxin, and is often mistakenly taken into DAergic neurons as a transmitter because the structure is similar to dopamine (DA). The death of the DAergic neurons is selectively caused by the formation of hydroxyl radicals, the inhibition of mitochondrial oxidation of respiratory chain complexes, and the interference in ATP synthesis. The DA content in the damaged striatum is decreased, causing rats to develop symptoms similar to human PD. The model has the characteristics of reliability, stability and irreversibility, in which the behavioral defects can be intuitively and quantitatively analyzed, and the model can be widely used for evaluating drugs against Parkinson's disease, especially drugs that have effects on DA and receptors thereof, so that a single-side MFB injection 6-OHDA-induced Parkinson's disease model was adopted in this example to evaluate the treatment effects of HRPDAAV-02AI and HRPDAAV-03DI on Parkinson's disease.

The method was as follows: SD rats were divided into 6 experimental groups, i.e., a sham surgery control group (i.e., normal control group), a model control group, a 02AI high-dose group (1E+10 vg/dose), a 02AI low-dose group (2E+9 vg/dose), a 03DI high-dose group (1E+10 vg/dose), and a 03DI low-dose group (2E+9 vg/dose), and AAV9 capsids were used in all of them. The rat was anesthetized by intraperitoneal injection of 3% pentobarbital sodium (at a dose of 50-60 mg/kg), and then fixed in the prone position; the scalp was incised after the instruments were sterilized conventionally, and soft tissues and periosteum were stripped bluntly to expose the right parietal bone; connective tissues near the midline were removed, and a sagittal suture, a bregma and a herringbone point were determined; a micro-syringe (specification: 10 µL; outer diameter: 0.5 mm) with a modeling reagent pumped in was fixed on a stereotaxic apparatus for coordinate positioning; the striatum was positioned on the right side of the rat by taking the bregma as a positioning central point; and a bone window was drilled at the position of 0.0 mm (AP = +0.0 mm) in the front direction of the bregma and 3 mm (ML = 3 mm) beside the midline. The needle insertion speed was 1 mm/min; the needle was inserted to subdural 6 mm (DV = -6 mm), and withdrawn to 5.8 mm (DV = -5.8 mm); 2 µL of liquid was injected at a constant speed of 0.6 µL/min; after the injection was completed, the needle was withdrawn to 3.8 mm (DV = -3.8 mm), and then 2 µL of liquid was injected at a constant speed of 0.6 µL/min; the needle was retained for 3 min after the two-point injection was completed, and the needle withdrawing speed was 2 mm/min. The scalp was sutured and disinfected, and the rat was placed on a heating pad at 37 °C until the rat was awake; and penicillin sodium (80,000 units/day) was injected and an ibuprofen oral liquid was intragastrically administered for three consecutive days after the operation.

Three weeks after the striatum was injected with the drug, modeling was performed, and 3% pentobarbital sodium was adopted for anesthesia by intraperitoneal injection at an injection dosage of 60 mg/kg with an injection volume of 2 mL/kg. The rat after anesthesia and induction was transferred to an operation table and fixed to the cerebral stereotaxic apparatus in the prone position to ensure that the brain was on the horizontal plane; eyelid reflexes and nociceptive responses of the rat were observed, and the operation could be started after the eyelid reflex reactions and the nociceptive responses of limbs and the tail disappeared. A minimal-size drill was used to vertically drill a hole, taking care not to deflect and injure the brain tissue. The hole was positioned on the right side of the rat (administrating side), and a bone window was drilled at the position of 2.0 mm (AP = -2.0 mm) in the rear direction of the bregma and 2 mm (ML = 2 mm) beside the midline. The needle insertion speed was 6 mm/min; the needle was inserted to subdural 8.8 mm (DV = -8.8 mm), and withdrawn to 8.5 mm (-8.5 mm); 6-OHDA was injected at a constant speed of 0.6 µL/min; 4 µL of 6-OHDA (5 µg/µL) was injected into each rat; after the injection was completed, the needle was retained for 3 min, and the needle withdrawing speed was 2 mm/min. The scalp was sutured and disinfected, and the rat was placed on the heating pad at 37 °C until the rat was awake; and penicillin sodium (80,000 units/day) was injected and the ibuprofen oral liquid was intragastrically administered for three consecutive days after the operation.

Three weeks after molding, the response of the rats to the apomorphine drug was detected. The rats were intraperitoneally injected with apomorphine (0.5 mg/kg). 10 min later, the rats were placed in the test environment, and the number of rotations of the rats within 30 min was recorded.

Referring to FIG. 8A, the 6-OHDA-modeled rats all had significantly increased number of rotations compared to the sham surgery control group, demonstrating that the modeling experiment was successful.

### 1) Rotation test

To detect the response of exogenously expressed AADC in the striatum to exogenous L-DOPA, the rotation at the healthy side (rotation at the left side) of the rats after intragastric administration of L-DOPA was detected. The rats were orally and intragastrically administered L-DOPA (20 mg/kg, 5 mL/kg) + benserazide (5 mg/kg, 5 mL/kg). 45 min later, the rats were placed in the test environment, and the number of rotations of the rats within 30 min was recorded.

Referring to FIG. 8B, the number of rotations of the model control group was not significantly increased compared to the sham surgery control group, while the numbers of rotations of the HRPDAAV-02AI and HRPDAAV-03DI high and low dose administration groups were all significantly increased compared to the model control group, indicating that AADC expressed in the striatum delivered by AAV has a good response effect to exogenous L-DOPA drugs.

### 2) Cylinder test

To detect the forelimb utilization preference of rats in the background state, a cylinder test was performed. The rat in each group was placed in a transparent cylinder with a diameter of 20 cm, and the number of times the rat touched the wall with the left forelimb, the right forelimb, and the bilateral forelimbs within 10 min was observed and recorded. The rat touched the wall with one or both of forelimbs using the hind limbs as fulcrums, and then returned to the bottom of the cylinder, which was recorded as one time (one limb of the rat touched the wall first, and the other limb touched the wall later, which was recorded as bilateral touching). The left forelimb usage rate of the rat was calculated: left forelimb usage rate (%) = (left + 0.5 bilateral)/(right + left + bilateral) × 100.

Referring to FIG. 8C, the left forelimb utilization rates were all decreased in the model rat and the administration groups compared to the blank control group, further verifying the validity of the model.

To detect the utilization of L-DOPA by rats treated with AAV, the rats were orally and intragastrically administered L-DOPA (5 mg/kg, 5 mL/kg) + benserazide (2.5 mg/kg, 5 mL/kg). 30 min later, the rat was placed in the transparent cylinder with the diameter of 20 cm, and the number of times the rat touched the wall with the left forelimb, the right forelimb, and the bilateral forelimbs within 10 min was observed and recorded. Referring to FIG. 8D, the left forelimb utilization rate of the model control group after L-DOPA supplementation was not significantly improved, while the left forelimb utilization rates of the HRPDAAV-02AI and HRPDAAV-03DI groups were significantly improved compared to the model control group, demonstrating that the utilization rate of L-DOPA by rats after administration is significantly improved, and further demonstrating that AADC expressed in the striatum delivered by AAV has a very good response effect to exogenous L-DOPA drugs.

### 3) Detection of L-DOPA metabolizing ability of striatum after administration of HRPDAAV-02AI and HRPDAAV-03DI

After the behavioral tests, the rats in each group were taken, and L-DOPA (20 mg/kg, 5 mL/kg) and benserazide (5 mg/kg, 5 mL/kg) were intragastrically administered. The rats were sacrificed 1 h after the end of the administration. The striata at the healthy side and the affected side were separately collected after sacrifice. DOPA was detected by using a UPLC-MS/MS method.

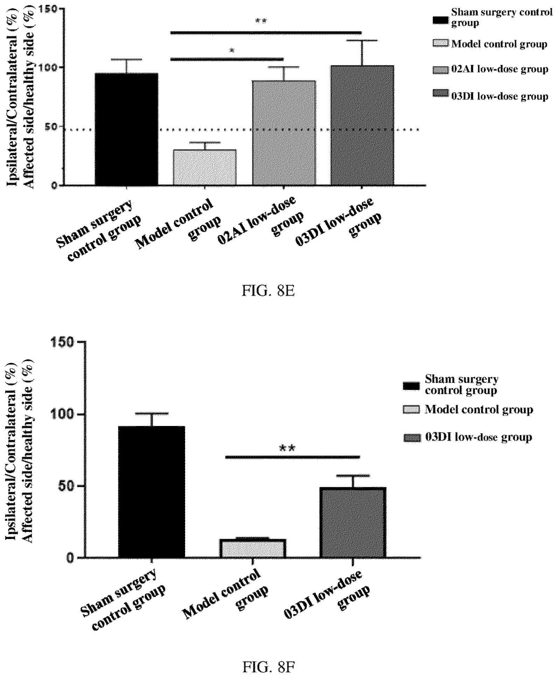

Referring to FIG. 8E and Table 8, the DOPA ratio of affected side/healthy side was detected, and it was found that DOPA at the affected side of the model control group was significantly decreased compared to the administration groups, demonstrating the success of 6-OHDA molding.

**Table 8. DOPA detection results in rat bilateral striata**

| Group | Number of animals | Affected side (mean ± SEM) | Healthy side (mean ± SEM) | Affected side/healthy side (%) |
|---|---|---|---|---|
| Sham surgery control group | 3 | 27729.03±8121.90 | 28687.57±3329.29 | 95.49±20.20 |
| Model control group | 9 | 7299.01±3113.39 | 25872.46±5398.04 | 30.05±19.39* |
| 02AI low-dose group | 9 | 17323.06±5465.61 | 25432.89±12362.06 | 77.76±33.49 |
| 03DI low-dose group | 9 | 19029.10±6859.95 | 20756.06±5501.87 | 101.99±63.33 |

The DOPA detection in the striatum at the administration side (affected side) showed a significant increase in DOPA for both HRPDAAV-02AI and HRPDAAV-03DI administration groups compared to the model control group. Compared to the model control group, the DOPA ratio of affected side/healthy side of each administration group was significantly improved, demonstrating that the L-DOPA drug metabolizing ability of the striatum after administration of HRPDAAV-02AI and HRPDAAV-03DI is significantly improved.

### 4) Detection of protection effect of HRPDAAV-03DI on DA neurons after administration

Cardiac perfusion was performed on rats in each group, and the brain tissue was collected for fluorescence immunohistochemical detection. Specifically, after general observation was performed on the experimental rats, the heart tissue in the thoracic cavity was dissociated, a venous transfusion needle was inserted from the left ventricle and fixed, and the right auricle was cut off; perfusion was performed on the right auricle by using pre-cooled PBS until a transparent liquid flowed out, and then perfusion was performed on the heart by using 4% paraformaldehyde; the brain tissue was cut off, collected, and placed into a fixing agent (4% paraformaldehyde solution); and the fixed tissue was stained with tyrosine dehydrogenase TH by paraffin sectioning.

Referring to FIG. 8F, the TH positive cell ratio of affected side/contralateral side was analyzed by fluorescence immunohistochemistry, and it was found that the TH cell viability at the affected side was less than 15% in the experimental model control group compared to the sham surgery group, indicating that 6-OHDA modeling was successful. The TH fluorescence intensity ratio of the affected side to the healthy side of the HRPDAAV-03DI low-dose administration group was significantly increased compared to that of the model control group, demonstrating that the HRPDAAV-03DI drug has a very good protection effect on rat dopamine neurons and improves the TH cell viability.

The sequences used in the present disclosure are as follows:
>Amino acid sequence of AADC (SEQ ID NO: 1) 
MNASEFRRRGKEMVDYVANYMEGIEGRQVYPDVEPGYLRPLIPAAAPQEPDTF
EDIINDVEKIIMPGVTHWHSPYFFAYFPTASSYPAMLADMLCGAIGCIGFSWAASP
ACTELETVMMDWLGKMLELPKAFLNEKAGEGGGVIQGSASEATLVALLAART
KVIHRLQAASPELTQAAIMEKLVAYSSDQAHSSVERAGLIGGVKLKAIPSDGNFA
MRASALQEALERDKAAGLIPFFMVATLGTTTCCSFDNLLEVGPICNKEDIWLHV
DAAYAGSAFICPEFRHLLNGVEFADSFNFNPHKWLLVNFDCSAMWVKKRTDLT
GAFRLDPTYLKHSHQDSGLITDYRHWQIPLGRRFRSLKMWFVFRMYGVKGLQ
 
AYIRKHVQLSHEFESLVRQDPRFEICVEVILGLVCFRLKGSNKVNEALLQRINSA
KKIHLVPCHLRDKFVLRFAICSRTVESAHVQRAWEHIKELAADVLRAERE
>Amino acid sequence of GDNF (SEQ ID NO: 2) 
MKLWDVVAVCLVLLHTASAFPLPAGKRPPEAPAEDRSLGRRRAPFALSSDSNMP
EDYPDQFDDVMDFIQATIKRLKRSPDKQMAVLPRRERNRQAAAANPENSRGKG
RRGQRGKNRGCVLTAIHLNVTDLGLGYETKEELIFRYCSGSCDAAETTYDKILK
NLSRNRRLVSDKVGQACCRPIAFDDDLSFLDDNLVYHILRKHSAKRCGCI
> AADC wild-type DNA (SEQ ID NO: 3) 
atgaacgcaagtgaattccgaaggagagggaaggagatggtggattacatggccaactacatggaaggcattgagggacgc
caggtctaccctgacgtggagcccgggtacctgcggccgctgatccctgccgctgcccctcaggagccagacacgtttgag
gacatcatcaacgacgttgagaagataatcatgcctggggtgacgcactggcacagcccctacttcttcgcctacttccccact
gccagctcgtacccggccatgcttgcggacatgctgtgcggggccattggctgcatcggcttctcctgggcggcaagcccag
catgcacagagctggagactgtgatgatggactggctcgggaagatgctggaactaccaaaggcatttttgaatgagaaagct
ggagaagggggaggagtgatccagggaagtgccagtgaagccaccctggtggccctgctggccgctcggaccaaagtga
tccatcggctgcaggcagcgtccccagagctcacacaggccgctatcatggagaagctggtggcttactcatccgatcaggc
acactcctcagtggaaagagctgggttaattggtggagtgaaattaaaagccatcccctcagatggcaacttcgccatgcgtgc
gtctgccctgcaggaagccctggagagagacaaagcggctggcctgattcctttctttatggttgccaccctggggaccacaa
catgctgctcctttgacaatctcttagaagtcggtcctatctgcaacaaggaagacatatggctgcacgttgatgcagcctacgc
aggcagtgcattcatctgccctgagttccggcaccttctgaatggagtggagtttgcagattcattcaactttaatccccacaaat
ggctattggtgaattttgactgttctgccatgtgggtgaaaaagagaacagacttaacgggagcctttagactggaccccactta
cctgaagcacagccatcaggattcagggcttatcactgactaccggcattggcagataccactgggcagaagatttcgctcttt
gaaaatgtggtttgtatttaggatgtatggagtcaaaggactgcaggcttatatccgcaagcatgtccagctgtcccatgagtttg
agtcactggtgcgccaggatccccgctttgaaatctgtgtggaagtcattctggggcttgtctgctttcggctaaagggttccaac
aaagtgaatgaagctcttctgcaaagaataaacagtgccaaaaaaatccacttggttccatgtcacctcagggacaagtttgtcc
tgcgctttgccatctgttctcgcacggtggaatctgcccatgtgcagcgggcctgggaacacatcaaagagctggcggccga
cgtgctgcgagcagagagggagtag
>AADC01 DNA (SEQ ID NO: 4) 
atgaatgctagcgagtttcgcagaaggggcaaggagatggtggattacgtggctaattacatggagggcatcgagggccgg
caggtgtacccagatgtggagccaggctacctgagaccactgatccctgctgccgccccacaggagccagacaccttcgag
gatatcatcaatgacgtggagaagatcatcatgcctggcgtgacccactggcatagcccatacttctttgcctactttccaacag
cctcttcctatcccgctatgctggctgatatgctgtgcggcgctatcggctgcatcggcttctcttgggccgctagccctgcttgc
acagagctggagaccgtgatgatggattggctgggcaagatgctggagctgcctaaggcctttctgaacgagaaggctggc
gagggcggcggcgtgatccagggctctgcctccgaggctacactggtggctctgctggctgcccggacaaaggtaatccac
agactgcaggctgcctctccagagctgacccaggccgctatcatggagaagctggtggcttactctagcgaccaggctcattc
ttccgtggagcgggctggcctgatcggcggcgtgaagctgaaggccatcccttccgacggcaatttcgccatgagagcttctg
ccctgcaggaggctctggagagagataaggctgccggcctgatccctttctttatggtggccaccctgggcacaaccacctgt
tgctccttcgacaacctgctggaggtgggccctatctgtaacaaggaggacatctggctgcatgtggatgccgcttatgctggc
tctgcctttatctgccccgagtttagacatctgctgaacggcgtggagtttgccgacagctttaacttcaaccctcacaagtggct
gctggtgaattttgactgctctgccatgtgggtgaagaagaggacagacctgaccggcgcttttaggctggatccaacctacct
gaagcacagccatcaggacagcggcctgatcacagactacagacactggcagatcccactgggcagaaggtttcggtctct
gaagatgtggttcgtgtttcgcatgtatggcgtgaagggcctgcaggcttatatccggaagcacgtgcagctgtctcatgagttc
 
gagtccctggtgcggcaggacccaagatttgagatctgcgtggaggtaatcctgggcctggtgtgcttcaggctgaagggctc
taataaggtgaacgaggctctgctgcagaggatcaacagcgccaagaagatccatctggtgccttgtcatctgagggacaagt
tcgtgctgagattcgccatctgctctagaacagtggagtctgctcatgtgcagagggcttgggagcatatcaaggagctggcc
gctgacgtgctgagggctgagagggagtga
>AADC02 DNA (SEQ ID NO: 5) 
atgaacgctagcgagtttcggaggagaggcaaggagatggtggactacgtggccaactacatggagggcatcgagggcag
acaggtgtacccagacgtggagcctggctatctgcggccactgatccctgctgccgccccacaggagccagatacctttgag
gacatcatcaacgacgtggagaagatcatcatgcccggcgtgacccactggcattccccttacttctttgcctactttcctacagc
ttcttcctatcctgccatgctggccgatatgctgtgcggcgctatcggctgcatcggcttctcttgggccgcttcccccgcttgca
ccgagctggagaccgtgatgatggattggctgggcaagatgctggagctgcccaaggctttcctgaatgagaaggctggcg
agggcggcggcgtgatccagggctctgcttccgaggccacactggtggccctgctggctgccaggacaaaggtaatccaca
gactgcaggctgcctcccccgagctgacccaggccgctatcatggagaagctggtggcttactcctctgaccaggctcactct
tccgtggagagagctggcctgatcggcggcgtgaagctgaaggctatcccttctgatggcaatttcgccatgagggcttctgct
ctgcaggaggctctggagagggataaggctgccggcctgatcccctttttcatggtggccaccctgggcacaaccacctgttg
cagctttgacaatctgctggaggtgggccctatctgcaacaaggaggacatctggctgcacgtggatgctgcctacgctggca
gcgccttcatctgtcccgagtttaggcacctgctgaatggcgtggagtttgctgactcctttaatttcaacccccataagtggctg
ctggtgaacttcgactgctctgctatgtgggtgaagaagaggacagatctgacaggcgcctttaggctggacccaacctacct
gaagcactcccatcaggatagcggcctgatcaccgactaccggcactggcagatccctctgggcaggcgctttagatccctg
aagatgtggttcgtgtttaggatgtacggcgtgaagggcctgcaggcttatatccggaagcatgtgcagctgtcccacgagttc
gagtctctggtgcggcaggacccaaggtttgagatctgcgtggaggtaatcctgggcctggtgtgcttcagactgaagggctc
caataaggtgaacgaggctctgctgcagagaatcaactccgccaagaagatccacctggtgccttgtcacctgagggataagt
tcgtgctgaggtttgccatctgctctagaaccgtggagagcgcccatgtgcagagggcttgggagcacatcaaggagctggc
tgccgacgtgctgagggctgagagagagtga
>AADC03 DNA (SEQ ID NO: 6) 
atgaatgcctctgagttccgcagacgcggcaaggagatggtggactacgtggctaactacatggagggcatcgagggcaga
caggtgtaccccgacgtggagcctggctatctgaggccactgatccctgctgcccctcaggagcccgacaccttcgagg
atatcatcaatgacgtggagaagatcatcatgccaggcgtgacccattggcactctccatacttctttgcttacttccctaccgcct
cttcctatccagctatgctggccgatatgctgtgcggcgccatcggctgcatcggctttagctgggctgcctctccagcttgtac
cgagctggagacagtgatgatggactggctgggcaagatgctggagctgcctaaggcctttctgaacgagaaggccggcga
gggcggcggcgtgatccagggctccgcttctgaggccaccctggtggctctgctggctgccagaaccaaggtaatccacag
gctgcaggccgctagccctgagctgacccaggccgctatcatggagaagctggtggcttactcttccgaccaggcccattcta
gcgtggagagggctggcctgatcggcggcgtgaagctgaaggctatcccttctgacggcaacttcgccatgagagctagcg
ccctgcaggaggccctggagcgggacaaggctgccggcctgatcccatttttcatggtggctaccctgggcacaacaacctg
ctgttctttcgacaacctgctggaggtgggccctatctgcaataaggaggacatctggctgcatgtggatgctgcctatgctggc
agcgcctttatctgcccagagttcaggcatctgctgaacggcgtggagtttgccgactcttttaacttcaatccccataagtggct
gctggtgaactttgactgtagcgccatgtgggtgaagaagaggacagatctgaccggcgcctttcggctggatcctacctacct
gaagcactctcatcaggattctggcctgatcaccgattacagacattggcagatccctctgggccggagatttcggagcctgaa
gatgtggttcgtgtttcggatgtatggcgtgaagggcctgcaggcctatatcagaaagcacgtgcagctgtcccacgagtttga
gagcctggtgcgccaggacccacggtttgagatctgcgtggaggtaatcctgggcctggtgtgcttcaggctgaagggctcta
ataaggtgaacgaggctctgctgcagcgcatcaactccgctaagaagatccacctggtgccttgtcacctgcgggacaagttc
gtgctgaggtttgccatctgctccagaaccgtggagtctgcccatgtgcagagagcttgggagcatatcaaggagctggccgc
 tgatgtgctgagggctgagagagagtga
>AADC04 DNA (SEQ ID NO: 7) 
atgaacgcctctgagtttcggaggaggggcaaggagatggtggactacgtggctaactatatggagggcatcgagggcaga
caggtgtaccctgacgtggagccaggctacctgagaccactgatcccagccgccgctcctcaggagcctgacacctttgagg
acatcatcaatgatgtggagaagatcatcatgcctggcgtgacccattggcacagcccttactttttcgcctatttcccaaccgct
agctcctatccagccatgctggctgatatgctgtgcggcgctatcggctgtatcggctttagctgggctgcctctcctgcttgtac
agagctggagacagtgatgatggattggctgggcaagatgctggagctgcccaaggcctttctgaacgagaaggccggcga
gggcggcggcgtgatccagggctctgcttctgaggccacactggtggctctgctggccgctaggaccaaggtaatccacag
gctgcaggctgccagcccagagctgacccaggctgccatcatggagaagctggtggcctactctagcgaccaggctcattcc
tctgtggagagggctggcctgatcggcggcgtgaagctgaaggctatcccttccgatggcaatttcgctatgagagccagcg
ccctgcaggaggctctggagagggacaaggctgccggcctgatccctttcttcatggtggccacactgggcacaaccacatg
ctgttctttcgacaacctgctggaggtgggccccatctgcaataaggaggacatctggctgcatgtggatgctgcctacgccgg
ctccgcttttatctgccctgagtttcggcatctgctgaacggcgtggagtttgccgacagctttaacttcaatccccacaagtggct
gctggtgaacttcgactgctctgctatgtgggtgaagaagaggacagatctgaccggcgctttcagactggatccaacctacct
gaagcactctcatcaggactctggcctgatcaccgattaccggcattggcagatccctctgggccgccggtttaggagcctga
agatgtggttcgtgtttcggatgtatggcgtgaagggcctgcaggcctatatccggaagcatgtgcagctgtctcatgagtttga
gtccctggtgcgccaggaccctaggttcgagatctgcgtggaggtaatcctgggcctggtgtgcttcaggctgaagggcagc
aataaggtgaacgaggctctgctgcagaggatcaattccgctaagaagatccacctggtgccttgtcatctgcgggataagttt
gtgctgaggttcgccatctgtagccgcaccgtggagtctgcccacgtgcagagagcttgggagcacatcaaggagctggcc
gctgatgtgctgagagctgagagggagtga
>GDNF wild-type DNA (SEQ ID NO: 8) 
atgaagttatgggatgtcgtggctgtctgcctggtgctgctccacaccgcgtccgccttcccgctgcccgccggtaagaggcct
cccgaggcgcccgccgaagaccgctccctcggccgccgccgcgcgcccttcgcgctgagcagtgactcaaatatgccaga
ggattatcctgatcagttcgatgatgtcatggattttattcaagccaccattaaaagactgaaaaggtcaccagataaacaaatgg
cagtgcttcctagaagagagcggaatcggcaggctgcagctgccaacccagagaattccagaggaaaaggtcggagaggc
cagaggggcaaaaaccggggttgtgtcttaactgcaatacatttaaatgtcactgacttgggtctgggctatgaaaccaaggag
gaactgatttttaggtactgcagcggctcttgcgatgcagctgagacaacgtacgacaaaatattgaaaaacttatccagaaata
gaaggctggtgagtgacaaagtagggcaggcatgttgcagacccatcgcctttgatgatgacctgtcgtttttagatgataacct
ggtttaccatattctaagaaagcattccgctaaaaggtgtggatgtatctga
>GDNF01 DNA (SEQ ID NO: 9) 
atgaagctgtgggatgtggtggccgtgtgcctggtgctgctgcatacagctagcgcctttcccctgccagctggcaagagacc
acctgaggctcctgccgaggacagatctctgggcaggagacgggcccctttcgctctgtctagcgattccaacatgcccgag
gactatccagaccagttcgatgacgtgatggacttcatccaggctaccatcaagaggctgaagagaagcccagacaagcaga
tggctgtgctgcctagacgcgagaggaatagacaggccgctgctgccaatcctgagaacagcaggggcaagggccggag
gggccagcgcggcaagaatagaggctgcgtgctgaccgctatccacctgaacgtgacagacctgggcctgggctacgaga
ccaaggaggagctgatcttccgctattgttccggctcttgtgatgccgctgagaccacatatgacaagatcctgaagaacctga
gcagaaacaggagactggtgtctgacaaggtgggccaggcctgttgcagaccaatcgccttcgatgatgacctgagctttctg
gacgataacctggtgtatcacatcctgagaaagcactctgccaagagatgtggctgcatctga
>GDNF02 DNA (SEQ ID NO: 10) 
atgaagctgtgggatgtggtggccgtgtgcctggtgctgctgcatacagcttctgcctttcctctgcccgctggcaagaggcct
ccagaggccccagctgaggacagatccctgggcagaaggagagccccttttgctctgtccagcgactctaacatgcccgag
 
gattatcccgaccagttcgacgatgtgatggacttcatccaggctaccatcaagaggctgaagagaagcccagataagcagat
ggctgtgctgccacgcagagagaggaacagacaggccgctgctgctaaccctgagaactctcggggcaagggcagaagg
ggccagagaggcaagaatcgcggctgcgtgctgacagctatccacctgaacgtgacagatctgggcctgggctacgagac
aaaggaggagctgatcttcagatactgttctggcagctgtgatgctgccgagacaacctacgacaagatcctgaagaacctga
gcagaaatcggaggctggtgtccgataaggtgggccaggcctgttgcagacctatcgctttcgacgacgatctgagcttcctg
gacgataacctggtgtaccacatcctgcggaagcattctgccaagagatgcggctgtatctga
>GDNF03 DNA (SEQ ID NO: 11) 
atgaagctgtgggatgtggtggccgtgtgcctggtgctgctgcatacagcttctgccttccctctgccagctggcaagagacca
cctgaggcccccgctgaggatagaagcctgggccgcagaagggctccttttgccctgagctccgactctaatatgcctgagg
attacccagaccagttcgatgacgtgatggactttatccaggccacaatcaagcgcctgaagaggtctccagacaagcagatg
gctgtgctgcctaggagagagagaaaccggcaggctgccgctgctaatccagagaactctaggggcaagggcaggagag
gccagaggggcaagaatcgcggctgcgtgctgaccgctatccatctgaatgtgaccgatctgggcctgggctacgagacaa
aggaggagctgatcttcaggtactgcagcggctcttgtgacgccgctgagaccacatacgacaagatcctgaagaacctgag
cagaaaccgcagactggtgtctgataaggtgggccaggcttgttgcaggcccatcgcttttgatgacgatctgtctttcctggac
gataatctggtgtaccacatcctgcggaagcactctgctaagcggtgtggctgcatctga
>GDNF04 DNA (SEQ ID NO: 12) 
atgaagctgtgggatgtggtggccgtgtgcctggtgctgctgcacaccgcttctgccttcccactgcctgccggcaagagacc
tcccgaggcccctgccgaggacagaagcctgggcaggcggagagccccatttgctctgtctagcgattccaacatgcctga
ggattaccccgatcagttcgatgacgtgatggatttcatccaggccaccatcaagagactgaagagatctcctgacaagcagat
ggctgtgctgcctagaagggagagaaacaggcaggccgctgctgccaatccagagaactccaggggcaagggcagaagg
ggccagcgcggcaagaatagaggctgcgtgctgacagccatccacctgaacgtgaccgacctgggcctgggctacgagac
caaggaggagctgatcttcaggtactgtagcggctcctgtgatgctgccgagaccacatacgacaagatcctgaagaacctgt
ccaggaacagaaggctggtgtctgacaaggtgggccaggcttgctgtaggccaatcgctttcgacgacgatctgtcctttctg
gatgacaacctggtgtaccacatcctgaggaagcattccgctaagagatgtggctgcatctga
>HRPDAAV01-A (SEQ ID NO: 13) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcctatcgatatcaagcttcgttac
ataacttacggtaaatggcccgcctggctgaccgcccaacgacccccgcccattgacgtcaatagtaacgccaatagggactt
tccattgacgtcaatgggtggagtatttacggtaaactgcccacttggcagtacatcaagtgtatcatatgccaagtacgccccct
attgacgtcaatgacggtaaatggcccgcctggcattgtgcccagtacatgaccttatgggactttcctacttggcagtacatcta
cgtattagtcatcgctattaccatggtcgaggtgagccccacgttctgcttcactctccccatctcccccccctccccacccccaa
ttttgtatttatttattttttaattattttgtgcagcgatgggggcggggggggggggggggcgcgcgccaggcggggcggggc
ggggcgaggggcggggcggggcgaggcggagaggtgcggcggcagccaatcagagcggcgcgctccgaaagtttcct
tttatggcgaggcggcggcggcggcggccctataaaaagcgaagcgcgcggcgggcgggagtcgctgcgcgctgccttc
gccccgtgccccgctccgccgccgcctcgcgccgcccgccccggctctgactgaccgcgttactcccacaggtgagcggg
cgggacggcccttctcctccgggctgtaattagctgagcaagaggtaagggtttaagggatggttggttggtggggtattaatg
tttaattacctggagcacctgcctgaaatcactttttttcaggttgggggattcgaacatcggccgccaccatgaacgccagcga
gttcaggaggaggggcaaggagatggtggactacgtggccaactacatggagggcatcgagggcaggcaggtgtacccc
gacgtggagcccggctacctgaggcccctgatccccgccgccgccccccaggagcccgacaccttcgaggacatcatcaa
cgacgtggagaagatcatcatgcccggcgtgacccactggcacagcccctacttcttcgcctacttccccaccgccagcagct
accccgccatgctggccgacatgctgtgcggcgccatcggctgcatcggcttcagctgggccgccagccccgcctgcaccg
 
agctggagaccgtgatgatggactggctgggcaagatgctggagctgcccaaggccttcctgaacgagaaggccggcgag
ggcggcggcgtgatccagggcagcgccagcgaggccaccctggtggccctgctggccgccaggaccaaggtgatccaca
ggctgcaggccgccagccccgagctgacccaggccgccatcatggagaagctggtggcctacagcagcgaccaggccca
cagcagcgtggagagggccggcctgatcggcggcgtgaagctgaaggccatccccagcgacggcaacttcgccatgagg
gccagcgccctgcaggaggccctggagagggacaaggccgccggcctgatccccttcttcatggtggccaccctgggcac
caccacctgctgcagcttcgacaacctgctggaggtgggccccatctgcaacaaggaggacatctggctgcacgtggacgc
cgcctacgccggcagcgccttcatctgccccgagttcaggcacctgctgaacggcgtggagttcgccgacagcttcaacttca
acccccacaagtggctgctggtgaacttcgactgcagcgccatgtgggtgaagaagaggaccgacctgaccggcgccttca
ggctggaccccacctacctgaagcacagccaccaggacagcggcctgatcaccgactacaggcactggcagatccccctg
ggcaggaggttcaggagcctgaagatgtggttcgtgttcaggatgtacggcgtgaagggcctgcaggcctacatcaggaag
cacgtgcagctgagccacgagttcgagagcctggtgaggcaggaccccaggttcgagatctgcgtggaggtgatcctgggc
ctggtgtgcttcaggctgaagggcagcaacaaggtgaacgaggccctgctgcagaggatcaacagcgccaagaagatcca
cctggtgccctgccacctgagggacaagttcgtgctgaggttcgccatctgcagcaggaccgtggagagcgcccacgtgca
gagggcctgggagcacatcaaggagctggccgccgacgtgctgagggccgagagggagtgactcgaggggtggcatcc
ctgtgacccctccccagtgcctctcctggccctggaagttgccactccagtgcccaccagccttgtcctaataaaattaagttgc
atcattttgtctgactaggtgtccttctataatattatggggtggaggggggtggtatggagcaaggggcaagttgggaagaca
acctgtagggcctgcggggtctattgggaaccaagctggagtgcagtggcacaatcttggctcactgcaatctccgcctcctg
ggttcaagcgattctcctgcctcagcctcccgagttgttgggattccaggcatgcatgaccaggctcagctaatttttgtttttttgg
tagagacggggtttcaccatattggccaggctggtctccaactcctaatctcaggtgatctacccaccttggcctcccaaattgct
gggattacaggcgtgaaccactgctcccttccctgtccttcggaccgagcggcagatctaggaacccctagtgatggagttgg
ccactccctctctgcgcgctcgctcgctcactgaggccgggcgaccaaaggtcgcccgacgcccgggctttgcccgggcgg
cctcagtgagcgagcgagcgcgcagctgcctgcagg
>HRPDAAV01-B (SEQ ID NO: 14) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcctatcgatatcaagcttcgttac
ataacttacggtaaatggcccgcctggctgaccgcccaacgacccccgcccattgacgtcaatagtaacgccaatagggactt
tccattgacgtcaatgggtggagtatttacggtaaactgcccacttggcagtacatcaagtgtatcatatgccaagtacgccccct
attgacgtcaatgacggtaaatggcccgcctggcattgtgcccagtacatgaccttatgggactttcctacttggcagtacatcta
cgtattagtcatcgctattaccatggtcgaggtgagccccacgttctgcttcactctccccatctcccccccctccccacccccaa
ttttgtatttatttattttttaattattttgtgcagcgatgggggcggggggggggggggggcgcgcgccaggcggggcggggc
ggggcgaggggcggggcggggcgaggcggagaggtgcggcggcagccaatcagagcggcgcgctccgaaagtttcct
tttatggcgaggcggcggcggcggcggccctataaaaagcgaagcgcgcggcgggcgggagtcgctgcgcgctgccttc
gccccgtgccccgctccgccgccgcctcgcgccgcccgccccggctctgactgaccgcgttactcccacaggtgagcggg
cgggacggcccttctcctccgggctgtaattagctgagcaagaggtaagggtttaagggatggttggttggtggggtattaatg
tttaattacctggagcacctgcctgaaatcactttttttcaggttgggggattcgaacatcggccgccaccatgaacgccagcga
gttcaggaggaggggcaaggagatggtggactacgtggccaactacatggagggcatcgagggcaggcaggtgtacccc
gacgtggagcccggctacctgaggcccctgatccccgccgccgccccccaggagcccgacaccttcgaggacatcatcaa
cgacgtggagaagatcatcatgcccggcgtgacccactggcacagcccctacttcttcgcctacttccccaccgccagcagct
accccgccatgctggccgacatgctgtgcggcgccatcggctgcatcggcttcagctgggccgccagccccgcctgcaccg
agctggagaccgtgatgatggactggctgggcaagatgctggagctgcccaaggccttcctgaacgagaaggccggcgag
ggcggcggcgtgatccagggcagcgccagcgaggccaccctggtggccctgctggccgccaggaccaaggtgatccaca
 
ggctgcaggccgccagccccgagctgacccaggccgccatcatggagaagctggtggcctacagcagcgaccaggccca
cagcagcgtggagagggccggcctgatcggcggcgtgaagctgaaggccatccccagcgacggcaacttcgccatgagg
gccagcgccctgcaggaggccctggagagggacaaggccgccggcctgatccccttcttcatggtggccaccctgggcac
caccacctgctgcagcttcgacaacctgctggaggtgggccccatctgcaacaaggaggacatctggctgcacgtggacgc
cgcctacgccggcagcgccttcatctgccccgagttcaggcacctgctgaacggcgtggagttcgccgacagcttcaacttca
acccccacaagtggctgctggtgaacttcgactgcagcgccatgtgggtgaagaagaggaccgacctgaccggcgccttca
ggctggaccccacctacctgaagcacagccaccaggacagcggcctgatcaccgactacaggcactggcagatccccctg
ggcaggaggttcaggagcctgaagatgtggttcgtgttcaggatgtacggcgtgaagggcctgcaggcctacatcaggaag
cacgtgcagctgagccacgagttcgagagcctggtgaggcaggaccccaggttcgagatctgcgtggaggtgatcctgggc
ctggtgtgcttcaggctgaagggcagcaacaaggtgaacgaggccctgctgcagaggatcaacagcgccaagaagatcca
cctggtgccctgccacctgagggacaagttcgtgctgaggttcgccatctgcagcaggaccgtggagagcgcccacgtgca
gagggcctgggagcacatcaaggagctggccgccgacgtgctgagggccgagagggagtgactcgagtaagatacattg
atgagtttggacaaaccacaactagaatgcagtgaaaaaaatgctttatttgtgaaatttgtgatgctattgctttatttgtaaccatt
ataagctgcaataaacaagttcggaccgagcggcagatctaggaacccctagtgatggagttggccactccctctctgcgcgc
tcgctcgctcactgaggccgggcgaccaaaggtcgcccgacgcccgggctttgcccgggcggcctcagtgagcgagcga
gcgcgcagctgcctgcagg
>HRPDAAV01-C (SEQ ID NO: 15) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcctatcgatatcaagcttcgttac
ataacttacggtaaatggcccgcctggctgaccgcccaacgacccccgcccattgacgtcaatagtaacgccaatagggactt
tccattgacgtcaatgggtggagtatttacggtaaactgcccacttggcagtacatcaagtgtatcatatgccaagtacgccccct
attgacgtcaatgacggtaaatggcccgcctggcattgtgcccagtacatgaccttatgggactttcctacttggcagtacatcta
cgtattagtcatcgctattaccatggtcgaggtgagccccacgttctgcttcactctccccatctcccccccctccccacccccaa
ttttgtatttatttattttttaattattttgtgcagcgatgggggcggggggggggggggggcgcgcgccaggcggggcggggc
ggggcgaggggcggggcggggcgaggcggagaggtgcggcggcagccaatcagagcggcgcgctccgaaagtttcct
tttatggcgaggcggcggcggcggcggccctataaaaagcgaagcgcgcggcgggcgggagtcgctgcgcgctgccttc
gccccgtgccccgctccgccgccgcctcgcgccgcccgccccggctctgactgaccgcgttactcccacaggtgagcggg
cgggacggcccttctcctccgggctgtaattagctgagcaagaggtaagggtttaagggatggttggttggtggggtattaatg
tttaattacctggagcacctgcctgaaatcactttttttcaggttgggggattcgaacatcggccgccaccatgaacgccagcga
gttcaggaggaggggcaaggagatggtggactacgtggccaactacatggagggcatcgagggcaggcaggtgtacccc
gacgtggagcccggctacctgaggcccctgatccccgccgccgccccccaggagcccgacaccttcgaggacatcatcaa
cgacgtggagaagatcatcatgcccggcgtgacccactggcacagcccctacttcttcgcctacttccccaccgccagcagct
accccgccatgctggccgacatgctgtgcggcgccatcggctgcatcggcttcagctgggccgccagccccgcctgcaccg
agctggagaccgtgatgatggactggctgggcaagatgctggagctgcccaaggccttcctgaacgagaaggccggcgag
ggcggcggcgtgatccagggcagcgccagcgaggccaccctggtggccctgctggccgccaggaccaaggtgatccaca
ggctgcaggccgccagccccgagctgacccaggccgccatcatggagaagctggtggcctacagcagcgaccaggccca
cagcagcgtggagagggccggcctgatcggcggcgtgaagctgaaggccatccccagcgacggcaacttcgccatgagg
gccagcgccctgcaggaggccctggagagggacaaggccgccggcctgatccccttcttcatggtggccaccctgggcac
caccacctgctgcagcttcgacaacctgctggaggtgggccccatctgcaacaaggaggacatctggctgcacgtggacgc
cgcctacgccggcagcgccttcatctgccccgagttcaggcacctgctgaacggcgtggagttcgccgacagcttcaacttca
acccccacaagtggctgctggtgaacttcgactgcagcgccatgtgggtgaagaagaggaccgacctgaccggcgccttca
 
ggctggaccccacctacctgaagcacagccaccaggacagcggcctgatcaccgactacaggcactggcagatccccctg
ggcaggaggttcaggagcctgaagatgtggttcgtgttcaggatgtacggcgtgaagggcctgcaggcctacatcaggaag
cacgtgcagctgagccacgagttcgagagcctggtgaggcaggaccccaggttcgagatctgcgtggaggtgatcctgggc
ctggtgtgcttcaggctgaagggcagcaacaaggtgaacgaggccctgctgcagaggatcaacagcgccaagaagatcca
cctggtgccctgccacctgagggacaagttcgtgctgaggttcgccatctgcagcaggaccgtggagagcgcccacgtgca
gagggcctgggagcacatcaaggagctggccgccgacgtgctgagggccgagagggagtgactcgagaatcaacctctg
gattacaaaatttgtgaaagattgactggtattcttaactatgttgctccttttacgctatgtggatacgctgctttaatgcctttgtatc
atgctattgcttcccgtatggctttcattttctcctccttgtataaatcctggttgctgtctctttatgaggagttgtggcccgttgtcag
gcaacgtggcgtggtgtgcactgtgtttgctgacgcaacccccactggttggggcattgccaccacctgtcagctcctttccgg
gactttcgctttccccctccctattgccacggcggaactcatcgccgcctgccttgcccgctgctggacaggggctcggctgtt
gggcactgacaattccgtggtgttgtcggggaaatcatcgtcctttccttggctgctcgcctgtgttgccacctggattctgcgcg
ggacgtccttctgctacgtcccttcggccctcaatccagcggaccttccttcccgcggcctgctgccggctctgcggcctcttcc
gcgtcttcgccttcgccctcagacgagtcggatctccctttgggccgcctccccgctaagatacattgatgagtttggacaaacc
acaactagaatgcagtgaaaaaaatgctttatttgtgaaatttgtgatgctattgctttatttgtaaccattataagctgcaataaaca
agttcggaccgagcggcagatctaggaacccctagtgatggagttggccactccctctctgcgcgctcgctcgctcactgagg
ccgggcgaccaaaggtcgcccgacgcccgggctttgcccgggcggcctcagtgagcgagcgagcgcgcagctgcctgca
gg
>HRPDAAV02-A (SEQ ID NO: 16) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcctatcgatatcaagcttcgttac
ataacttacggtaaatggcccgcctggctgaccgcccaacgacccccgcccattgacgtcaatagtaacgccaatagggactt
tccattgacgtcaatgggtggagtatttacggtaaactgcccacttggcagtacatcaagtgtatcatatgccaagtacgccccct
attgacgtcaatgacggtaaatggcccgcctggcattgtgcccagtacatgaccttatgggactttcctacttggcagtacatcta
cgtattagtcatcgctattaccatggtcgaggtgagccccacgttctgcttcactctccccatctcccccccctccccacccccaa
ttttgtatttatttattttttaattattttgtgcagcgatgggggcggggggggggggggggcgcgcgccaggcggggcggggc
ggggcgaggggcggggcggggcgaggcggagaggtgcggcggcagccaatcagagcggcgcgctccgaaagtttcct
tttatggcgaggcggcggcggcggcggccctataaaaagcgaagcgcgcggcgggcgggagtcgctgcgcgctgccttc
gccccgtgccccgctccgccgccgcctcgcgccgcccgccccggctctgactgaccgcgttactcccacaggtgagcggg
cgggacggcccttctcctccgggctgtaattagctgagcaagaggtaagggtttaagggatggttggttggtggggtattaatg
tttaattacctggagcacctgcctgaaatcactttttttcaggttgggggattcgaacatcggccgccaccatgaacgccagcga
gttcaggaggaggggcaaggagatggtggactacgtggccaactacatggagggcatcgagggcaggcaggtgtacccc
gacgtggagcccggctacctgaggcccctgatccccgccgccgccccccaggagcccgacaccttcgaggacatcatcaa
cgacgtggagaagatcatcatgcccggcgtgacccactggcacagcccctacttcttcgcctacttccccaccgccagcagct
accccgccatgctggccgacatgctgtgcggcgccatcggctgcatcggcttcagctgggccgccagccccgcctgcaccg
agctggagaccgtgatgatggactggctgggcaagatgctggagctgcccaaggccttcctgaacgagaaggccggcgag
ggcggcggcgtgatccagggcagcgccagcgaggccaccctggtggccctgctggccgccaggaccaaggtgatccaca
ggctgcaggccgccagccccgagctgacccaggccgccatcatggagaagctggtggcctacagcagcgaccaggccca
cagcagcgtggagagggccggcctgatcggcggcgtgaagctgaaggccatccccagcgacggcaacttcgccatgagg
gccagcgccctgcaggaggccctggagagggacaaggccgccggcctgatccccttcttcatggtggccaccctgggcac
caccacctgctgcagcttcgacaacctgctggaggtgggccccatctgcaacaaggaggacatctggctgcacgtggacgc
cgcctacgccggcagcgccttcatctgccccgagttcaggcacctgctgaacggcgtggagttcgccgacagcttcaacttca
 
accccccacaagtggctgctggtgaacttcgactgcagcgccatgtgggtgaagaagaggaccgacctgaccggcgccttca
ggctggaccccacctacctgaagcacagccaccaggacagcggcctgatcaccgactacaggcactggcagatccccctg
ggcaggaggttcaggagcctgaagatgtggttcgtgttcaggatgtacggcgtgaagggcctgcaggcctacatcaggaag
cacgtgcagctgagccacgagttcgagagcctggtgaggcaggaccccaggttcgagatctgcgtggaggtgatcctgggc
ctggtgtgcttcaggctgaagggcagcaacaaggtgaacgaggccctgctgcagaggatcaacagcgccaagaagatcca
cctggtgccctgccacctgagggacaagttcgtgctgaggttcgccatctgcagcaggaccgtggagagcgcccacgtgca
gagggcctgggagcacatcaaggagctggccgccgacgtgctgagggccgagagggaggccaccaacttctccctgctg
aagcaggccggcgacgtggaggagaaccccggccccatgaagttatgggatgtcgtggctgtctgcctggtgctgctccac
accgcgtccgccttcccgctgcccgccggtaagaggcctcccgaggcgcccgccgaagaccgctccctcggccgccgcc
gcgcgcccttcgcgctgagcagtgactcaaatatgccagaggattatcctgatcagttcgatgatgtcatggattttattcaagcc
accattaaaagactgaaaaggtcaccagataaacaaatggcagtgcttcctagaagagagcggaatcggcaggctgcagct
gccaacccagagaattccagaggaaaaggtcggagaggccagaggggcaaaaaccggggttgtgtcttaactgcaatacat
ttaaatgtcactgacttgggtctgggctatgaaaccaaggaggaactgatttttaggtactgcagcggctcttgcgatgcagctg
agacaacgtacgacaaaatattgaaaaacttatccagaaatagaaggctggtgagtgacaaagtagggcaggcatgttgcag
acccatcgcctttgatgatgacctgtcgtttttagatgataacctggtttaccatattctaagaaagcattccgctaaaaggtgtgga
tgtatctgactcgagaatcaacctctggattacaaaatttgtgaaagattgactggtattcttaactatgttgctccttttacgctatgt
ggatacgctgctttaatgcctttgtatcatgctattgcttcccgtatggctttcattttctcctccttgtataaatcctggttgctgtctctt
tatgaggagttgtggcccgttgtcaggcaacgtggcgtggtgtgcactgtgtttgctgacgcaacccccactggttggggcatt
gccaccacctgtcagctcctttccgggactttcgctttccccctccctattgccacggcggaactcatcgccgcctgccttgccc
gctgctggacaggggctcggctgttgggcactgacaattccgtggtgttgtcggggaaatcatcgtcctttccttggctgctcgc
ctgtgttgccacctggattctgcgcgggacgtccttctgctacgtcccttcggccctcaatccagcggaccttccttcccgcggc
ctgctgccggctctgcggcctcttccgcgtcttcgccttcgccctcagacgagtcggatctccctttgggccgcctccccgctaa
gatacattgatgagtttggacaaaccacaactagaatgcagtgaaaaaaatgctttatttgtgaaatttgtgatgctattgctttattt
gtaaccattataagctgcaataaacaagttcggaccgagcggcagatctaggaacccctagtgatggagttggccactccctct
ctgcgcgctcgctcgctcactgaggccgggcgaccaaaggtcgcccgacgcccgggctttgcccgggcggcctcagtgag
cgagcgagcgcgcagctgcctgcagg
>HRPDAAV02-B (SEQ ID NO: 17) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcctatcgatatcaagcttcgttac
ataacttacggtaaatggcccgcctggctgaccgcccaacgacccccgcccattgacgtcaatagtaacgccaatagggactt
tccattgacgtcaatgggtggagtatttacggtaaactgcccacttggcagtacatcaagtgtatcatatgccaagtacgcccccct
attgacgtcaatgacggtaaatggcccgcctggcattgtgcccagtacatgaccttatgggactttcctacttggcagtacatcta
cgtattagtcatcgctattaccatggtcgaggtgagccccacgttctgcttcactctccccatctcccccccctccccacccccaa
ttttgtatttatttattttttaattattttgtgcagcgatgggggcgggggggggggggggcgcgcgccaggcggggcggggc
ggggcgaggggcggggcggggcgaggcggagaggtgcggcggcagccaatcagagcggcgcgctccgaaagtttcct
tttatggcgaggcggcggcggcggcggccctataaaaagcgaagcgcgcggcgggcgggagtcgctgcgcgctgccttc
gccccgtgccccgctccgccgccgcctcgcgccgcccgccccggctctgactgaccgcgttactcccacaggtgagcggg
cgggacggcccttctcctccgggctgtaattagctgagcaagaggtaagggtttaagggatggttggttggtggggtattaatg
tttaattacctggagcacctgcctgaaatcactttttttcaggttggggggattcgaacatcggccgccaccatgaagttatgggat
gtcgtggctgtctgcctggtgctgctccacaccgcgtccgccttcccgctgcccgccggtaagaggcctcccgaggcgcccg
ccgaagaccgctccctcggccgccgccgcgcgcccttcgcgctgagcagtgactcaaatatgccagaggattatcctgatca
 
gttcgatgatgtcatggattttattcaagccaccattaaaagactgaaaaggtcaccagataaacaaatggcagtgcttcctaga
agagagcggaatcggcaggctgcagctgccaacccagagaattccagaggaaaaggtcggagaggccagaggggcaaa
aaccggggttgtgtcttaactgcaatacatttaaatgtcactgacttgggtctgggctatgaaaccaaggaggaactgatttttag
gtactgcagcggctcttgcgatgcagctgagacaacgtacgacaaaatattgaaaaacttatccagaaatagaaggctggtga
gtgacaaagtagggcaggcatgttgcagacccatcgcctttgatgatgacctgtcgtttttagatgataacctggtttaccatattc
taagaaagcattccgctaaaaggtgtggatgtatctgagccaccaacttctccctgctgaagcaggccggcgacgtggagga
gaaccccggccccatgaacgccagcgagttcaggaggaggggcaaggagatggtggactacgtggccaactacatggag
ggcatcgagggcaggcaggtgtaccccgacgtggagcccggctacctgaggcccctgatccccgccgccgccccccagg
agcccgacaccttcgaggacatcatcaacgacgtggagaagatcatcatgcccggcgtgacccactggcacagcccctactt
cttcgcctacttccccaccgccagcagctaccccgccatgctggccgacatgctgtgcggcgccatcggctgcatcggcttca
gctgggccgccagccccgcctgcaccgagctggagaccgtgatgatggactggctgggcaagatgctggagctgcccaag
gccttcctgaacgagaaggccggcgagggcggcggcgtgatccagggcagcgccagcgaggccaccctggtggccctg
ctggccgccaggaccaaggtgatccacaggctgcaggccgccagccccgagctgacccaggccgccatcatggagaagc
tggtggcctacagcagcgaccaggcccacagcagcgtggagagggccggcctgatcggcggcgtgaagctgaaggccat
ccccagcgacggcaacttcgccatgagggccagcgccctgcaggaggccctggagagggacaaggccgccggcctgat
ccccttcttcatggtggccaccctgggcaccaccacctgctgcagcttcgacaacctgctggaggtgggccccatctgcaaca
aggaggacatctggctgcacgtggacgccgcctacgccggcagcgccttcatctgccccgagttcaggcacctgctgaacg
gcgtggagttcgccgacagcttcaacttcaacccccacaagtggctgctggtgaacttcgactgcagcgccatgtgggtgaag
aagaggaccgacctgaccggcgccttcaggctggaccccacctacctgaagcacagccaccaggacagcggcctgatcac
cgactacaggcactggcagatccccctgggcaggaggttcaggagcctgaagatgtggttcgtgttcaggatgtacggcgtg
aagggcctgcaggcctacatcaggaagcacgtgcagctgagccacgagttcgagagcctggtgaggcaggaccccaggtt
cgagatctgcgtggaggtgatcctgggcctggtgtgcttcaggctgaagggcagcaacaaggtgaacgaggccctgctgca
gaggatcaacagcgccaagaagatccacctggtgccctgccacctgagggacaagttcgtgctgaggttcgccatctgcagc
aggaccgtggagagcgcccacgtgcagagggcctgggagcacatcaaggagctggccgccgacgtgctgagggccgag
agggagtgactcgagaatcaacctctggattacaaaatttgtgaaagattgactggtattcttaactatgttgctccttttacgctat
gtggatacgctgctttaatgcctttgtatcatgctattgcttcccgtatggctttcattttctcctccttgtataaatcctggttgctgtct
ctttatgaggagttgtggcccgttgtcaggcaacgtggcgtggtgtgcactgtgtttgctgacgcaacccccactggttggggc
attgccaccacctgtcagctcctttccgggactttcgctttccccctccctattgccacggcggaactcatcgccgcctgccttgc
ccgctgctggacaggggctcggctgttgggcactgacaattccgtggtgttgtcggggaaatcatcgtcctttccttggctgctc
gcctgtgttgccacctggattctgcgcgggacgtccttctgctacgtcccttcggccctcaatccagcggaccttccttcccgcg
gcctgctgccggctctgcggcctcttccgcgtcttcgccttcgccctcagacgagtcggatctccctttgggccgcctccccgc
taagatacattgatgagtttggacaaaccacaactagaatgcagtgaaaaaaatgctttatttgtgaaatttgtgatgctattgcttt
atttgtaaccattataagctgcaataaacaagttcggaccgagcggcagatctaggaacccctagtgatggagttggccactcc
ctctctgcgcgctcgctcgctcactgaggccgggcgaccaaaggtcgcccgacgcccgggctttgcccgggcggcctcagt
gagcgagcgagcgcgcagctgcctgcagg
>HRPDAAV03-A (SEQ ID NO: 18) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcctatcgatatcaagcttcgttac
ataacttacggtaaatggcccgcctggctgaccgcccaacgacccccgcccattgacgtcaatagtaacgccaatagggactt
tccattgacgtcaatgggtggagtatttacggtaaactgcccacttggcagtacatcaagtgtatcatatgccaagtacgccccct
attgacgtcaatgacggtaaatggcccgcctggcattgtgcccagtacatgaccttatgggactttcctacttggcagtacatcta
 
cgtattagtcatcgctattaccatggtcgaggtgagccccacgttctgcttcactctccccatctcccccccctccccacccccaa
ttttgtatttatttattttttaattattttgtgcagcgatgggggcggggggggggggggggcgcgcgccaggcggggcggggc
ggggcgaggggcggggcggggcgaggcggagaggtgcggcggcagccaatcagagcggcgcgctccgaaagtttcct
tttatggcgaggcggcggcggcggcggccctataaaaagcgaagcgcgcggcgggcgggagtcgctgcgcgctgccttc
gccccgtgccccgctccgccgccgcctcgcgccgcccgccccggctctgactgaccgcgttactcccacaggtgagcggg
cgggacggcccttctcctccgggctgtaattagctgagcaagaggtaagggtttaagggatggttggttggtggggtattaatg
tttaattacctggagcacctgcctgaaatcactttttttcaggttgggggattcgaacatcggccgccaccatgaacgccagcga
gttcaggaggaggggcaaggagatggtggactacgtggccaactacatggagggcatcgagggcaggcaggtgtacccc
gacgtggagcccggctacctgaggcccctgatccccgccgccgccccccaggagcccgacaccttcgaggacatcatcaa
cgacgtggagaagatcatcatgcccggcgtgacccactggcacagcccctacttcttcgcctacttccccaccgccagcagct
accccgccatgctggccgacatgctgtgcggcgccatcggctgcatcggcttcagctgggccgccagccccgcctgcaccg
agctggagaccgtgatgatggactggctgggcaagatgctggagctgcccaaggccttcctgaacgagaaggccggcgag
ggcggcggcgtgatccagggcagcgccagcgaggccaccctggtggccctgctggccgccaggaccaaggtgatccaca
ggctgcaggccgccagccccgagctgacccaggccgccatcatggagaagctggtggcctacagcagcgaccaggccca
cagcagcgtggagagggccggcctgatcggcggcgtgaagctgaaggccatccccagcgacggcaacttcgccatgagg
gccagcgccctgcaggaggccctggagagggacaaggccgccggcctgatccccttcttcatggtggccaccctgggcac
caccacctgctgcagcttcgacaacctgctggaggtgggccccatctgcaacaaggaggacatctggctgcacgtggacgc
cgcctacgccggcagcgccttcatctgccccgagttcaggcacctgctgaacggcgtggagttcgccgacagcttcaacttca
acccccacaagtggctgctggtgaacttcgactgcagcgccatgtgggtgaagaagaggaccgacctgaccggcgccttca
ggctggaccccacctacctgaagcacagccaccaggacagcggcctgatcaccgactacaggcactggcagatccccctg
ggcaggaggttcaggagcctgaagatgtggttcgtgttcaggatgtacggcgtgaagggcctgcaggcctacatcaggaag
cacgtgcagctgagccacgagttcgagagcctggtgaggcaggaccccaggttcgagatctgcgtggaggtgatcctgggc
ctggtgtgcttcaggctgaagggcagcaacaaggtgaacgaggccctgctgcagaggatcaacagcgccaagaagatcca
cctggtgccctgccacctgagggacaagttcgtgctgaggttcgccatctgcagcaggaccgtggagagcgcccacgtgca
gagggcctgggagcacatcaaggagctggccgccgacgtgctgagggccgagagggagtgactcgagtaagatacattg
atgagtttggacaaaccacaactagaatgcagtgaaaaaaatgctttatttgtgaaatttgtgatgctattgctttatttgtaaccatt
ataagctgcaataaacaagttcgttacataacttacggtaaatggcccgcctggctgaccgcccaacgacccccgcccattga
cgtcaataatgacgtatgttcccatagtaacgtcaatagggactttccattgacgtcaatgggtggagtatttacggtaaactgcc
cacttggcagtacatcaagtgtatcatatgccaagtacgccccctattgacgtcaatgacggtaaatggcccgcctggcattatg
cccagtacatgaccttatgggactttcctacttggcagtacatctacgtattagtcatcgctattaccatggtgatgcggttttggca
gtacatcaatgggcgtggatagcggtttgactcacggggatttccaagtctccaccccattgacgtcaatgggagtttgttttgca
ccaaaatcaacgggactttccaaaatgtcgtaacaactccgccccattgacgcaaatgggcggtaggcgtgtacggtgggag
gtctatataagcagagctgccaccatgaagttatgggatgtcgtggctgtctgcctggtgctgctccacaccgcgtccgccttcc
cgctgcccgccggtaagaggcctcccgaggcgcccgccgaagaccgctccctcggccgccgccgcgcgcccttcgcgct
gagcagtgactcaaatatgccagaggattatcctgatcagttcgatgatgtcatggattttattcaagccaccattaaaagactga
aaaggtcaccagataaacaaatggcagtgcttcctagaagagagcggaatcggcaggctgcagctgccaacccagagaatt
ccagaggaaaaggtcggagaggccagaggggcaaaaaccggggttgtgtcttaactgcaatacatttaaatgtcactgacttg
ggtctgggctatgaaaccaaggaggaactgatttttaggtactgcagcggctcttgcgatgcagctgagacaacgtacgacaa
aatattgaaaaacttatccagaaatagaaggctggtgagtgacaaagtagggcaggcatgttgcagacccatcgcctttgatga
tgacctgtcgtttttagatgataacctggtttaccatattctaagaaagcattccgctaaaaggtgtggatgtatctgaaataaagtc
tgagtgggcggcagcctgtgtgtgcctgggttctctctgtcccggaatgtgcaaacaatggaggtgcggaccgagcggcaga
 
tctaggaacccctagtgatggagttggccactccctctctgcgcgctcgctcgctcactgaggccgggcgaccaaaggtcgc
ccgacgcccgggctttgcccgggcggcctcagtgagcgagcgagcgcgcagctgcctgcagg
>HRPDAAV03-B (SEQ ID NO: 19) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcctatcgatatcaagcttcgttac
ataacttacggtaaatggcccgcctggctgaccgcccaacgacccccgcccattgacgtcaatagtaacgccaatagggactt
tccattgacgtcaatgggtggagtatttacggtaaactgcccacttggcagtacatcaagtgtatcatatgccaagtacgccccct
attgacgtcaatgacggtaaatggcccgcctggcattgtgcccagtacatgaccttatgggactttcctacttggcagtacatcta
cgtattagtcatcgctattaccatggtcgaggtgagccccacgttctgcttcactctccccatctcccccccctccccacccccaa
ttttgtatttatttattttttaattattttgtgcagcgatgggggcggggggggggggggggcgcgcgccaggcggggcggggc
ggggcgaggggcggggcggggcgaggcggagaggtgcggcggcagccaatcagagcggcgcgctccgaaagtttcct
tttatggcgaggcggcggcggcggcggccctataaaaagcgaagcgcgcggcgggcgggagtcgctgcgcgctgccttc
gccccgtgccccgctccgccgccgcctcgcgccgcccgccccggctctgactgaccgcgttactcccacaggtgagcggg
cgggacggcccttctcctccgggctgtaattagctgagcaagaggtaagggtttaagggatggttggttggtggggtattaatg
tttaattacctggagcacctgcctgaaatcactttttttcaggttgggggattcgaacatcggccgccaccatgaacgccagcga
gttcaggaggaggggcaaggagatggtggactacgtggccaactacatggagggcatcgagggcaggcaggtgtacccc
gacgtggagcccggctacctgaggcccctgatccccgccgccgccccccaggagcccgacaccttcgaggacatcatcaa
cgacgtggagaagatcatcatgcccggcgtgacccactggcacagcccctacttcttcgcctacttccccaccgccagcagct
accccgccatgctggccgacatgctgtgcggcgccatcggctgcatcggcttcagctgggccgccagccccgcctgcaccg
agctggagaccgtgatgatggactggctgggcaagatgctggagctgcccaaggccttcctgaacgagaaggccggcgag
ggcggcggcgtgatccagggcagcgccagcgaggccaccctggtggccctgctggccgccaggaccaaggtgatccaca
ggctgcaggccgccagccccgagctgacccaggccgccatcatggagaagctggtggcctacagcagcgaccaggccca
cagcagcgtggagagggccggcctgatcggcggcgtgaagctgaaggccatccccagcgacggcaacttcgccatgagg
gccagcgccctgcaggaggccctggagagggacaaggccgccggcctgatccccttcttcatggtggccaccctgggcac
caccacctgctgcagcttcgacaacctgctggaggtgggccccatctgcaacaaggaggacatctggctgcacgtggacgc
cgcctacgccggcagcgccttcatctgccccgagttcaggcacctgctgaacggcgtggagttcgccgacagcttcaacttca
acccccacaagtggctgctggtgaacttcgactgcagcgccatgtgggtgaagaagaggaccgacctgaccggcgccttca
ggctggaccccacctacctgaagcacagccaccaggacagcggcctgatcaccgactacaggcactggcagatccccctg
ggcaggaggttcaggagcctgaagatgtggttcgtgttcaggatgtacggcgtgaagggcctgcaggcctacatcaggaag
cacgtgcagctgagccacgagttcgagagcctggtgaggcaggaccccaggttcgagatctgcgtggaggtgatcctgggc
ctggtgtgcttcaggctgaagggcagcaacaaggtgaacgaggccctgctgcagaggatcaacagcgccaagaagatcca
cctggtgccctgccacctgagggacaagttcgtgctgaggttcgccatctgcagcaggaccgtggagagcgcccacgtgca
gagggcctgggagcacatcaaggagctggccgccgacgtgctgagggccgagagggagtgactcgagtaagatacattg
atgagtttggacaaaccacaactagaatgcagtgaaaaaaatgctttatttgtgaaatttgtgatgctattgctttatttgtaaccatt
ataagctgcaataaacaagttcgttacataacttacggtaaatggcccgcctggctgaccgcccaacgacccccgcccattga
cgtcaataatgacgtatgttcccatagtaacgtcaatagggactttccattgacgtcaatgggtggagtatttacggtaaactgcc
cacttggcagtacatcaagtgtatcatatgccaagtacgccccctattgacgtcaatgacggtaaatggcccgcctggcattatg
cccagtacatgaccttatgggactttcctacttggcagtacatctacgtattagtcatcgctattaccatggtgatgcggttttggca
gtacatcaatgggcgtggatagcggtttgactcacggggatttccaagtctccaccccattgacgtcaatgggagtttgttttgca
ccaaaatcaacgggactttccaaaatgtcgtaacaactccgccccattgacgcaaatgggcggtaggcgtgtacggtgggag
gtctatataagcagagctgccaccatgaagttatgggatgtcgtggctgtctgcctggtgctgctccacaccgcgtccgccttcc
 
cgctgcccgccggtaagaggcctcccgaggcgcccgccgaagaccgctccctcggccgccgccgcgcgcccttcgcgct
gagcagtgactcaaatatgccagaggattatcctgatcagttcgatgatgtcatggattttattcaagccaccattaaaagactga
aaaggtcaccagataaacaaatggcagtgcttcctagaagagagcggaatcggcaggctgcagctgccaacccagagaatt
ccagaggaaaaggtcggagaggccagaggggcaaaaaccggggttgtgtcttaactgcaatacatttaaatgtcactgacttg
ggtctgggctatgaaaccaaggaggaactgatttttaggtactgcagcggctcttgcgatgcagctgagacaacgtacgacaa
aatattgaaaaacttatccagaaatagaaggctggtgagtgacaaagtagggcaggcatgttgcagacccatcgcctttgatga
tgacctgtcgtttttagatgataacctggtttaccatattctaagaaagcattccgctaaaaggtgtggatgtatctgaaatcaacct
ctggattacaaaatttgtgaaagattgactggtattcttaactatgttgctccttttacgctatgtggatacgctgctttaatgcctttgt
atcatgctattgcttcccgtatggctttcattttctcctccttgtataaatcctggttgctgtctctttatgaggagttgtggcccgttgtc
aggcaacgtggcgtggtgtgcactgtgtttgctgacgcaacccccactggttggggcattgccaccacctgtcagctcctttcc
gggactttcgctttccccctccctattgccacggcggaactcatcgccgcctgccttgcccgctgctggacaggggctcggct
gttgggcactgacaattccgtggtgttgtcggggaaatcatcgtcctttccttggctgctcgcctgtgttgccacctggattctgcg
cgggacgtccttctgctacgtcccttcggccctcaatccagcggaccttccttcccgcggcctgctgccggctctgcggcctctt
ccgcgtcttcgccttcgccctcagacgagtcggatctccctttgggccgcctccccgcaataaagtctgagtgggcggcagcc
tgtgtgtgcctgggttctctctgtcccggaatgtgcaaacaatggaggtgcggaccgagcggcagatctaggaacccctagtg
atggagttggccactccctctctgcgcgctcgctcgctcactgaggccgggcgaccaaaggtcgcccgacgcccgggctttg
cccgggcggcctcagtgagcgagcgagcgcgcagctgcctgcagg
>HRPDAAV03-C (SEQ ID NO: 20) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcctatcgatatcaagcttcgttac
ataacttacggtaaatggcccgcctggctgaccgcccaacgacccccgcccattgacgtcaatagtaacgccaatagggactt
tccattgacgtcaatgggtggagtatttacggtaaactgcccacttggcagtacatcaagtgtatcatatgccaagtacgccccct
attgacgtcaatgacggtaaatggcccgcctggcattgtgcccagtacatgaccttatgggactttcctacttggcagtacatcta
cgtattagtcatcgctattaccatggtcgaggtgagccccacgttctgcttcactctccccatctcccccccctccccacccccaa
ttttgtatttatttattttttaattattttgtgcagcgatgggggcggggggggggggggggcgcgcgccaggcggggcggggc
ggggcgaggggcggggcggggcgaggcggagaggtgcggcggcagccaatcagagcggcgcgctccgaaagtttcct
tttatggcgaggcggcggcggcggcggccctataaaaagcgaagcgcgcggcgggcgggagtcgctgcgcgctgccttc
gccccgtgccccgctccgccgccgcctcgcgccgcccgccccggctctgactgaccgcgttactcccacaggtgagcggg
cgggacggcccttctcctccgggctgtaattagctgagcaagaggtaagggtttaagggatggttggttggtggggtattaatg
tttaattacctggagcacctgcctgaaatcactttttttcaggttgggggattcgaacatcggccgccaccatgaacgccagcga
gttcaggaggaggggcaaggagatggtggactacgtggccaactacatggagggcatcgagggcaggcaggtgtacccc
gacgtggagcccggctacctgaggcccctgatccccgccgccgccccccaggagcccgacaccttcgaggacatcatcaa
cgacgtggagaagatcatcatgcccggcgtgacccactggcacagcccctacttcttcgcctacttccccaccgccagcagct
accccgccatgctggccgacatgctgtgcggcgccatcggctgcatcggcttcagctgggccgccagccccgcctgcaccg
agctggagaccgtgatgatggactggctgggcaagatgctggagctgcccaaggccttcctgaacgagaaggccggcgag
ggcggcggcgtgatccagggcagcgccagcgaggccaccctggtggccctgctggccgccaggaccaaggtgatccaca
ggctgcaggccgccagccccgagctgacccaggccgccatcatggagaagctggtggcctacagcagcgaccaggccca
cagcagcgtggagagggccggcctgatcggcggcgtgaagctgaaggccatccccagcgacggcaacttcgccatgagg
gccagcgccctgcaggaggccctggagagggacaaggccgccggcctgatccccttcttcatggtggccaccctgggcac
caccacctgctgcagcttcgacaacctgctggaggtgggccccatctgcaacaaggaggacatctggctgcacgtggacgc
cgcctacgccggcagcgccttcatctgccccgagttcaggcacctgctgaacggcgtggagttcgccgacagcttcaacttca
 
acccccacaagtggctgctggtgaacttcgactgcagcgccatgtgggtgaagaagaggaccgacctgaccggcgccttca
ggctggaccccacctacctgaagcacagccaccaggacagcggcctgatcaccgactacaggcactggcagatccccctg
ggcaggaggttcaggagcctgaagatgtggttcgtgttcaggatgtacggcgtgaagggcctgcaggcctacatcaggaag
cacgtgcagctgagccacgagttcgagagcctggtgaggcaggaccccaggttcgagatctgcgtggaggtgatcctgggc
ctggtgtgcttcaggctgaagggcagcaacaaggtgaacgaggccctgctgcagaggatcaacagcgccaagaagatcca
cctggtgccctgccacctgagggacaagttcgtgctgaggttcgccatctgcagcaggaccgtggagagcgcccacgtgca
gagggcctgggagcacatcaaggagctggccgccgacgtgctgagggccgagagggagtgactcgagtaagatacattg
atgagtttggacaaaccacaactagaatgcagtgaaaaaaatgctttatttgtgaaatttgtgatgctattgctttatttgtaaccatt
ataagctgcaataaacaagttcgttacataacttacggtaaatggcccgcctggctgaccgcccaacgacccccgcccattga
cgtcaataatgacgtatgttcccatagtaacgtcaatagggactttccattgacgtcaatgggtggagtatttacggtaaactgcc
cacttggcagtacatcaagtgtatcatatgccaagtacgccccctattgacgtcaatgacggtaaatggcccgcctggcattatg
cccagtacatgaccttatgggactttcctacttggcagtacatctacgtattagtcatcgctattaccatggtgatgcggttttggca
gtacatcaatgggcgtggatagcggtttgactcacggggatttccaagtctccaccccattgacgtcaatgggagtttgttttgca
ccaaaatcaacgggactttccaaaatgtcgtaacaactccgccccattgacgcaaatgggcggtaggcgtgtacggtgggag
gtctatataagcagagctgtgagtttggggacccttgattgttctttctttttcgctattgtaaaattcatgttatatggagggggcaa
agttttcagggtgttgtttagaatgggaagatgtcccttgtatcaccatggaccctcatgataattttgtttctttcactttctactctgtt
gacaaccattgtctcctcttattttcttttcattttctgtaactttttcgttaaactttagcttgcatttgtaacgaatttttaaattcacttttgt
ttatttgtcagattgtaagtactttctctaatcacttttttttcaaggcaatcagggtatattatattgtacttcagcacagttttagagaa
caattgttataattaaatgataaggtagaatatttctgcatataaattctggctggcgtggaaatattcttattggtagaaacaactac
atcctggtcatcatcctgcctttctctttatggttacaatgatatacactgtttgagatgaggataaaatactctgagtccaaaccgg
gcccctctgctaaccatgttcatgccttcttctttttcctacaggccaccatgaagttatgggatgtcgtggctgtctgcctggtgct
gctccacaccgcgtccgccttcccgctgcccgccggtaagaggcctcccgaggcgcccgccgaagaccgctccctcggcc
gccgccgcgcgcccttcgcgctgagcagtgactcaaatatgccagaggattatcctgatcagttcgatgatgtcatggattttatt
caagccaccattaaaagactgaaaaggtcaccagataaacaaatggcagtgcttcctagaagagagcggaatcggcaggct
gcagctgccaacccagagaattccagaggaaaaggtcggagaggccagaggggcaaaaaccggggttgtgtcttaactgc
aatacatttaaatgtcactgacttgggtctgggctatgaaaccaaggaggaactgatttttaggtactgcagcggctcttgcgatg
cagctgagacaacgtacgacaaaatattgaaaaacttatccagaaatagaaggctggtgagtgacaaagtagggcaggcatg
ttgcagacccatcgcctttgatgatgacctgtcgtttttagatgataacctggtttaccatattctaagaaagcattccgctaaaagg
tgtggatgtatctgaaataaagtctgagtgggcggcagcctgtgtgtgcctgggttctctctgtcccggaatgtgcaaacaatgg
aggtgcggaccgagcggcagatctaggaacccctagtgatggagttggccactccctctctgcgcgctcgctcgctcactga
ggccgggcgaccaaaggtcgcccgacgcccgggctttgcccgggcggcctcagtgagcgagcgagcgcgcagctgcct
gcagg
>HRPDAAV03-D (SEQ ID NO: 21) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcctatcgatatccgttacataactt
acggtaaatggcccgcctggctgaccgcccaacgacccccgcccattgacgtcaataatgacgtatgttcccatagtaacgtc
aatagggactttccattgacgtcaatgggtggagtatttacggtaaactgcccacttggcagtacatcaagtgtatcatatgccaa
gtacgccccctattgacgtcaatgacggtaaatggcccgcctggcattatgcccagtacatgaccttatgggactttcctacttg
gcagtacatctacgtattagtcatcgctattaccatggtgatgcggttttggcagtacatcaatgggcgtggatagcggtttgact
cacggggatttccaagtctccaccccattgacgtcaatgggagtttgttttgcaccaaaatcaacgggactttccaaaatgtcgta
acaactccgccccattgacgcaaatgggcggtaggcgtgtacggtgggaggtctatataagcagagctgtgagtttggggac
 
ccttgattgttctttctttttcgctattgtaaaattcatgttatatggagggggcaaagttttcagggtgttgtttagaatgggaagatgt
cccttgtatcaccatggaccctcatgataattttgtttctttcactttctactctgttgacaaccattgtctcctcttattttcttttcattttct
gtaactttttcgttaaactttagcttgcatttgtaacgaatttttaaattcacttttgtttatttgtcagattgtaagtactttctctaatcactt
ttttttcaaggcaatcagggtatattatattgtacttcagcacagttttagagaacaattgttataattaaatgataaggtagaatatttc
tgcatataaattctggctggcgtggaaatattcttattggtagaaacaactacatcctggtcatcatcctgcctttctctttatggttac
aatgatatacactgtttgagatgaggataaaatactctgagtccaaaccgggcccctctgctaaccatgttcatgccttcttcttttt
cctacaggccaccatgaagttatgggatgtcgtggctgtctgcctggtgctgctccacaccgcgtccgccttcccgctgcccgc
cggtaagaggcctcccgaggcgcccgccgaagaccgctccctcggccgccgccgcgcgcccttcgcgctgagcagtgac
tcaaatatgccagaggattatcctgatcagttcgatgatgtcatggattttattcaagccaccattaaaagactgaaaaggtcacc
agataaacaaatggcagtgcttcctagaagagagcggaatcggcaggctgcagctgccaacccagagaattccagaggaaa
aggtcggagaggccagaggggcaaaaaccggggttgtgtcttaactgcaatacatttaaatgtcactgacttgggtctgggct
atgaaaccaaggaggaactgatttttaggtactgcagcggctcttgcgatgcagctgagacaacgtacgacaaaatattgaaa
aacttatccagaaatagaaggctggtgagtgacaaagtagggcaggcatgttgcagacccatcgcctttgatgatgacctgtc
gtttttagatgataacctggtttaccatattctaagaaagcattccgctaaaaggtgtggatgtatctgaaataaagtctgagtggg
cggcagcctgtgtgtgcctgggttctctctgtcccggaatgtgcaaacaatggaggtgaagcttcgttacataacttacggtaaa
tggcccgcctggctgaccgcccaacgacccccgcccattgacgtcaatagtaacgccaatagggactttccattgacgtcaat
gggtggagtatttacggtaaactgcccacttggcagtacatcaagtgtatcatatgccaagtacgccccctattgacgtcaatga
cggtaaatggcccgcctggcattgtgcccagtacatgaccttatgggactttcctacttggcagtacatctacgtattagtcatcg
ctattaccatggtcgaggtgagccccacgttctgcttcactctccccatctcccccccctccccacccccaattttgtatttattt att
ttttaattattttgtgcagcgatgggggcggggggggggggggggcgcgcgccaggcggggcggggcggggcgagggg
cggggcggggcgaggcggagaggtgcggcggcagccaatcagagcggcgcgctccgaaagtttccttttatggcgaggc
ggcggcggcggcggccctataaaaagcgaagcgcgcggcgggcgggagtcgctgcgcgctgccttcgccccgtgcccc
gctccgccgccgcctcgcgccgcccgccccggctctgactgaccgcgttactcccacaggtgagcgggcgggacggccct
tctcctccgggctgtaattagctgagcaagaggtaagggtttaagggatggttggttggggtattaatgtttaattacctgga
gcacctgcctgaaatcactttttttcaggttgggggattcgaacatcggccgccaccatgaacgccagcgagttcaggaggag
gggcaaggagatggtggactacgtggccaactacatggagggcatcgagggcaggcaggtgtaccccgacgtggagccc
ggctacctgaggcccctgatccccgccgccgccccccaggagcccgacaccttcgaggacatcatcaacgacgtggagaa
gatcatcatgcccggcgtgacccactggcacagcccctacttcttcgcctacttccccaccgccagcagctaccccgccatgc
tggccgacatgctgtgcggcgccatcggctgcatcggcttcagctgggccgccagccccgcctgcaccgagctggagacc
gtgatgatggactggctgggcaagatgctggagctgcccaaggccttcctgaacgagaaggccggcgagggcggcggcgt
gatccagggcagcgccagcgaggccaccctggtggccctgctggccgccaggaccaaggtgatccacaggctgcaggcc
gccagccccgagctgacccaggccgccatcatggagaagctggtggcctacagcagcgaccaggcccacagcagcgtgg
agagggccggcctgatcggcggcgtgaagctgaaggccatccccagcgacggcaacttcgccatgagggccagcgccct
gcaggaggccctggagagggacaaggccgccggcctgatccccttcttcatggtggccaccctgggcaccaccacctgctg
cagcttcgacaacctgctggaggtgggccccatctgcaacaaggaggacatctggctgcacgtggacgccgcctacgccgg
cagcgccttcatctgccccgagttcaggcacctgctgaacggcgtggagttcgccgacagcttcaacttcaacccccacaagt
ggctgctggtgaacttcgactgcagcgccatgtgggtgaagaagaggaccgacctgaccggcgccttcaggctggacccca
cctacctgaagcacagccaccaggacagcggcctgatcaccgactacaggcactggcagatccccctgggcaggaggttc
aggagcctgaagatgtggttcgtgttcaggatgtacggcgtgaagggcctgcaggcctacatcaggaagcacgtgcagctga
gccacgagttcgagagcctggtgaggcaggaccccaggttcgagatctgcgtggaggtgatcctgggcctggtgtgcttcag
gctgaagggcagcaacaaggtgaacgaggccctgctgcagaggatcaacagcgccaagaagatccacctggtgccctgcc
 
acctgagggacaagttcgtgctgaggttcgccatctgcagcaggaccgtggagagcgcccacgtgcagagggcctgggag
cacatcaaggagctggccgccgacgtgctgagggccgagagggagtgactcgagtaagatacattgatgagtttggacaaa
ccacaactagaatgcagtgaaaaaaatgctttatttgtgaaatttgtgatgctattgctttatttgtaaccattataagctgcaataaa
caagttcggaccgagcggcagatctaggaacccctagtgatggagttggccactccctctctgcgcgctcgctcgctcactga
ggccgggcgaccaaaggtcgcccgacgcccgggctttgcccgggcggcctcagtgagcgagcgagcgcgcagctgcct
gcagg
>HRPDAAV03-E (SEQ ID NO: 22) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcctatcgatatccgttacataactt
acggtaaatggcccgcctggctgaccgcccaacgacccccgcccattgacgtcaataatgacgtatgttcccatagtaacgtc
aatagggactttccattgacgtcaatgggtggagtatttacggtaaactgcccacttggcagtacatcaagtgtatcatatgccaa
gtacgccccctattgacgtcaatgacggtaaatggcccgcctggcattatgcccagtacatgaccttatgggactttcctacttg
gcagtacatctacgtattagtcatcgctattaccatggtgatgcggttttggcagtacatcaatgggcgtggatagcggtttgact
cacggggatttccaagtctccaccccattgacgtcaatgggagtttgttttgcaccaaaatcaacgggactttccaaaatgtcgta
acaactccgccccattgacgcaaatgggcggtaggcgtgtacggtgggaggtctatataagcagagctgccaccatgaagtt
atgggatgtcgtggctgtctgcctggtgctgctccacaccgcgtccgccttcccgctgcccgccggtaagaggcctcccgag
gcgcccgccgaagaccgctccctcggccgccgccgcgcgcccttcgcgctgagcagtgactcaaatatgccagaggattat
cctgatcagttcgatgatgtcatggattttattcaagccaccattaaaagactgaaaaggtcaccagataaacaaatggcagtgc
ttcctagaagagagcggaatcggcaggctgcagctgccaacccagagaattccagaggaaaaggtcggagaggccagag
gggcaaaaaccggggttgtgtcttaactgcaatacatttaaatgtcactgacttgggtctgggctatgaaaccaaggaggaact
gatttttaggtactgcagcggctcttgcgatgcagctgagacaacgtacgacaaaatattgaaaaacttatccagaaatagaag
gctggtgagtgacaaagtagggcaggcatgttgcagacccatcgcctttgatgatgacctgtcgtttttagatgataacctggttt
accatattctaagaaagcattccgctaaaaggtgtggatgtatctgaaataaagtctgagtgggcggcagcctgtgtgtgcctg
ggttctctctgtcccggaatgtgcaaacaatggaggtgaagcttcgttacataacttacggtaaatggcccgcctggctgaccg
cccaacgacccccgcccattgacgtcaatagtaacgccaatagggactttccattgacgtcaatgggtggagtatttacggtaa
actgcccacttggcagtacatcaagtgtatcatatgccaagtacgccccctattgacgtcaatgacggtaaatggcccgcctgg
cattgtgcccagtacatgaccttatgggactttcctacttggcagtacatctacgtattagtcatcgctattaccatggtcgaggtg
agccccacgttctgcttcactctccccatctcccccccctccccacccccaattttgtatttatttattttttaattattttgtgcagcga
tgggggcggggggggggggggggcgcgcgccaggcggggcggggcggggcgaggggcggggcggggcgaggcg
gagaggtgcggcggcagccaatcagagcggcgcgctccgaaagtttccttttatggcgaggcggcggcggcggcggccct
ataaaaagcgaagcgcgcggcgggcgggagtcgctgcgcgctgccttcgccccgtgccccgctccgccgccgcctcgcg
ccgcccgccccggctctgactgaccgcgttactcccacaggtgagcgggcgggacggcccttctcctccgggctgtaattag
ctgagcaagaggtaagggtttaagggatggttggttggtggggtattaatgtttaattacctggagcacctgcctgaaatcacttt
ttttcaggttgggggattcgaacatcggccgccaccatgaacgccagcgagttcaggaggaggggcaaggagatggtggac
tacgtggccaactacatggagggcatcgagggcaggcaggtgtaccccgacgtggagcccggctacctgaggcccctgat
ccccgccgccgccccccaggagcccgacaccttcgaggacatcatcaacgacgtggagaagatcatcatgcccggcgtga
cccactggcacagcccctacttcttcgcctacttccccaccgccagcagctaccccgccatgctggccgacatgctgtgcggc
gccatcggctgcatcggcttcagctgggccgccagccccgcctgcaccgagctggagaccgtgatgatggactggctgggc
aagatgctggagctgcccaaggccttcctgaacgagaaggccggcgagggcggcggcgtgatccagggcagcgccagc
gaggccaccctggtggccctgctggccgccaggaccaaggtgatccacaggctgcaggccgccagccccgagctgaccc
aggccgccatcatggagaagctggtggcctacagcagcgaccaggcccacagcagcgtggagagggccggcctgatcgg
 
cggcgtgaagctgaaggccatccccagcgacggcaacttcgccatgagggccagcgccctgcaggaggccctggagagg
gacaaggccgccggcctgatccccttcttcatggtggccaccctgggcaccaccacctgctgcagcttcgacaacctgctgg
aggtgggccccatctgcaacaaggaggacatctggctgcacgtggacgccgcctacgccggcagcgccttcatctgccccg
agttcaggcacctgctgaacggcgtggagttcgccgacagcttcaacttcaacccccacaagtggctgctggtgaacttcgac
tgcagcgccatgtgggtgaagaagaggaccgacctgaccggcgccttcaggctggaccccacctacctgaagcacagcca
ccaggacagcggcctgatcaccgactacaggcactggcagatccccctgggcaggaggttcaggagcctgaagatgtggtt
cgtgttcaggatgtacggcgtgaagggcctgcaggcctacatcaggaagcacgtgcagctgagccacgagttcgagagcct
ggtgaggcaggaccccaggttcgagatctgcgtggaggtgatcctgggcctggtgtgcttcaggctgaagggcagcaacaa
ggtgaacgaggccctgctgcagaggatcaacagcgccaagaagatccacctggtgccctgccacctgagggacaagttcgt
gctgaggttcgccatctgcagcaggaccgtggagagcgcccacgtgcagagggcctgggagcacatcaaggagctggcc
gccgacgtgctgagggccgagagggagtgactcgagaatcaacctctggattacaaaatttgtgaaagattgactggtattctt
aactatgttgctccttttacgctatgtggatacgctgctttaatgcctttgtatcatgctattgcttcccgtatggctttcattttctcctc
cttgtataaatcctggttgctgtctctttatgaggagttgtggcccgttgtcaggcaacgtggcgtggtgtgcactgtgtttgctga
cgcaacccccactggttggggcattgccaccacctgtcagctcctttccgggactttcgctttccccctccctattgccacggcg
gaactcatcgccgcctgccttgcccgctgctggacaggggctcggctgttgggcactgacaattccgtggtgttgtcggggaa
atcatcgtcctttccttggctgctcgcctgtgttgccacctggattctgcgcgggacgtccttctgctacgtcccttcggccctcaa
tccagcggaccttccttcccgcggcctgctgccggctctgcggcctcttccgcgtcttcgccttcgccctcagacgagtcggat
ctccctttgggccgcctccccgctaagatacattgatgagtttggacaaaccacaactagaatgcagtgaaaaaaatgctttattt
gtgaaatttgtgatgctattgctttatttgtaaccattataagctgcaataaacaagttcggaccgagcggcagatctaggaaccc
ctagtgatggagttggccactccctctctgcgcgctcgctcgctcactgaggccgggcgaccaaaggtcgcccgacgcccg
ggctttgcccgggcggcctcagtgagcgagcgagcgcgcagctgcctgcagg
>HRPDAAV03-F (SEQ ID NO: 23) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcctatcgatatccgttacataactt
acggtaaatggcccgcctggctgaccgcccaacgacccccgcccattgacgtcaataatgacgtatgttcccatagtaacgtc
aatagggactttccattgacgtcaatgggtggagtatttacggtaaactgcccacttggcagtacatcaagtgtatcatatgccaa
gtacgccccctattgacgtcaatgacggtaaatggcccgcctggcattatgcccagtacatgaccttatgggactttcctacttg
gcagtacatctacgtattagtcatcgctattaccatggtgatgcggttttggcagtacatcaatgggcgtggatagcggtttgact
cacggggatttccaagtctccaccccattgacgtcaatgggagtttgttttgcaccaaaatcaacgggactttccaaaatgtcgta
acaactccgccccattgacgcaaatgggcggtaggcgtgtacggtgggaggtctatataagcagagctaagaggtaagggtt
taagggatggttggttggtggggtattaatgtttaattacctggagcacctgcctgaaatcactttttttcaggttgggccaccatg
aagttatgggatgtcgtggctgtctgcctggtgctgctccacaccgcgtccgccttcccgctgcccgccggtaagaggcctcc
cgaggcgcccgccgaagaccgctccctcggccgccgcgcgcccttcgcgctgagcagtgactcaaatatgccagagg
attatcctgatcagttcgatgatgtcatggattttattcaagccaccattaaaagactgaaaaggtcaccagataaacaaatggca
gtgcttcctagaagagagcggaatcggcaggctgcagctgccaacccagagaattccagaggaaaaggtcggagaggcca
gaggggcaaaaaccggggttgtgtcttaactgcaatacatttaaatgtcactgacttgggtctgggctatgaaaccaaggagga
actgatttttaggtactgcagcggctcttgcgatgcagctgagacaacgtacgacaaaatattgaaaaacttatccagaaataga
aggctggtgagtgacaaagtagggcaggcatgttgcagacccatcgcctttgatgatgacctgtcgtttttagatgataacctgg
tttaccatattctaagaaagcattccgctaaaaggtgtggatgtatctgaaataaagtctgagtgggcggcagcctgtgtgtgcct
gggttctctctgtcccggaatgtgcaaacaatggaggtgaagcttcgttacataacttacggtaaatggcccgcctggctgacc
gcccaacgacccccgcccattgacgtcaatagtaacgccaatagggactttccattgacgtcaatgggtggagtatttacggta
 
aactgcccacttggcagtacatcaagtgtatcatatgccaagtacgccccctattgacgtcaatgacggtaaatggcccgcctg
gcattgtgcccagtacatgaccttatgggactttcctacttggcagtacatctacgtattagtcatcgctattaccatggtcgaggt
gagccccacgttctgcttcactctccccatctcccccccctccccacccccaattttgtatttatttattttttaattattttgtgcagcg
atgggggcggggggggggggggggcgcgcgccaggcggggcggggcggggcgaggggcggggcggggcgaggc
ggagaggtgcggcggcagccaatcagagcggcgcgctccgaaagtttccttttatggcgaggcggcggcggcggcggcc
ctataaaaagcgaagcgcgcggcgggcgggagtcgctgcgcgctgccttcgccccgtgccccgctccgccgccgcctcgc
gccgcccgccccggctctgactgaccgcgttactcccacaggtgagcgggcgggacggcccttctcctccgggctgtaatta
gctgagcaagaggtaagggtttaagggatggttggttggtggggtattaatgtttaattacctggagcacctgcctgaaatcactt
tttttcaggttgggggattcgaacatcggccgccaccatgaacgccagcgagttcaggaggaggggcaaggagatggtgga
ctacgtggccaactacatggagggcatcgagggcaggcaggtgtaccccgacgtggagcccggctacctgaggcccctga
tccccgccgccgccccccaggagcccgacaccttcgaggacatcatcaacgacgtggagaagatcatcatgcccggcgtg
acccactggcacagcccctacttcttcgcctacttccccaccgccagcagctaccccgccatgctggccgacatgctgtgcgg
cgccatcggctgcatcggcttcagctgggccgccagccccgcctgcaccgagctggagaccgtgatgatggactggctggg
caagatgctggagctgcccaaggccttcctgaacgagaaggccggcgagggcggcggcgtgatccagggcagcgccag
cgaggccaccctggtggccctgctggccgccaggaccaaggtgatccacaggctgcaggccgccagccccgagctgacc
caggccgccatcatggagaagctggtggcctacagcagcgaccaggcccacagcagcgtggagagggccggcctgatcg
gcggcgtgaagctgaaggccatccccagcgacggcaacttcgccatgagggccagcgccctgcaggaggccctggagag
ggacaaggccgccggcctgatccccttcttcatggtggccaccctgggcaccaccacctgctgcagcttcgacaacctgctg
gaggtgggccccatctgcaacaaggaggacatctggctgcacgtggacgccgcctacgccggcagcgccttcatctgcccc
gagttcaggcacctgctgaacggcgtggagttcgccgacagcttcaacttcaacccccacaagtggctgctggtgaacttcga
ctgcagcgccatgtgggtgaagaagaggaccgacctgaccggcgccttcaggctggaccccacctacctgaagcacagcc
accaggacagcggcctgatcaccgactacaggcactggcagatccccctgggcaggaggttcaggagcctgaagatgtgg
ttcgtgttcaggatgtacggcgtgaagggcctgcaggcctacatcaggaagcacgtgcagctgagccacgagttcgagagc
ctggtgaggcaggaccccaggttcgagatctgcgtggaggtgatcctgggcctggtgtgcttcaggctgaagggcagcaac
aaggtgaacgaggccctgctgcagaggatcaacagcgccaagaagatccacctggtgccctgccacctgagggacaagtt
cgtgctgaggttcgccatctgcagcaggaccgtggagagcgcccacgtgcagagggcctgggagcacatcaaggagctgg
ccgccgacgtgctgagggccgagagggagtgactcgagaatcaacctctggattacaaaatttgtgaaagattgactggtatt
cttaactatgttgctccttttacgctatgtggatacgctgctttaatgcctttgtatcatgctattgcttcccgtatggctttcattttctcc
tccttgtataaatcctggttgctgtctctttatgaggagttgtggcccgttgtcaggcaacgtggcgtggtgtgcactgtgtttgctg
acgcaacccccactggttggggcattgccaccacctgtcagctcctttccgggactttcgctttccccctccctattgccacggc
ggaactcatcgccgcctgccttgcccgctgctggacaggggctcggctgttgggcactgacaattccgtggtgttgtcgggga
aatcatcgtcctttccttggctgctcgcctgtgttgccacctggattctgcgcgggacgtccttctgctacgtcccttcggccctca
atccagcggaccttccttcccgcggcctgctgccggctctgcggcctcttccgcgtcttcgccttcgccctcagacgagtcgga
tctccctttgggccgcctccccgctaagatacattgatgagtttggacaaaccacaactagaatgcagtgaaaaaaatgctttatt
tgtgaaatttgtgatgctattgctttatttgtaaccattataagctgcaataaacaagttcggaccgagcggcagatctaggaaccc
ctagtgatggagttggccactccctctctgcgcgctcgctcgctcactgaggccgggcgaccaaaggtcgcccgacgcccg
ggctttgcccgggcggcctcagtgagcgagcgagcgcgcagctgcctgcagg
>HRPDAAV02-AI (SEQ ID NO: 24) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcctatcgatatcaagcttcgttac
ataacttacggtaaatggcccgcctggctgaccgcccaacgacccccgcccattgacgtcaatagtaacgccaatagggactt
 
tccattgacgtcaatgggtggagtatttacggtaaactgcccacttggcagtacatcaagtgtatcatatgccaagtacgccccct
attgacgtcaatgacggtaaatggcccgcctggcattgtgcccagtacatgaccttatgggactttcctacttggcagtacatcta
cgtattagtcatcgctattaccatggtcgaggtgagccccacgttctgcttcactctccccatctcccccccctccccacccccaa
ttttgtatttatttattttttaattattttgtgcagcgatgggggcggggggggggggggggcgcgcgccaggcggggcggggc
ggggcgaggggcggggcggggcgaggcggagaggtgcggcggcagccaatcagagcggcgcgctccgaaagtttcct
tttatggcgaggcggcggcggcggcggccctataaaaagcgaagcgcgcggcgggcgggagtcgctgcgcgctgccttc
gccccgtgccccgctccgccgccgcctcgcgccgcccgccccggctctgactgaccgcgttactcccacaggtgagcggg
cgggacggcccttctcctccgggctgtaattagctgagcaagaggtaagggtttaagggatggttggttggtggggtattaatg
tttaattacctggagcacctgcctgaaatcactttttttcaggttgggggattcgaacatcggccgccaccatgaatgctagcgag
tttcgcagaaggggcaaggagatggtggattacgtggctaattacatggagggcatcgagggccggcaggtgtacccagat
gtggagccaggctacctgagaccactgatccctgctgccgccccacaggagccagacaccttcgaggatatcatcaatgacg
tggagaagatcatcatgcctggcgtgacccactggcatagcccatacttctttgcctactttccaacagcctcttcctatcccgct
atgctggctgatatgctgtgcggcgctatcggctgcatcggcttctcttgggccgctagccctgcttgcacagagctggagacc
gtgatgatggattggctgggcaagatgctggagctgcctaaggcctttctgaacgagaaggctggcgagggcggcggcgtg
atccagggctctgcctccgaggctacactggtggctctgctggctgcccggacaaaggtaatccacagactgcaggctgcct
ctccagagctgacccaggccgctatcatggagaagctggtggcttactctagcgaccaggctcattcttccgtggagcgggct
ggcctgatcggcggcgtgaagctgaaggccatcccttccgacggcaatttcgccatgagagcttctgccctgcaggaggctc
tggagagagataaggctgccggcctgatccctttctttatggtggccaccctgggcacaaccacctgttgctccttcgacaacct
gctggaggtgggccctatctgtaacaaggaggacatctggctgcatgtggatgccgcttatgctggctctgcctttatctgcccc
gagtttagacatctgctgaacggcgtggagtttgccgacagctttaacttcaaccctcacaagtggctgctggtgaattttgactg
ctctgccatgtgggtgaagaagaggacagacctgaccggcgcttttaggctggatccaacctacctgaagcacagccatcag
gacagcggcctgatcacagactacagacactggcagatcccactgggcagaaggtttcggtctctgaagatgtggttcgtgttt
cgcatgtatggcgtgaagggcctgcaggcttatatccggaagcacgtgcagctgtctcatgagttcgagtccctggtgcggca
ggacccaagatttgagatctgcgtggaggtaatcctgggcctggtgtgcttcaggctgaagggctctaataaggtgaacgagg
ctctgctgcagaggatcaacagcgccaagaagatccatctggtgccttgtcatctgagggacaagttcgtgctgagattcgcca
tctgctctagaacagtggagtctgctcatgtgcagagggcttgggagcatatcaaggagctggccgctgacgtgctgagggct
gagagggaggccaccaacttctccctgctgaagcaggccggcgacgtggaggagaaccccggccccatgaagctgtggg
atgtggtggccgtgtgcctggtgctgctgcacaccgcttctgccttcccactgcctgccggcaagagacctcccgaggcccct
gccgaggacagaagcctgggcaggcggagagccccatttgctctgtctagcgattccaacatgcctgaggattaccccgatc
agttcgatgacgtgatggatttcatccaggccaccatcaagagactgaagagatctcctgacaagcagatggctgtgctgccta
gaagggagagaaacaggcaggccgctgctgccaatccagagaactccaggggcaagggcagaaggggccagcgcggc
aagaatagaggctgcgtgctgacagccatccacctgaacgtgaccgacctgggcctgggctacgagaccaaggaggagct
gatcttcaggtactgtagcggctcctgtgatgctgccgagaccacatacgacaagatcctgaagaacctgtccaggaacagaa
ggctggtgtctgacaaggtgggccaggcttgctgtaggccaatcgctttcgacgacgatctgtcctttctggatgacaacctggt
gtaccacatcctgaggaagcattccgctaagagatgtggctgcatctgactcgagaatcaacctctggattacaaaatttgtgaa
agattgactggtattcttaactatgttgctccttttacgctatgtggatacgctgctttaatgcctttgtatcatgctattgcttcccgtat
ggctttcattttctcctccttgtataaatcctggttgctgtctctttatgaggagttgtggcccgttgtcaggcaacgtggcgtggtgt
gcactgtgtttgctgacgcaacccccactggttggggcattgccaccacctgtcagctcctttccgggactttcgctttccccctc
cctattgccacggcggaactcatcgccgcctgccttgcccgctgctggacaggggctcggctgttgggcactgacaattccgt
ggtgttgtcggggaaatcatcgtcctttccttggctgctcgcctgtgttgccacctggattctgcgcgggacgtccttctgctacgt
cccttcggccctcaatccagcggaccttccttcccgcggcctgctgccggctctgcggcctcttccgcgtcttcgccttcgccct
 
cagacgagtcggatctccctttgggccgcctccccgctaagatacattgatgagtttggacaaaccacaactagaatgcagtga
aaaaaatgctttatttgtgaaatttgtgatgctattgctttatttgtaaccattataagctgcaataaacaagttcggaccgagcggc
agatctaggaacccctagtgatggagttggccactccctctctgcgcgctcgctcgctcactgaggccgggcgaccaaaggt
cgcccgacgcccgggctttgcccgggcggcctcagtgagcgagcgagcgcgcagctgcctgcagg
>HRPDAAV03-DI (SEQ ID NO: 25) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcctatcgatatccgttacataactt
acggtaaatggcccgcctggctgaccgcccaacgacccccgcccattgacgtcaataatgacgtatgttcccatagtaacgtc
aatagggactttccattgacgtcaatgggtggagtatttacggtaaactgcccacttggcagtacatcaagtgtatcatatgccaa
gtacgccccctattgacgtcaatgacggtaaatggcccgcctggcattatgcccagtacatgaccttatgggactttcctacttg
gcagtacatctacgtattagtcatcgctattaccatggtgatgcggttttggcagtacatcaatgggcgtggatagcggtttgact
cacggggatttccaagtctccaccccattgacgtcaatgggagtttgttttgcaccaaaatcaacgggactttccaaaatgtcgta
acaactccgccccattgacgcaaatgggcggtaggcgtgtacggtgggaggtctatataagcagagctgtgagtttggggac
ccttgattgttctttctttttcgctattgtaaaattcatgttatatggagggggcaaagttttcagggtgttgtttagaatgggaagatgt
cccttgtatcaccatggaccctcatgataattttgtttctttcactttctactctgttgacaaccattgtctcctcttattttcttttcattttct
gtaactttttcgttaaactttagcttgcatttgtaacgaatttttaaattcacttttgtttatttgtcagattgtaagtactttctctaatcactt
ttttttcaaggcaatcagggtatattatattgtacttcagcacagttttagagaacaattgttataattaaatgataaggtagaatatttc
tgcatataaattctggctggcgtggaaatattcttattggtagaaacaactacatcctggtcatcatcctgcctttctctttatggttac
aatgatatacactgtttgagatgaggataaaatactctgagtccaaaccgggcccctctgctaaccatgttcatgccttcttcttttt
cctacaggccaccatgaagctgtgggatgtggtggccgtgtgcctggtgctgctgcacaccgcttctgccttcccactgcctgc
cggcaagagacctcccgaggcccctgccgaggacagaagcctgggcaggcggagagccccatttgctctgtctagcgattc
caacatgcctgaggattaccccgatcagttcgatgacgtgatggatttcatccaggccaccatcaagagactgaagagatctcc
tgacaagcagatggctgtgctgcctagaagggagagaaacaggcaggccgctgctgccaatccagagaactccaggggca
agggcagaaggggccagcgcggcaagaatagaggctgcgtgctgacagccatccacctgaacgtgaccgacctgggcct
gggctacgagaccaaggaggagctgatcttcaggtactgtagcggctcctgtgatgctgccgagaccacatacgacaagatc
ctgaagaacctgtccaggaacagaaggctggtgtctgacaaggtgggccaggcttgctgtaggccaatcgctttcgacgacg
atctgtcctttctggatgacaacctggtgtaccacatcctgaggaagcattccgctaagagatgtggctgcatctgaaataaagt
ctgagtgggcggcagcctgtgtgtgcctgggttctctctgtcccggaatgtgcaaacaatggaggtgaagcttcgttacataact
tacggtaaatggcccgcctggctgaccgcccaacgacccccgcccattgacgtcaatagtaacgccaatagggactttccatt
gacgtcaatgggtggagtatttacggtaaactgcccacttggcagtacatcaagtgtatcatatgccaagtacgccccctattga
cgtcaatgacggtaaatggcccgcctggcattgtgcccagtacatgaccttatgggactttcctacttggcagtacatctacgtat
tagtcatcgctattaccatggtcgaggtgagccccacgttctgcttcactctccccatctcccccccctccccacccccaattttgt
atttatttattttttaattattttgtgcagcgatgggggcggggggggggggggggcgcgcgccaggcggggcggggcggg
gcgaggggcggggcggggcgaggcggagaggtgcggcggcagccaatcagagcggcgcgctccgaaagtttccttttat
ggcgaggcggcggcggcggcggccctataaaaagcgaagcgcgcggcgggcgggagtcgctgcgcgctgccttcgccc
cgtgccccgctccgccgccgcctcgcgccgcccgccccggctctgactgaccgcgttactcccacaggtgagcgggcggg
acggcccttctcctccgggctgtaattagctgagcaagaggtaagggtttaagggatggttggttggtggggtattaatgtttaat
tacctggagcacctgcctgaaatcactttttttcaggttgggggattcgaacatcggccgccaccatgaatgctagcgagtttcg
cagaaggggcaaggagatggtggattacgtggctaattacatggagggcatcgagggccggcaggtgtacccagatgtgg
agccaggctacctgagaccactgatccctgctgccgccccacaggagccagacaccttcgaggatatcatcaatgacgtgga
gaagatcatcatgcctggcgtgacccactggcatagcccatacttctttgcctactttccaacagcctcttcctatcccgctatgct
 
ggctgatatgctgtgcggcgctatcggctgcatcggcttctcttgggccgctagccctgcttgcacagagctggagaccgtgat
gatggattggctgggcaagatgctggagctgcctaaggcctttctgaacgagaaggctggcgagggcggcggcgtgatcca
gggctctgcctccgaggctacactggtggctctgctggctgcccggacaaaggtaatccacagactgcaggctgcctctcca
gagctgacccaggccgctatcatggagaagctggtggcttactctagcgaccaggctcattcttccgtggagcgggctggcct
gatcggcggcgtgaagctgaaggccatcccttccgacggcaatttcgccatgagagcttctgccctgcaggaggctctggag
agagataaggctgccggcctgatccctttctttatggtggccaccctgggcacaaccacctgttgctccttcgacaacctgctgg
aggtgggccctatctgtaacaaggaggacatctggctgcatgtggatgccgcttatgctggctctgcctttatctgccccgagttt
agacatctgctgaacggcgtggagtttgccgacagctttaacttcaaccctcacaagtggctgctggtgaattttgactgctctgc
catgtgggtgaagaagaggacagacctgaccggcgcttttaggctggatccaacctacctgaagcacagccatcaggacag
cggcctgatcacagactacagacactggcagatcccactgggcagaaggtttcggtctctgaagatgtggttcgtgtttcgcat
gtatggcgtgaagggcctgcaggcttatatccggaagcacgtgcagctgtctcatgagttcgagtccctggtgcggcaggac
ccaagatttgagatctgcgtggaggtaatcctgggcctggtgtgcttcaggctgaagggctctaataaggtgaacgaggctctg
ctgcagaggatcaacagcgccaagaagatccatctggtgccttgtcatctgagggacaagttcgtgctgagattcgccatctgc
tctagaacagtggagtctgctcatgtgcagagggcttgggagcatatcaaggagctggccgctgacgtgctgagggctgaga
gggagctcgagtaagatacattgatgagtttggacaaaccacaactagaatgcagtgaaaaaaatgctttatttgtgaaatttgtg
atgctattgctttatttgtaaccattataagctgcaataaacaagttcggaccgagcggcagatctaggaacccctagtgatgga
gttggccactccctctctgcgcgctcgctcgctcactgaggccgggcgaccaaaggtcgcccgacgcccgggctttgcccg
ggcggcctcagtgagcgagcgagcgcgcagctgcctgcagg
>5' ITR (SEQ ID NO: 26) 
cctgcaggcagctgcgcgctcgctcgctcactgaggccgcccgggcaaagcccgggcgtcgggcgacctttggtcgcccg
gcctcagtgagcgagcgagcgcgcagagagggagtggccaactccatcactaggggttcct
>CMV enhancer 1 (SEQ ID NO: 27) 
cgttacataacttacggtaaatggcccgcctggctgaccgcccaacgacccccgcccattgacgtcaatagtaacgccaatag
ggactttccattgacgtcaatgggtggagtatttacggtaaactgcccacttggcagtacatcaagtgtatcatatgccaagtacg
ccccctattgacgtcaatgacggtaaatggcccgcctggcattgtgcccagtacatgaccttatgggactttcctacttggcagt
acatctacgtattagtcatcgctattaccatg
>CBA promoter (SEQ ID NO: 28) 
tcgaggtgagccccacgttctgcttcactctccccatctcccccccctccccacccccaattttgtatttatttattttttaattattttgt
gcagcgatgggggcggggggggggggggggcgcgcgccaggcggggcggggcggggcgaggggcggggcgggg
cgaggcggagaggtgcggcggcagccaatcagagcggcgcgctccgaaagtttccttttatggcgaggcggcggcggcg
gcggccctataaaaagcgaagcgcgcggcgggcg
>hybrid intron (SEQ ID NO: 29) 
ggagtcgctgcgcgctgccttcgccccgtgccccgctccgccgccgcctcgcgccgcccgccccggctctgactgaccgc
gttactcccacaggtgagcgggcgggacggcccttctcctccgggctgtaattagctgagcaagaggtaagggtttaaggga
tggttggttggtggggtattaatgtttaattacctggagcacctgcctgaaatcactttttttcag
>hGH poly(A) (SEQ ID NO: 30) 
Gggtggcatccctgtgacccctccccagtgcctctcctggccctggaagttgccactccagtgcccaccagccttgtcctaata
aaattaagttgcatcattttgtctgactaggtgtccttctataatattatggggtggaggggggtggtatggagcaaggggcaagt
tgggaagacaacctgtagggcctgcggggtctattgggaaccaagctggagtgcagtggcacaatcttggctcactgcaatct
ccgcctcctgggttcaagcgattctcctgcctcagcctcccgagttgttgggattccaggcatgcatgaccaggctcagctaatt
tttgtttttttggtagagacggggtttcaccatattggccaggctggtctccaactcctaatctcaggtgatctacccaccttggcct
 cccaaattgctgggattacaggcgtgaaccactgctcccttccctgtcctt
>3' ITR (SEQ ID NO: 31) 
Aggaacccctagtgatggagttggccactccctctctgcgcgctcgctcgctcactgaggccgggcgaccaaaggtcgccc
gacgcccgggctttgcccgggcggcctcagtgagcgagcgagcgcgcagctgcctgcagg
>SV40 poly(A) (SEQ ID NO: 32) 
Taagatacattgatgagtttggacaaaccacaactagaatgcagtgaaaaaaatgctttatttgtgaaatttgtgatgctattgcttt
atttgtaaccattataagctgcaataaacaagtt
>HPRE (SEQ ID NO: 33) 
ataacaggcctattgattggaaagtttgtcaacgaattgtgggtcttttggggtttgctgccccttttacgcaatgtggatatcctgc
tttaatgcctttatatgcatgtatacaagcaaaacaggcttttactttctcgccaacttacaaggcctttctcagtaaacagtatatga
ccctttaccccgttgctcggcaacggcctggtctgtgccaagtgtttgctgacgcaacccccactggttggggcttggccatag
gccatcagcgcatgcgtggaacctttgtgtctcctctgccgatccatactgcggaactcctagccgcttgttttgctcgcagcag
gtctggagcaaacctcatcgggaccgacaattctgtcgtactctcccgcaagtatacatcgtttccatggctgctaggctgtgct
gccaactggatcctgcgcgggacgtcctttgtttacgtcccgtcggcgctgaatcccgcggacgacccctcccggggccgctt
ggggctctaccgcccgcttctccgtctgccgtaccgtccgaccacggggcgcacctctctttacgcggactccccgtctgtgc
cttctcatctgccggaccgtgtgcacttcgcttcacctctgcacgtcgcatggaggccaccgtgaacgcccaccggaacctgc
ccaaggtcttgcataagaggactcttggactttcagcaatgtcatc
>WPRE (SEQ ID NO: 34) 
Aatcaacctctggattacaaaatttgtgaaagattgactggtattcttaactatgttgctccttttacgctatgtggatacgctgcttt
aatgcctttgtatcatgctattgcttcccgtatggctttcattttctcctccttgtataaatcctggttgctgtctctttatgaggagttgt
ggcccgttgtcaggcaacgtggcgtggtgtgcactgtgtttgctgacgcaacccccactggttggggcattgccaccacctgt
cagctcctttccgggactttcgctttccccctccctattgccacggcggaactcatcgccgcctgccttgcccgctgctggacag
gggctcggctgttgggcactgacaattccgtggtgttgtcggggaaatcatcgtcctttccttggctgctcgcctgtgttgccacc
tggattctgcgcgggacgtccttctgctacgtcccttcggccctcaatccagcggaccttccttcccgcggcctgctgccggct
ctgcggcctcttccgcgtcttcgccttcgccctcagacgagtcggatctccctttgggccgcctccccgc
> CMV promoter (SEQ ID NO: 35) 
Gtgatgcggttttggcagtacatcaatgggcgtggatagcggtttgactcacggggatttccaagtctccaccccattgacgtc
aatgggagtttgttttgcaccaaaatcaacgggactttccaaaatgtcgtaacaactccgccccattgacgcaaatgggcggta
ggcgtgtacggtgggaggtctatataagcagagct
> PA75 polyA (SEQ ID NO: 36) 
Aataaagtctgagtgggcggcagcctgtgtgtgcctgggttctctctgtcccggaatgtgcaaacaatggaggtgggcctaca
tcaggaagcacgtgcagctgagccacgagttcgagagcctggtgaggcaggaccccaggttcgagatctgcgtggaggtg
atcctgggcctggtgtgcttcaggctgaagggcagcaacaaggtgaacgaggccctgctgcagaggatcaacagcgccaa
gaagatccacctggtgccctgccacctgagggacaagttcgtgctgaggttcgccatctgcagcaggaccgtggagagcgc
ccacgtgcagagggcctgggagcacatcaaggagctggccgccgacgtgctgagggccgagagggagtgactcgagtaa
gatacattgatgagtttggacaaaccacaactagaatgcagtgaaaaaaatgctttatttgtgaaatttgtgatgctattgctttattt
gtaaccattataagctgcaataaacaagttcgttacataacttacggtaaatggcccgcctggctgaccgcccaacgacccccg
cccattgacgtcaataatgacgtatgttcccatagtaacgtcaatagggactttccattgacgtcaatgggtggagtatttacggta
aactgcccacttggcagtacatcaagtgtatcatatgccaagtacgccccctattgacgtcaatgacggtaaatggcccgcctg
gcattatgcccagtacatgaccttatgggactttcctacttggcagtacatctacgtattagtcatcgctattaccatggtgatgcg
gttttggcagtacatcaatgggcgtggatagcggtttgactcacggggatttccaagtctccaccccattgacgtcaatgggagt
 
ttgttttgcaccaaaatcaacgggactttccaaaatgtcgtaacaactccgccccattgacgcaaatgggcggtaggcgtgtac
ggtgggaggtctatataagcagagctGCCACCatgaagttatgggatgtcgtggctgtctgcctggtgctgctccacacc
gcgtccgccttcccgctgcccgccggtaagaggcctcccgaggcgcccgccgaagaccgctccctcggccgccgccgcg
cgcccttcgcgctgagcagtgactcaaatatgccagaggattatcctgatcagttcgatgatgtcatggattttattcaagccacc
attaaaagactgaaaaggtc
> CMV enhancer 2 (SEQ ID NO: 37) 
Cgttacataacttacggtaaatggcccgcctggctgaccgcccaacgacccccgcccattgacgtcaataatgacgtatgttc
ccatagtaacgtcaatagggactttccattgacgtcaatgggtggagtatttacggtaaactgcccacttggcagtacatcaagt
gtatcatatgccaagtacgccccctattgacgtcaatgacggtaaatggcccgcctggcattatgcccagtacatgaccttatgg
gactttcctacttggcagtacatctacgtattagtcatcgctattaccatg
> β-globin intron (SEQ ID NO: 38) 
Gtgagtttggggacccttgattgttctttctttttcgctattgtaaaattcatgttatatggagggggcaaagttttcagggtgttgttt
agaatgggaagatgtcccttgtatcaccatggaccctcatgataattttgtttctttcactttctactctgttgacaaccattgtctcct
cttattttcttttcattttctgtaactttttcgttaaactttagcttgcatttgtaacgaatttttaaattcacttttgtttatttgtcagattgtaa
gtactttctctaatcacttttttttcaaggcaatcagggtatattatattgtacttcagcacagttttagagaacaattgttataattaaat
gataaggtagaatatttctgcatataaattctggctggcgtggaaatattcttattggtagaaacaactacatcctggtcatcatcct
gcctttctctttatggttacaatgatatacactgtttgagatgaggataaaatactctgagtccaaaccgggcccctctgctaacca
tgttcatgccttcttctttttcctacag
> MVM intron (SEQ ID NO: 39) 
aagaggtaagggtttaagggatggttggttggtggggtattaatgtttaattacctggagcacctgcctgaaatcactttttttcag
gttgg
> MCS (SEQ ID NO: 40)
   atcgatatccgttaca
> AAV2 capsid (SEQ ID NO: 41) 
atggctgccgatggttatcttccagattggctcgaggacactctctctgaaggaataagacagtggtggaagctcaaacctggc
ccaccaccaccaaagcccgcagagcggcataaggacgacagcaggggtcttgtgcttcctgggtacaagtacctcggaccc
ttcaacggactcgacaagggagagccggtcaacgaggcagacgccgcggccctcgagcacgacaaagcctacgaccgg
cagctcgacagcggagacaacccgtacctcaagtacaaccacgccgacgcggagtttcaggagcgccttaaagaagatac
gtcttttgggggcaacctcggacgagcagtcttccaggcgaaaaagagggttcttgaacctctgggcctggttgaggaacctg
ttaagacggctccgggaaaaaagaggccggtagagcactctcctgtggagccagactcctcctcgggaaccggaaaggcg
ggccagcagcctgcaagaaaaagattgaattttggtcagactggagacgcagactcagtacctgacccccagcctctcggac
agccaccagcagccccctctggtctgggaactaatacgatggctacaggcagtggcgcaccaatggcagacaataacgagg
gcgccgacggagtgggtaattcctcgggaaattggcattgcgattccacatggatgggcgacagagtcatcaccaccagcac
ccgaacctgggccctgcccacctacaacaaccacctctacaaacaaatttccagccaatcaggagcctcgaacgacaatcac
tactttggctacagcaccccttgggggtattttgacttcaacagattccactgccacttttcaccacgtgactggcaaagactcatc
aacaacaactggggattccgacccaagagactcaacttcaagctctttaacattcaagtcaaagaggtcacgcagaatgacgg
tacgacgacgattgccaataaccttaccagcacggttcaggtgtttactgactcggagtaccagctcccgtacgtcctcggctc
ggcgcatcaaggatgcctcccgccgttcccagcagacgtcttcatggtgccacagtatggatacctcaccctgaacaacggg
agtcaggcagtaggacgctcttcattttactgcctggagtactttccttctcagatgctgcgtaccggaaacaactttaccttcagc
tacacttttgaggacgttcctttccacagcagctacgctcacagccagagtctggaccgtctcatgaatcctctcatcgaccagta
cctgtattacttgagcagaacaaacactccaagtggaaccaccacgcagtcaaggcttcagttttctcaggccggagcgagtg
 
acattcgggaccagtctaggaactggcttcctggaccctgttaccgccagcagcgagtatcaaagacatctgcggataacaac
aacagtgaatactcgtggactggagctaccaagtaccacctcaatggcagagactctctggtgaatccgggcccggccatgg
caagccacaaggacgatgaagaaaagttttttcctcagagcggggttctcatctttgggaagcaaggctcagagaaaacaaat
gtggacattgaaaaggtcatgattacagacgaagaggaaatcaggacaaccaatcccgtggctacggagcagtatggttctgt
atctaccaacctccagagaggcaacagacaagcagctaccgcagatgtcaacacacaaggcgttcttccaggcatggtctgg
caggacagagatgtgtaccttcaggggcccatctgggcaaagattccacacacggacggacattttcacccctctcccctcat
gggtggattcggacttaaacaccctcctccacagattctcatcaagaacaccccggtacctgcgaatccttcgaccaccttcagt
gcggcaaagtttgcttccttcatcacacagtactccacgggacaggtcagcgtggagatcgagtgggagctgcagaaggaaa
acagcaaacgctggaatcccgaaattcagtacacttccaactacaacaagtctgttaatgtggactttactgtggacactaatgg
cgtgtattcagagcctcgccccattggcaccagatacctgactcgtaatctgtaa
> AAV9 capsid (SEQ ID NO: 42) 
atggctgccgatggttatcttccagattggctcgaggacaaccttagtgaaggaattcgcgagtggtgggctttgaaacctgga
gcccctcaacccaaggcaaatcaacaacatcaagacaacgctcgaggtcttgtgcttccgggttacaaataccttggacccgg
caacggactcgacaagggggagccggtcaacgcagcagacgcggcggccctcgagcacgacaaggcctacgaccagca
gctcaaggccggagacaacccgtacctcaagtacaaccacgccgacgccgagttccaggagcggctcaaagaagatacgt
cttttgggggcaacctcgggcgagcagtcttccaggccaaaaagaggcttcttgaacctcttggtctggttgaggaagcggct
aagacggctcctggaaagaagaggcctgtagagcagtctcctcaggaaccggactcctccgcgggtattggcaaatcgggt
gcacagcccgctaaaaagagactcaatttcggtcagactggcgacacagagtcagtcccagaccctcaaccaatcggagaa
cctcccgcagccccctcaggtgtgggatctcttacaatggcttcaggtggtggcgcaccagtggcagacaataacgaaggtg
ccgatggagtgggtagttcctcgggaaattggcattgcgattcccaatggctgggggacagagtcatcaccaccagcacccg
aacctgggccctgcccacctacaacaatcacctctacaagcaaatctccaacagcacatctggaggatcttcaaatgacaacg
cctacttcggctacagcaccccctgggggtattttgacttcaacagattccactgccacttctcaccacgtgactggcagcgact
catcaacaacaactggggattccggcctaagcgactcaacttcaagctcttcaacattcaggtcaaagaggttacggacaaca
atggagtcaagaccatcgccaataaccttaccagcacggtccaggtcttcacggactcagactatcagctcccgtacgtgctc
gggtcggctcacgagggctgcctcccgccgttcccagcggacgttttcatgattcctcagtacgggtatctgacgcttaatgat
ggaagccaggccgtgggtcgttcgtccttttactgcctggaatatttcccgtcgcaaatgctaagaacgggtaacaacttccagt
tcagctacgagtttgagaacgtacctttccatagcagctacgctcacagccaaagcctggaccgactaatgaatccactcatcg
accaatacttgtactatctctcaaagactattaacggttctggacagaatcaacaaacgctaaaattcagtgtggccggacccag
caacatggctgtccagggaagaaactacatacctggacccagctaccgacaacaacgtgtctcaaccactgtgactcaaaac
aacaacagcgaatttgcttggcctggagcttcttcttgggctctcaatggacgtaatagcttgatgaatcctggacctgctatggc
cagccacaaagaaggagaggaccgtttctttcctttgtctggatctttaatttttggcaaacaaggaactggaagagacaacgtg
gatgcggacaaagtcatgataaccaacgaagaagaaattaaaactactaacccggtagcaacggagtcctatggacaagtgg
ccacaaaccaccagagtgcccaagcacaggcgcagaccggctgggttcaaaaccaaggaatacttccgggtatggtttggc
aggacagagatgtgtacctgcaaggacccatttgggccaaaattcctcacacggacggcaactttcacccttctccgctgatgg
gagggtttggaatgaagcacccgcctcctcagatcctcatcaaaaacacacctgtacctgcggatcctccaacggccttcaac
aaggacaagctgaactctttcatcacccagtattctactggccaagtcagcgtggagatcgagtgggagctgcagaaggaaa
acagcaagcgctggaacccggagatccagtacacttccaactattacaagtctaataatgttgaatttgctgttaatactgaaggt
gtatatagtgaaccccgccccattggcaccagatacctgactcgtaatctgtaa
> T2A amino acid sequence (SEQ ID NO: 43)
   EGRGSLLTCGDVEENPGP
> P2A amino acid sequence (SEQ ID NO: 44)
   ATNF SLLKQ AGD VEENPGP
> E2A amino acid sequence (SEQ ID NO: 45)
   QCTNYALLKLAGDVESNPGP
> F2A amino acid sequence (SEQ ID NO: 46)
   VKQTLNFDLLKLAGDVESNPGP
> T2A nucleotide sequence (SEQ ID NO: 47)
   gagggcagaggcagtctgctgacatgcggtgacgtggaagagaatcccggccct
> P2A nucleotide sequence (SEQ ID NO: 48)
   gccaccaacttctccctgctgaagcaggccggcgacgtggaggagaaccccggcccc
> E2A nucleotide sequence (SEQ ID NO: 49)
   cagtgcaccaactacgccctgctgaagctggccggcgatgtggagagcaaccccgggccc
> F2A nucleotide sequence (SEQ ID NO: 50)
   Gtgaaacagactttgaattttgaccttctcaagttggcgggagacgtggagtccaaccctggacct

## Claims

1. A nucleic acid molecule, comprising a first polynucleotide and a second polynucleotide, wherein the first polynucleotide encodes aromatic L-amino acid decarboxylase (AADC) protein, and the second polynucleotide encodes glial cell line-derived neurotrophic factor (GDNF) protein.

2. The nucleic acid molecule according to claim 1, wherein the AADC protein comprises the amino acid sequence set forth in SEQ ID NO: 1, and/or the GDNF protein comprises the amino acid sequence set forth in SEQ ID NO: 2.

3. The nucleic acid molecule according to claim 1 or 2, wherein the first polynucleotide comprises a sequence having at least 75% identity to SEQ ID NO: 3 or a sequence having at least 95% identity to any one of SEQ ID NOs: 4-7, and/or the second polynucleotide comprises a sequence having at least 70% identity to SEQ ID NO: 8 or a sequence having at least 95% identity to any one of SEQ ID NOs: 9-12;
preferably, the first polynucleotide comprises the sequence set forth in any one of SEQ ID NOs: 3-7;
preferably, the second polynucleotide comprises the sequence set forth in any one of SEQ ID NOs: 8-12.

4. The nucleic acid molecule according to any one of the preceding claims, wherein the first polynucleotide and the second polynucleotide are linked by a third polynucleotide; preferably, the third polynucleotide encodes an amino acid sequence with linker function; more preferably, the amino acid sequence encoded by the third polynucleotide is set forth in any one of SEQ ID NOs: 43-46; most preferably, the sequence of the third polynucleotide is set forth in any one of SEQ ID NOs: 47-50 or has at least 95% identity thereto.

5. The nucleic acid molecule according to any one of the preceding claims, wherein the nucleic acid molecule further comprises the same expression control sequence operably linked to the first polynucleotide and the second polynucleotide, or two identical or different expression control sequences operably linked to the first polynucleotide and the second polynucleotide, respectively;
the expression control sequence comprises a promoter and/or an enhancer.

6. The nucleic acid molecule according to any one of the preceding claims, wherein the nucleic acid molecule further comprises any one or any combination of a 5' inverted terminal repeat (5' ITR), a 3' inverted terminal repeat (3' ITR), an intron, a post-transcriptional regulatory element, a polyadenylation signal (polyA), and a multiple cloning site (MCS).

7. The nucleic acid molecule according to claim 6, wherein:
the 5' ITR and/or 3' ITR are/is derived from AAV1, AAV2, AAV3, AAV4, AAV5, AAV6, AAV7, AAV8, AAV9, AAV9.47, AAV9(hu14), AAV10, AAV11, AAV12, AAV13, AAVrh8, AAVrh10, AAV-DJ, or AAV-DJ8; preferably from AAV2 or AAV9;
the promoter is selected from the group consisting of CMV, CAG, CBA, CBh, EFS, EF1, PGK, SV40, Ubi and RSV promoters, or any combination thereof;
the enhancer is selected from the group consisting of Ubi, CMV and RSV enhancers, or any combination thereof;
the intron is selected from the group consisting of MVM, SV40, β globin, EF-1α and hybrid introns, or any combinations thereof;
the polyA is selected from the group consisting of PA75 polyA, SV40 polyA, hGH polyA, BGH polyA, rbGlob polyA, or any combination thereof; and/or
the post-transcriptional regulatory element is selected from the group consisting of WPRE, HPRE, or a combination thereof.

8. The nucleic acid molecule according to any one of the preceding claims, wherein the nucleic acid molecule comprises, from the 5' end to the 3' end:
a) the CMV enhancer, the CBA promoter, the first polynucleotide, the second polynucleotide, and the polyA; wherein optionally, the hybrid intron is further comprised between the CBA promoter and the first polynucleotide; optionally, the WPRE sequence is further comprised between the second polynucleotide and the polyA;
b) the CMV enhancer, the CBA promoter, the second polynucleotide, the first polynucleotide, and the polyA; wherein optionally, the hybrid intron is further comprised between the CBA promoter and the second polynucleotide; optionally, the WPRE sequence is further comprised between the first polynucleotide and the polyA;
c) the CMV enhancer, the CBA promoter, the first polynucleotide, the polyA, the CMV enhancer, the CMV promoter, the second polynucleotide, and the polyA; wherein optionally, the hybrid intron is further comprised between the CBA promoter and the first polynucleotide; optionally, the β globin intron is further comprised between the CMV promoter and the second polynucleotide; optionally, the WPRE sequence is further comprised between the second polynucleotide and the polyA;
d) the CMV enhancer, the CMV promoter, the second polynucleotide, the polyA, the CMV enhancer, the CBA promoter, the first polynucleotide, and the polyA; wherein optionally, the β globin intron or the MVM intron is further comprised between the CMV promoter and the second polynucleotide; optionally, the hybrid intron is further comprised between the CBA promoter and the first polynucleotide; optionally, the WPRE sequence is further comprised between the first polynucleotide and the polyA;
e) the CMV enhancer, the CBA promoter, the first polynucleotide, the third polynucleotide, the second polynucleotide, and the polyA; wherein optionally, the hybrid intron is further comprised between the CBA promoter and the first polynucleotide; optionally, the WPRE sequence is further comprised between the second polynucleotide and the polyA; or,
f) the CMV enhancer, the CBA promoter, the second polynucleotide, the third polynucleotide, the first polynucleotide, and the polyA; wherein optionally, the hybrid intron is further comprised between the CBA promoter and the second polynucleotide; optionally, the WPRE sequence is further comprised between the first polynucleotide and the polyA;
preferably, the polyA is selected from the group consisting of hGH polyA, PA75 polyA, or SV40 polyA.

9. The nucleic acid molecule according to any one of claims 6-8, wherein:
the CMV enhancer comprises the sequence set forth in SEQ ID NO: 27 or 37;
the CBA promoter comprises the sequence set forth in SEQ ID NO: 28;
the CMV promoter comprises the sequence set forth in SEQ ID NO: 35;
the β globin intron comprises the sequence set forth in SEQ ID NO: 38;
the hybrid intron comprises the sequence set forth in SEQ ID NO: 29;
the MVM intron comprises the sequence set forth in SEQ ID NO: 39;
the PA75 polyA comprises the sequence set forth in SEQ ID NO: 36;
the SV40 polyA comprises the sequence set forth in SEQ ID NO: 32;
the hGH polyA comprises the sequence set forth in SEQ ID NO: 30;
the WPRE sequence is set forth in SEQ ID NO: 34;
the 5' ITR comprises the sequence set forth in SEQ ID NO: 26; and/or
the 3' ITR comprises the sequence set forth in SEQ ID NO: 31.

10. The nucleic acid molecule according to any one of the preceding claims, wherein the nucleic acid molecule comprises, from the 5' end to the 3' end:
(1) CMV enhancer-CBA promoter-hybrid intron-first polynucleotide-third polynucleotide-second polynucleotide encoding GDNF-WPRE-SV40 polyA;
(2) CMV enhancer-CBA promoter-hybrid intron-second polynucleotide encoding GDNF-third polynucleotide-first polynucleotide-WPRE-SV40 polyA;
(3) CMV enhancer-CBA promoter-hybrid intron-first polynucleotide-SV40 polyA-CMV enhancer-CMV promoter-second polynucleotide encoding GDNF-PA75 polyA;
(4) CMV enhancer-CBA promoter-hybrid intron-first polynucleotide-SV40 polyA-CMV enhancer-CMV promoter-second polynucleotide encoding GDNF-WPRE-PA75 polyA;
(5) CMV enhancer-CBA promoter-hybrid intron-first polynucleotide-SV40 polyA-CMV enhancer-CMV promoter-β globin intron-second polynucleotide encoding GDNF-PA75 polyA;
(6) CMV enhancer-CMV promoter-β globin intron-second polynucleotide encoding GDNF-PA75 poly A-CMV enhancer-CBA promoter-hybrid intron-first polynucleotide-SV40 polyA;
(7) CMV enhancer-CMV promoter-second polynucleotide encoding GDNF-PA75 poly A-CMV enhancer-CBA promoter-hybrid intron-first polynucleotide-WPRE-SV40 polyA; or
(8) CMV enhancer-CMV promoter-MVM intron-second polynucleotide encoding GDNF-PA75 poly A-CMV enhancer-CBA promoter-hybrid intron-first polynucleotide-WPRE-SV40 polyA.

11. The nucleic acid molecule according to any one of the preceding claims, comprising the sequence set forth in any one of SEQ ID NOs: 13-25 or a sequence having at least 95% sequence identity to any one of SEQ ID NOs: 13-25.

12. A nucleic acid molecule comprising a polynucleotide encoding AADC protein, wherein the nucleic acid molecule comprises the sequence set forth in any one of SEQ ID NOs: 4-7 or a sequence having at least 95% sequence identity to any one of SEQ ID NOs: 4-7.

13. A nucleic acid molecule comprising a polynucleotide encoding GDNF protein, wherein the nucleic acid molecule comprises the sequence set forth in any one of SEQ ID NOs: 9-12 or a sequence having at least 95% sequence identity to any one of SEQ ID NOs: 9-12.

14. The nucleic acid molecule according to claim 12 or 13, further comprising a 5' ITR, a 3' ITR, a promoter, an enhancer, an intron, a post-transcriptional regulatory element, a polyadenylation signal (polyA), and a multiple cloning site (MCS); wherein
preferably, the 5' ITR and/or 3' ITR are/is derived from AAV1, AAV2, AAV3, AAV4, AAV5, AAV6, AAV7, AAV8, AAV9, AAV9.47, AAV9(hu14), AAV10, AAV11, AAV12, AAV13, AAVrh8, AAVrh10, AAV-DJ, or AAV-DJ8;
the promoter is selected from the group consisting of CMV, CAG, CBA, CBh, EFS, EF1, PGK, SV40, Ubi and RSV promoters, or any combination thereof;
the enhancer is selected from the group consisting of Ubi, CMV and RSV enhancers, or any combination thereof;
the intron is selected from the group consisting of MVM, SV40, β globin, EF-1α and hybrid introns, or any combination thereof;
the polyA is selected from the group consisting of PA75 polyA, SV40 polyA, hGH polyA, BGH polyA, rbGlob polyA, or any combination thereof; and/or
the post-transcriptional regulatory element is selected from the group consisting of WPRE, HPRE, or a combination thereof;
more preferably,
the CMV enhancer comprises the sequence set forth in SEQ ID NO: 27 or 37,
the CBA promoter comprises the sequence set forth in SEQ ID NO: 28,
the CMV promoter comprises the sequence set forth in SEQ ID NO: 35,
the β globin intron comprises the sequence set forth in SEQ ID NO: 38,
the hybrid intron comprises the sequence set forth in SEQ ID NO: 29,
the MVM intron comprises the sequence set forth in SEQ ID NO: 39,
the PA75 polyA comprises the sequence set forth in SEQ ID NO: 36,
the SV40 polyA comprises the sequence set forth in SEQ ID NO: 32,
the hGH polyA comprises the sequence set forth in SEQ ID NO: 30,
the WPRE sequence is set forth in SEQ ID NO: 34,
the 5' ITR comprises the sequence set forth in SEQ ID NO: 26, and/or
the 3' ITR comprises the sequence set forth in SEQ ID NO: 31;
preferably, the nucleic acid molecule comprises, from the 5' end to the 3' end: g) the CMV enhancer, the CBA promoter, the first polynucleotide or the second polynucleotide, and the polyA; wherein optionally, the hybrid intron is further comprised between the CBA promoter and the first polynucleotide, or the hybrid intron is further comprised between the CBA and the second polynucleotide; optionally, the WPRE sequence is further comprised between the first polynucleotide and the polyA, or the WPRE sequence is further comprised between the second polynucleotide and the polyA.

15. A recombinant adeno-associated virus (rAAV) particle, comprising the nucleic acid molecule according to any one of claims 1-14 and an AAV capsid.

16. The rAAV particle according to claim 15, wherein the AAV capsid is selected from the group consisting of AAV1, AAV2, AAV3, AAV4, AAV5, AAV6, AAV7, AAV8, AAV9, AAV9.47, AAV9(hu14), AAV10, AAV11, AAV12, AAV13, AAVPHP.B, AAVrh74, AAVrh8, AAVrh10, AAV-DJ, or AAV-DJ8, preferably AAV2 or AAV9, and more preferably AAV9.

17. A pharmaceutical composition, comprising the nucleic acid molecule according to any one of claims 1-14 or the rAAV particle according to any one of claims 15-16, and one or more pharmaceutically acceptable excipients.

18. A vector, comprising the nucleic acid molecule according to any one of claims 1-14, wherein preferably, the vector is selected from the group consisting of a plasmid, a lentivirus, an adenovirus, an mRNA-LNP, an adeno-associated virus (AAV) vector, and a recombinant adeno-associated virus (rAAV) vector.

19. A cell, comprising or expressing the nucleic acid molecule according to any one of claims 1-14 or the vector according to claim 18, or packaging and producing the recombinant rAAV particle according to claims 15-16.

20. A rAAV production system for producing the recombinant rAAV particle according to claim 15 or 16, wherein the production system comprises:
(a) a polynucleotide sequence encoding an amino acid sequence of the AAV capsid;
(b) a nucleic acid molecule according to any one of claims 1-14 or the vector according to claim 18; and
(c) sufficient AAV rep function and helper function to allow packaging of the nucleic acid molecule according to any one of claims 1-14 or the vector according to claim 18 into the AAV capsid;
wherein the AAV capsid is selected from the group consisting of AAV1, AAV2, AAV3, AAV4, AAV5, AAV6, AAV7, AAV8, AAV9, AAV9.47, AAV9(hu14), AAV10, AAV11, AAV12, AAVrh8, AAVrh10, AAV-DJ, or AAV-DJ8, preferably AAV2 or AAV9, and more preferably AAV9.

21. The rAAV production system according to claim 20, wherein the sufficient AAV rep function and helper function are provided by a packaging cell, wherein the packaging cell preferably comprises three plasmids pHelper, pRC9, and pGOI.

22. A method for producing the rAAV particle according to claim 15 or 16, comprising packaging the polynucleotide according to any one of claims 1-14 or the vector according to claim 18 into the AAV capsid using a packaging cell, wherein the AAV capsid is selected from the group consisting of AAV1, AAV2, AAV3, AAV4, AAV5, AAV6, AAV7, AAV8, AAV9, AAV9.47, AAV9(hu14), AAV10, AAV11, AAV12, AAVrh8, AAVrh10, AAV-DJ, or AAV-DJ8, preferably AAV2 or AAV9, and more preferably AAV9.

23. A method for treating, alleviating, or preventing a central nervous system disease or symptom, comprising administering to the brain of a subject in need thereof an amount, effective in treatment or alleviation, of the polynucleotide according to any one of claims 1-14 or the rAAV particle according to any one of claims 15-16, the pharmaceutical composition according to claim 17, or the vector according to claim 18, wherein such that the AADC protein and GDNF protein are expressed in the brain of the subj ect;
preferably, the AADC protein and GDNF protein are expressed in the striatum of the subj ect;
preferably, the central nervous system disease or symptom is a neurodegenerative disease or symptom, more preferably dyskinesia or sleep disorder, and most preferably Parkinson's disease
